# EUROPÄISCHE PATENTSCHRIFT

(11) **EP 2 408 754 B1**
(45) Veröffentlichungstag und Bekanntmachung des Hinweises auf die Patenterteilung: **09.10.2013**
(21) Anmeldenummer: 10707478.3
(22) Anmeldetag: 09.03.2010
(51) Int. Cl.: C07D 249/12, A61K 31/4196, A61P 9/04, A61P 1/16

(54) **TRIAZOL-DERIVATE ALS VASOPRESSIN-REZEPTOR INHIBITOREN ZUR BEHANDLUNG VON HERZINSUFIZIENZ**
TRIAZOLE DERIVATIVES AS INHIBITORS OF THE VASOPRESSIN RECEPTOR FOR THE TREATMENT OF CARDIAC INSUFFICIENCY
DERIVÉS DE TRIAZOLE EN TANT QU'INHIBITEURS DU RÉCEPTEUR VASOPRESSIN POUR LE TRAITEMENT DE L'INSUFFISANCE CARDIAQUE

(30) Priorität: 18.03.2009 DE 102009013642
(43) Veröffentlichungstag der Anmeldung: 25.01.2012
(73) Patentinhaber: Bayer Intellectual Property GmbH, 40789 Monheim (DE)
(72) Erfinder: BRÜGGEMEIER, Ulf, 42799 Leichlingen (DE); FÜRSTNER, Chantal, 45478 Mülheim/Ruhr (DE); GEISS, Volker, 40883 Ratingen (DE); KELDENICH, Joerg, 42113 Wuppertal (DE); KERN, Armin, 42109 Wuppertal (DE); DELBECK, Martina, 45239 Essen (DE); KOLKHOF, Peter, 42113 Wuppertal (DE); KRETSCHMER, Axel, 42113 Wuppertal (DE); POOK, Elisabeth, 42109 Wuppertal (DE); SCHMECK, Carsten, 45472 Mülheim (DE); TRÜBEL, Hubert, 42281 Wuppertal (DE)
(74) Vertreter: BIP Patents
(86) Internationale Anmeldenummer: PCT/EP2010/001442
(87) Internationale Veröffentlichungsnummer: WO 2010/105750

(56) Entgegenhaltungen:
- WO-A1-2007/134862

## Beschreibung

Die vorliegende Anmeldung betrifft neue, substituierte Phenylalaninderivate, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von kardiovaskulären Erkrankungen.

Der Flüssigkeitsgehalt des menschlichen Körpers unterliegt verschiedenen physiologischen Kontrollmechanismen, die auf seine Konstanterhaltung abzielen (Volumenhomöostase). Dabei werden sowohl die Volumenfüllung des Blutgefäßsystems als auch die Osmolarität des Blutplasmas kontinuierlich von entsprechenden Sensoren (Barorezeptoren und Osmorezeptoren) registriert. Die Informationen, die diese Sensoren an die zuständigen Zentren des Hirns liefern, regulieren das Trinkverhalten und steuern vermittels humoraler und nervaler Signale die Flüssigkeitsausscheidung über die Nieren. Eine zentrale Bedeutung kommt hierbei dem Peptidhormon Vasopressin zu [Schrier R.W., Abraham, W.T., New Engl. J. Med. 341, 577-585 (1999)].

Vasopressin wird in spezialisierten, endokrinen Neuronen im *Nucleus supraopticus* und *N. paraventricularis* in der Wand des dritten Ventrikels (Hypothalamus) produziert und von dort entlang ihrer Nervenfortsätze in den Hypophysenhinterlappen (Neurohypophyse) verbracht. Dort wird das Hormon je nach Stimulus in die Blutbahn abgegeben. Ein Volumenverlust, z.B. infolge akuter Blutung, starkem Schwitzen, langem Dursten oder Diarrhöe, ist ein Stimulus für die verstärkte Ausschüttung des Hormons. Umgekehrt wird die Sekretion von Vasopressin durch eine Erhöhung des Intravasalvolumens, z.B. infolge gesteigerter Flüssigkeitszufuhr, gehemmt.

Vasopressin entfaltet seine Wirkung hauptsächlich über Bindung an drei Rezeptoren, die als V1a-, V1b- und V2-Rezeptoren klassifiziert werden und zur Familie der G-Protein-gekoppelten Rezeptoren gehören. V1a-Rezeptoren sind vorwiegend auf den Zellen der glatten Gefäßmuskulatur lokalisiert. Ihre Aktivierung ruft eine Vasokonstriktion hervor, wodurch der periphere Widerstand und Blutdruck ansteigen. Daneben sind V1a-Rezeptoren auch in der Leber nachweisbar. V1b-Rezeptoren (auch V3-Rezeptoren genannt) sind im zentralen Nervensystem nachweisbar. Zusammen mit dem Corticotropin-Releasing-Hormon (CRH) reguliert Vasopressin über den V1b-Rezeptor die basale und stressinduzierte Sekretion des Adrenocorticotropen Hormons (ACTH). V2-Rezeptoren finden sich im distalen Tubulusepithel und dem Epithel der Sammelrohre der Niere. Ihre Aktivierung macht diese Epithelien für Wasser durchlässig. Diesem Phänomen liegt der Einbau von Aquaporinen (speziellen Wasserkanälen) in die luminale Membran der Epithelzellen zugrunde.

Welche Bedeutung Vasopressin für die Rückresorption von Wasser aus dem Harn in der Niere hat, wird durch das Krankheitsbild des Diabetes insipidus deutlich, das durch einen Mangel des Hormons - z.B. infolge einer Hypophysenschädigung - hervorgerufen wird. Patienten, die an diesem Krankheitsbild leiden, scheiden bis zu 20 Liter Harn pro 24 Stunden aus, sofern ihnen das Hormon nicht substituiert wird. Dieses Volumen entspricht in etwa 10% des Primärharns. Wegen seiner großen Bedeutung für die Rückresorption von Wasser aus dem Harn wird Vasopressin auch synonym als Antidiuretisches Hormon (ADH) bezeichnet. Folgerichtig führt eine pharmakologische Hemmung der Vasopressin/ADH-Wirkung am V2-Rezeptor zu einer vermehrten Urinausscheidung. Im Gegensatz zu der Wirkung anderer Diuretika (Thiazide und Schleifendiuretika) jedoch bewirken V2-Rezeptor-Antagonisten eine vermehrte Wasserausscheidung, ohne die Ausscheidung von Elektrolyten maßgeblich zu steigern. Das bedeutet, dass durch V2-antagonistische Pharmaka die Volumenhomöostase wiederhergestellt werden kann, ohne dabei in die Elektrolyt-Homöostase einzugreifen. Daher erscheinen V2-antagonistisch wirksame Pharmaka besonders geeignet für die Behandlung aller krankhaften Zustände, die mit einer Überfrachtung des Organismus mit Wasser einhergehen, ohne dass parallel die Elektrolyte adäquat erhöht sind. Eine bedeutende Elektrolyt-Abnormalität ist klinisch-chemisch als Hyponatriämie (Natriumkonzentration < 135 mmol/L) messbar; sie stellt die wichtigste Elektrolyt-Abnormalität bei Krankenhauspatienten dar mit einer Häufigkeit von ca. 5% bzw. 250.000 Fällen pro Jahr allein in den USA. Bei einem Absinken der Plasma-Natriumkonzentration unter 115 mmol/L drohen komatöse Zustände und Tod.

Entsprechend der zugrunde liegenden Ursache unterscheidet man die hypovolämische, euvolämische und hypervolämische Hyponatriämie. Klinisch bedeutsam sind die Formen der Hypervolämie mit Ödembildung. Typische Beispiele hierfür sind das Syndrom der inadäquaten ADH/Vasopressin-Sekretion (SIAD) (z.B. nach Schädel-Hirn-Trauma oder als Paraneoplasie bei Carcinomen) und die hypervolämische Hyponatriämie bei Leberzirrhose, verschiedenen Nierenerkrankungen und Herzinsuffizienz [De Luca L. et al., Am. J. Cardiol. 96 (suppl.), 19L-23L (2005)]. Gerade Patienten mit Herzinsuffizienz weisen trotz ihrer relativen Hyponatriämie und Hypervolämie häufig erhöhte Vasopressin-Spiegel auf, was als die Folge einer generell gestörten neurohumoralen Regulation bei der Herzinsuffizienz angesehen wird [Francis G.S. et al., Circulation 82, 1724-1729 (1990)].

Die gestörte neurohumorale Regulation manifestiert sich im Wesentlichen in einer Erhöhung des Sympathicotonus und einer inadäquaten Aktivierung des Renin-Angiotensin-Aldosteron-Systems. Während die Hemmung dieser Komponenten durch Beta-Rezeptoren-Blocker einerseits und durch ACE-Inhibitoren bzw. Angiotensin-Rezeptor-Blocker andererseits heute fester Bestandteil der pharmakologischen Behandlung der Herzinsuffizienz ist, ist die inadäquate Steigerung der Vasopressin-Sekretion in der fortgeschrittenen Herzinsuffizienz derzeit noch nicht ausreichend therapierbar. Neben der durch V2-Rezeptoren vermittelten Retention von Wasser und den damit verbundenen ungünstigen hämodynamischen Folgen im Sinne einer Nachlasterhöhung werden auch durch V1a-vermittelte Vasokonstriktion die Entleerung des linken Ventrikels, der Druck in den Pulmonalgefäßen und die Herzleistung negativ beeinflusst. Darüber hinaus wird aufgrund tierexperimenteller Daten dem Vasopressin auch eine direkte Hypertrophie-fördernde Wirkung am Herzmuskel beigemessen. Im Unterschied zur renalen Wirkung der Volumenexpansion, die über Aktivierung von V2-Rezeptoren vermittelt ist, wird die direkte Wirkung am Herzmuskel durch Aktivierung von V1a-Rezeptoren ausgelöst.

Aus diesen Gründen erscheinen Substanzen, die die Wirkung des Vasopressins am V2- und/oder am V1a-Rezeptor hemmen, zur Behandlung der Herzinsuffizienz geeignet. Vor allem Verbindungen mit kombinierter Aktivität an beiden Vasopressin-Rezeptoren (V1a und V2) sollten sowohl wünschenswerte renale als auch hämodynamische Effekte bewirken und somit ein besonders ideales Profil für die Behandlung von Patienten mit Herzinsuffizienz bieten. Die Bereitstellung derartig kombinierter Vasopressin-Antagonisten erscheint auch insofern sinnvoll, als ein allein über V2-Rezeptor-Blockade vermittelter Volumenentzug die Erregung von Osmorezeptoren und in der Folge eine weitere kompensatorische Steigerung der Vasopressin-Ausschüttung nach sich ziehen kann. Hierdurch könnten - bei Fehlen einer gleichzeitig den V1a-Rezeptor blockierenden Komponente - die schädlichen Wirkungen des Vasopressins, wie z.B. Vasokonstriktion und Herzmuskelhypertrophie, noch verstärkt werden [Saghi P. et al., Europ. Heart J. 26, 538-543 (2005)].

In WO 99/54315 werden substituierte Triazolone mit neuroprotektiver Wirkung offenbart, und in WO 2006/117657 werden Triazolon-Derivate als anti-inflammatorische Agentien beschrieben. Ferner werden in EP 503 548-A1 und EP 587 134-A2 cyclische Harnstoff-Derivate und ihre Verwendung zur Behandlung von Thrombosen beansprucht. Substituierte Triazolthione als Ionenkanal-Modulatoren werden in WO 2005/097112 offenbart. WO 2007/134862 beschreibt substituierte Imidazol-2-one und 1,2,4-Triazolone als Vasopressin Rezeptor Antagonisten zur Behandlung kardiovakulärer Erkrankungen.

Aufgabe der vorliegenden Erfindung ist die Bereitstellung neuer potenter, selektiver dualer V1a/V2-Rezeptor-Antagonisten mit verbesserter Wirksamkeit an beiden Vasopressin-Rezeptoren, und als solche zur Behandlung und/oder Prävention von Krankheiten, insbesondere zur Behandlung und/oder Prävention von kardiovaskulären Erkrankungen geeignet sind.

Gegenstand der vorliegenden Erfindung sind Verbindungen der allgemeinen Formel (I) 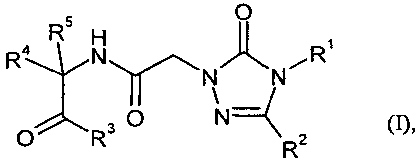 in welcher
- R¹: für (C₁-C₆)-Alkyl, (C₂-C₆)-Alkenyl, (C₂-C₆)-Alkinyl oder (C₃-C₇)-Cycloalkyl steht,
wobei (C₁-C₆)-Alkyl, (C₂-C₆)-Alkenyl und (C₂-C₆)-Alkinyl mit 1 bis 3 Substituenten unabhängig voneinander ausgewählt aus der Gruppe Halogen, Cyano, Oxo, Hydroxy, Trifluormethyl, (C₃-C₇)-Cycloalkyl, (C₁-C₆)-Alkoxy, Trifluormethoxy und Phenyl substituiert sein können,
worin (C₃-C₇)-Cycloalkyl mit 1 oder 2 Substituenten unabhängig voneinander ausgewählt aus der Gruppe (C₁-C₄)-Alkyl, Oxo, Hydroxy, (C₁-C₄)-Alkoxy und Amino substituiert sein kann,
   und
worin (C₁-C₆)-Alkoxy mit 1 oder 2 Substituenten unabhängig voneinander ausgewählt aus der Gruppe Amino, Hydroxy, (C₁-C₄)-Alkoxy, Hydroxycarbonyl und (C₁-C₄)-Alkoxycarbonyl substituiert sein kann
   und
worin Phenyl mit 1 bis 3 Substituenten unabhängig voneinander ausgewählt aus der Gruppe Halogen, Cyano, Nitro, (C₁-C₄)-Alkyl, Trifluormethyl, Hydroxy, Hydroxymethyl, (C₁-C₄)-Alkoxy, Trifluormethoxy, (C₁-C₄)-Alkoxymethyl, Hydroxycarbonyl und (C₁-C₄)-Alkoxycarbonyl substituiert sein kann,
und
wobei (C₃-C₇)-Cycloalkyl mit 1 oder 2 Substituenten unabhängig voneinander ausgewählt aus der Gruppe Fluor, (C₁-C₄)-Alkyl, (C₁-C₄)-Alkoxy, Hydroxy, Amino und Oxo substituiert sein kann,
- R²: für Phenyl, Naphthyl, Thienyl, Benzothienyl, Furyl oder Benzofuryl steht,
wobei Phenyl, Naphthyl, Thienyl, Benzothienyl, Furyl und Benzofuryl mit 1 bis 3 Substituenten unabhängig voneinander ausgewählt aus der Gruppe Halogen, Cyano, Nitro, (C₁-C₄)-Alkyl, Trifluormethyl, Hydroxy, (C₁-C₄)-Alkoxy, Trifluormethoxy und Phenyl substituiert sein können,
worin Phenyl mit 1 oder 2 Substituenten unabhängig von einander ausgewählt aus der Gruppe Halogen, Cyano, Nitro, (C₁-C₄)-Alkyl, Trifluormethyl, Hydroxy, (C₁-C₄)-Alkoxy, Trifluormethoxy, Hydroxy-(C₁-C₄)-alkyl und (C₁-C₄)-Alkylthio substituiert sein kann,
- R³: für Hydroxy oder -NR⁶R⁷ steht,
wobei
R⁶ für Wasserstoff oder (C₁-C₄)-Alkyl steht,
R⁷ für Wasserstoff, (C₁-C₄)-Alkyl oder (C₃-C₇)-Cycloalkyl steht,
- R⁴: für Phenyl steht,
wobei Phenyl mit 1 bis 3 Substituenten unabhängig voneinander ausgewählt aus der Gruppe Halogen, Cyano, Nitro, (C₁-C₄)-Alkyl, Difluormethyl, Trifluormethyl, Hydroxy, (C₁-C₄)-Alkoxy, Difluormethoxy, Trifluormethoxy und Phenyl substituiert sein kann,
worin Phenyl mit 1 oder 2 Substituenten unabhängig von einander ausgewählt aus der Gruppe Halogen, Cyano, Nitro, (C₁-C₄)-Alkyl, Trifluormethyl, Hydroxy, (C₁-C₄)-Alkoxy, Trifluormethoxy, Hydroxy-(C₁-C₄)-alkyl und (C₁-C₄)-Alkylthio substituiert sein kann,
- R⁵: für Trifluormethyl, (C₁-C₄)-Alkyl oder (C₃-C₇)-Cycloalkyl steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die vorliegende Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Trisethanolamin, Dicyclohexylamin, Dimethylaminoethanol, Prokain, Dibenzylamin, N-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:

Alkyl steht im Rahmen der Erfindung für einen linearen oder verzweigten Alkylrest mit 1 bis 6 bzw. 1 bis 4 Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl und 2-Ethylbutyl.

Hydroxy-alkyl steht im Rahmen der Erfindung für einen linearen oder verzweigten Alkylrest mit 1 bis 4 Kohlenstoffatomen, der in der Kette oder endständig eine Hydroxy-Gruppe als Substituenten trägt. Beispielhaft und vorzugsweise seien genannt: Hydroxymethyl, 1-Hydroxyethyl, 2-Hydroxyethyl, 1-Hydroxy-1-methylethyl, 1,1-Dimethyl-2-hydroxyethyl, 1-Hydroxypropyl, 2-Hydroxypropyl, 3-Hydroxypropyl, 1-Hydroxy-2-methylpropyl, 2-Hydroxy-1-methylpropyl, 2-Hydroxy-2-methylpropyl, 1-Hydroxybutyl, 2-Hydroxybutyl, 3- Hydroxybutyl und 4-Hydroxybutyl.

Cycloalkyl steht in Rahmen der Erfindung für einen monocyclischen, gesättigten Alkylrest mit 3 bis 7 bzw. 3 bis 6 Kohlenstoffatomen. Beispielhaft und vorzugsweise seien genannt: Cyclopropyl, Cyclobutyl, Cyclopentyl, Cyclohexyl und Cycloheptyl.

Alkenyl steht im Rahmen der Erfindung für einen linearen oder verzweigten Alkenylrest mit 2 bis 6 Kohlenstoffatomen und einer oder zwei Doppelbindungen. Bevorzugt ist ein geradkettiger oder verzweigter Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.

Alkinyl steht im Rahmen der Erfindung für einen linearen oder verzweigten Alkinylrest mit 2 bis 6 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.

Alkoxy steht im Rahmen der Erfindung für einen linearen oder verzweigten Alkoxyrest mit 1 bis 6 bzw. 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy und tert.-Butoxy.

Alkylthio steht im Rahmen der Erfindung für eine Thio-Gruppe mit einem linearen oder verzweigten Alkylsubstituenten, der 1 bis 4 Kohlenstoffatome aufweist. Beispielhaft und vorzugsweise seien genannt: Methylthio, Ethylthio, n-Propylthio, Isopropylthio, n-Butylthio und *tert.-*Butylthio.

Alkoxycarbonyl stehen im Rahmen der Erfindung für einen linearen oder verzweigten Alkoxyrest mit 1 bis 6 Kohlenstoffatomen und einer am Sauerstoff angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.

Halogen schließt im Rahmen der Erfindung Fluor, Chlor, Brom und Iod ein. Bevorzugt sind Chlor oder Fluor.

Eine Oxo-Gruppe steht im Rahmen der Erfindung für ein Sauerstoffatom, das über eine Doppelbindung an ein Kohlenstoffatom gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt sind im Rahmen der vorliegenden Erfindung Verbindungen der Formel (I), in welcher
- R¹: für (C₁-C₆)-Alkyl, (C₂-C₆)-Alkenyl oder (C₃-C₆)-Cycloalkyl steht,
wobei (C₁-C₆)-Alkyl und (C₂-C₆)-Alkenyl mit 1 bis 3 Substituenten unabhängig voneinander ausgewählt aus der Gruppe Fluor, Chlor, Cyano, Oxo, Hydroxy, Trifluormethyl, Cyclopropyl, Cyclobutyl, Methoxy, Ethoxy, Trifluormethoxy und Phenyl substituiert sein können,
worin Phenyl mit 1 bis 3 Substituenten unabhängig voneinander ausgewählt aus der Gruppe Fluor, Chlor, Cyano, Methyl, Ethyl, Trifluormethyl, Hydroxy, Hydroxymethyl, Methoxy, Ethoxy, Trifluormethoxy, Methoxymethyl, Ethoxymethyl, Hydroxycarbonyl, Methoxycarbonyl und Ethoxycarbonyl substituiert sein kann,
und
wobei (C₃-C₆)-Cycloalkyl mit 1 oder 2 Substituenten unabhängig voneinander ausgewählt aus der Gruppe Fluor, Methyl, Ethyl, Methoxy, Ethoxy, Hydroxy, Amino und Oxo substituiert sein kann,
- R²: für Phenyl oder Thienyl steht,
wobei Phenyl und Thienyl mit 1 oder 2 Substituenten unabhängig voneinander ausgewählt aus der Gruppe Fluor, Chlor, Cyano, Methyl, Ethyl, Trifluormethyl, Hydroxy, Methoxy, Ethoxy und Trifluormethoxy substituiert sein können,
- R³: für Hydroxy oder -NR⁶R⁷ steht,
wobei
R⁶ für Wasserstoff oder (C₁-C₄)-Alkyl steht,
R⁷ für Wasserstoff, (C₁-C₄)-Alkyl oder (C₃-C₅)-Cycloalkyl steht,
- R⁴: für Phenyl steht,
wobei Phenyl mit 1 bis 3 Substituenten unabhängig voneinander ausgewählt aus der Gruppe Fluor, Chlor, Cyano, Methyl, Ethyl, Difluormethyl, Trifluormethyl, Methoxy, Ethoxy, Difluormethoxy und Trifluormethoxy substituiert sein kann,
- R⁵: für Trifluormethyl, Methyl, Ethyl, iso-Propyl oder Cyclopropyl steht,
sowie ihre Salze, Solvate und Solvate der Salze.

Besonders bevorzugt sind im Rahmen der vorliegenden Erfindung Verbindungen der Formel (I), in welcher
- R¹: für (C₁-C₆)-Alkyl, (C₂-C₆)-Alkenyl oder Cyclopropyl steht,
wobei (C₁-C₆)-Alkyl und (C₂-C₆)-Alkenyl mit 1 oder 2 Substituenten unabhängig voneinander ausgewählt aus der Gruppe Fluor, Oxo, Hydroxy, Trifluormethyl, Cyclopropyl und Phenyl substituiert sein können,
worin Phenyl mit 1 oder 2 Substituenten unabhängig voneinander ausgewählt aus der Gruppe Fluor, Chlor, Methyl und Methoxy substituiert sein kann,
- R²: für Phenyl steht,
wobei Phenyl mit 1 oder 2 Substituenten unabhängig voneinander ausgewählt aus der Gruppe Fluor, Chlor, Methyl, Methoxy und Trifluormethoxy substituiert sein kann,
- R³: für Hydroxy oder -NR⁶R⁷ steht,
wobei
R⁶ für Wasserstoff oder Methyl steht,
R⁷ für Wasserstoff, Methyl oder Cyclopropyl steht,
- R⁴: für Phenyl steht,
wobei Phenyl mit 1 oder 2 Substituenten unabhängig voneinander ausgewählt aus der Gruppe Fluor, Chlor, Methyl, Trifluormethyl, Methoxy und Trifluormethoxy substituiert sein kann,
- R⁵: für Methyl oder Ethyl steht,
sowie ihre Salze, Solvate und Solvate der Salze.
Weiterhin besonders bevorzugt sind im Rahmen der vorliegenden Erfindung Verbindungen der Formel (I), in welcher
- R¹: für (C₂-C₄)-Alkyl, (C₂-C₄)-Alkenyl oder Cyclopropyl steht,
wobei (C₂-C₄)-Alkyl und (C₂-C₄)-Alkenyl mit 1 oder 2 Substituenten unabhängig voneinander ausgewählt aus der Gruppe Fluor, Oxo, Hydroxy, Trifluormethyl, Cyclopropyl und Phenyl substituiert sein können,
worin Phenyl mit 1 oder 2 Substituenten unabhängig voneinander ausgewählt aus der Gruppe Fluor, Chlor, Methyl und Methoxy substituiert sein kann,
- R²: für Phenyl steht,
wobei Phenyl mit 1 oder 2 Substituenten unabhängig voneinander ausgewählt aus der Gruppe Fluor, Chlor, Methyl, Methoxy und Trifluormethoxy substituiert sein kann,
- R³: für Hydroxy oder -NH₂ steht,
- R⁴: für Phenyl steht,
wobei Phenyl mit 1 oder 2 Substituenten unabhängig voneinander ausgewählt aus der Gruppe Fluor, Chlor, Methyl, Trifluormethyl, Methoxy und Trifluormethoxy substituiert sein kann,
- R⁵: für Methyl oder Ethyl steht,
sowie ihre Salze, Solvate und Solvate der Salze.

Weiterhin besonders bevorzugt sind im Rahmen der vorliegenden Erfindung Verbindungen der Formel (I), in welcher
- R¹: für 3,3,3-Trifluorprop-2-en-1-yl, 3,3,3-Trifluorpropyl oder 1,1,1-Trifluorpropan-2-ol-3-yl steht,
- R²: für Phenyl steht,
wobei Phenyl mit 1 oder 2 Substituenten unabhängig voneinander ausgewählt aus der Gruppe Fluor, Chlor, Methyl, Methoxy und Trifluormethoxy substituiert sein kann,
- R³: für Hydroxy oder -NH₂ steht,
- R⁴: für Phenyl steht,
wobei Phenyl mit 1 oder 2 Substituenten unabhängig voneinander ausgewählt aus der Gruppe Fluor, Chlor, Methyl, Trifluormethyl, Methoxy und Trifluormethoxy substituiert sein kann,
- R⁵: für Methyl oder Ethyl steht,
sowie ihre Salze, Solvate und Solvate der Salze.

Bevorzugt sind im Rahmen der vorliegenden Erfindung auch Verbindungen der Formel (I), in welcher R² für p-Chlorphenyl steht.

Bevorzugt sind im Rahmen der vorliegenden Erfindung auch Verbindungen der Formel (I), in welcher R⁵ für Trifluormethyl, Methyl oder Ethyl steht,

Bevorzugt sind im Rahmen der vorliegenden Erfindung auch Verbindungen der Formel (I), in welcher R³ für Amino steht.

Bevorzugt sind im Rahmen der vorliegenden Erfindung auch Verbindungen der Formel (I), in welcher R³ für Hydroxy steht.

Bevorzugt sind im Rahmen der vorliegenden Erfindung auch Verbindungen der Formel (I), in welcher

R³ für -NR⁶R⁷ steht,
wobei
- R⁶: für Wasserstoff oder Methyl steht,
- R⁷: für Wasserstoff, Methyl oder Cyclopropyl steht.

Bevorzugt sind im Rahmen der vorliegenden Erfindung auch Verbindungen der Formel (I), in welcher R¹ für 3,3,3-Trifluorprop-2-en-1-yl steht.

Bevorzugt sind im Rahmen der vorliegenden Erfindung auch Verbindungen der Formel (I), in welcher R¹ für 3,3,3-Trifluorpropyl steht.

Bevorzugt sind im Rahmen der vorliegenden Erfindung auch Verbindungen der Formel (I), in welcher R¹ für 1,1,1-Trifluorpropan-2-ol-3-yl steht.

Bevorzugt sind im Rahmen der vorliegenden Erfindung auch Verbindungen der Formel (I), in welcher
- R¹: für (C₂-C₄)-Alkyl oder (C₂-C₄)-Alkenyl steht,
wobei (C₂-C₄)-Alkyl und (C₂-C₄)-Alkenyl mit 1 oder 2 Substituenten unabhängig voneinander ausgewählt aus der Gruppe Fluor, Hydroxy, Oxo und Trifluormethyl substituiert sind.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man
[A] eine Verbindung der Formel (II) 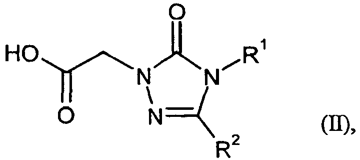 in welcher R¹ und R² jeweils die oben angegebenen Bedeutungen haben,
   in einem inerten Lösungsmittel unter Aktivierung der Carbonsäure-Funktion mit einer Verbindung der Formel (III) 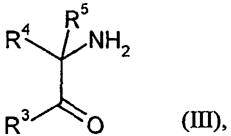 in welcher R³, R⁴ und R⁵ jeweils die oben angegebenen Bedeutungen haben,
   kuppelt
   oder
[B] eine Verbindung der Formel (IV) 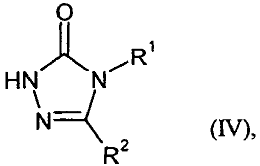 in welcher R¹ und R² jeweils die oben angegebenen Bedeutungen haben,
   in einem inerten Lösungsmittel in Gegenwart einer Base mit einer Verbindung der Formel (V) 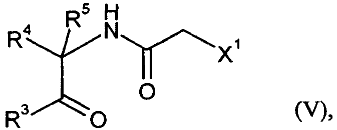 in welcher R³, R⁴ und R⁵ jeweils die oben angegebenen Bedeutungen haben
   und
   - X¹: für eine Abgangsgruppe, wie beispielsweise Halogen, Mesylat oder Tosylat, steht,
   umsetzt
   oder
[C] eine Verbindung der Formel (I-A) 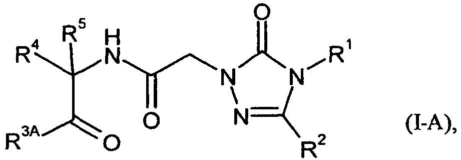 in welcher R¹, R², R⁴ und R⁵ jeweils die oben angegebenen Bedeutungen haben
   und
   - R^{3A}: für Hydroxy steht,
   in einem inerten Lösungsmittel unter Aktivierung der Carbonsäure-Funktion mit einem Amin der Formel (VI) 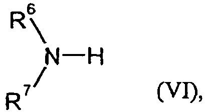 in welcher R⁶ und R⁷ jeweils die oben angegebenen Bedeutungen haben
   umsetzt
und die resultierenden Verbindungen der Formel (I) gegebenenfalls mit den entsprechenden (i) Lösungsmitteln und/oder (ii) Basen oder Säuren in ihre Solvate, Salze und/oder Solvate der Salze überführt.

Inerte Lösungsmittel für den Verfahrensschritte (II) + (III) bzw. (I-A) + (VI) → (I) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N,N*-Dimethylformamid, *N,N'*-Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan, Tetrahydrofuran, Dimethylformamid, Dimethylsulfoxid oder Gemische dieser Lösungsmittel.

Als Kondensationsmittel für die Amidbildung im Verfahrensschritt (II) + (III) bzw. (I-A) + (VI) → (I) eignen sich beispielsweise Carbodiimide wie *N,N'*-Diethyl-, *N,N'*-Dipropyl-, *N,N'*-Diisopropyl-, *N,N*'-Dicyclohexylcarbodiimid (DCC) oder *N*-(3-Dimethylaminoisopropyl)-*N*'-ethylcarbodiimid-Hydrochlorid (EDC), Phosgen-Derivate wie *N,N*'-Carbonyldiimidazol (CDI), 1,2-Oxazoliumverbindungen wie 2-Ethyl-5-phenyl-1,2-oxazolium-3-sulfat oder 2-*tert*.-Butyl-5-methyl-isoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat, Benzotriazol-1-yloxy-tris(pyrrolidino)phosphonium-hexafluorophosphat (PyBOP), *O*-(Benzotriazol-1-yl)-*N,N,N',N'*-tetramethyluronium-tetrafluoroborat (TBTU), *O-*(Benzotriazol-1-yl)-*N,N,N',N'*-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N,N,N',N'*-tetramethyluronium-hexafluorophosphat (HATU) oder *O*-(1*H*-6-Chlorbenzotriazol-1-yl)-1,1,3,3-tetramethyluronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, *N*-Methyl-piperidin oder *N,N*-Diisopropylethylamin. Bevorzugt wird EDC in Kombination mit HOBt oder TBTU in Verbindung mit *N,N*-Diisopropylethylamin verwendet.

Ebenfalls kann die Aktivierung der Carbonsäure-Funktion durch Umwandlung zum Säurechlorid, entweder in situ oder als separater Syntheseschritt stattfinden. Dafür eignen sich zum Beispiel Sulfonylchlorid oder 1-Chlor-N,N,2-trimethylprop-1-en-1-amin.

Die Kondensation (II) + (III) bzw. (I-A) + (VI) → (I) wird im Allgemeinen in einem Temperaturbereich von -20°C bis +60°C, bevorzugt bei 0°C bis +40°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (IV) + (V) → (I) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Aceton, Methylethylketon, Essigsäureethylester, Acetonitril, *N,N*-Dimethylformamid, Dimethylsulfoxid, *N,N'-*Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Acetonitril, Aceton oder Dimethylformamid verwendet.

Als Basen für den Verfahrensschritt (IV) + (V) → (I) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid, oder organische Amine wie Triethylamin, *N*-Methylmorpholin, *N-*Methylpiperidin, *N,N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Kalium- oder Cäsiumcarbonat verwendet.

Die Base wird hierbei in einer Menge von 1 bis 5 Mol, bevorzugt in einer Menge von 1 bis 2.5 Mol, bezogen auf 1 Mol der Verbindung der Formel (IV), eingesetzt. Die Reaktion erfolgt im Allgemeinen in einem Temperaturbereich von 0°C bis +100°C, bevorzugt bei +20°C bis +80°C. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Die Herstellung der erfindungsgemäßen Verbindungen kann durch die folgenden Syntheseschemata veranschaulicht werden:

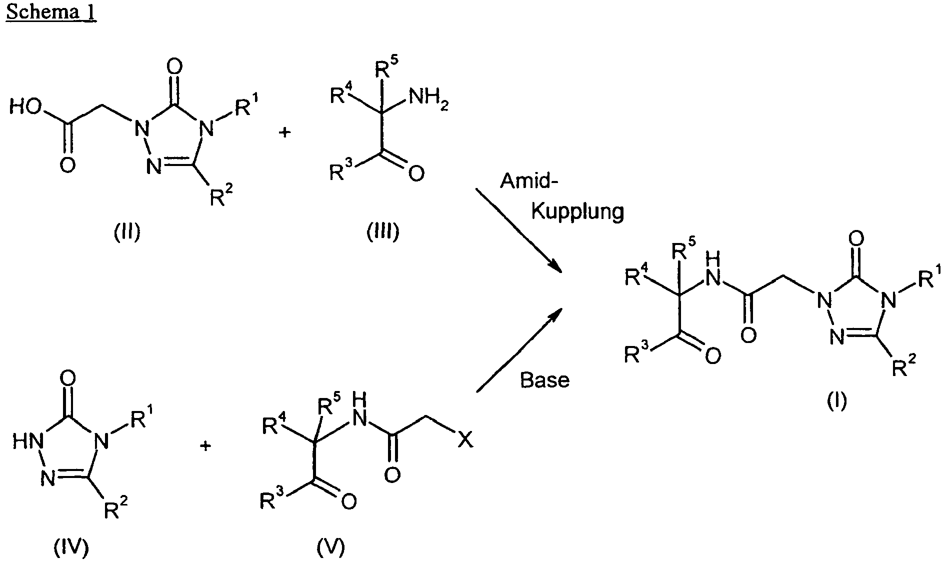

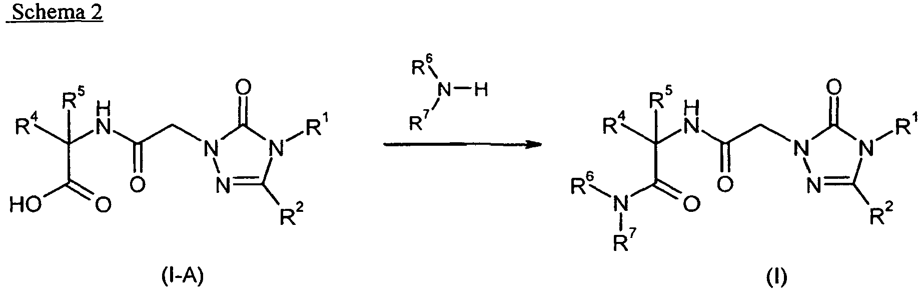

Die Verbindungen der Formel (II) können durch baseninduzierte Alkylierung von 5-Aryl-2,4-dihydro-3*H*-1,2,4-triazol-3-onen der Formel (IV) zu den *N*²-substituierten Verbindungen (VIII) und nachfolgende Ester-Hydrolyse erhalten werden (siehe Schema 3):

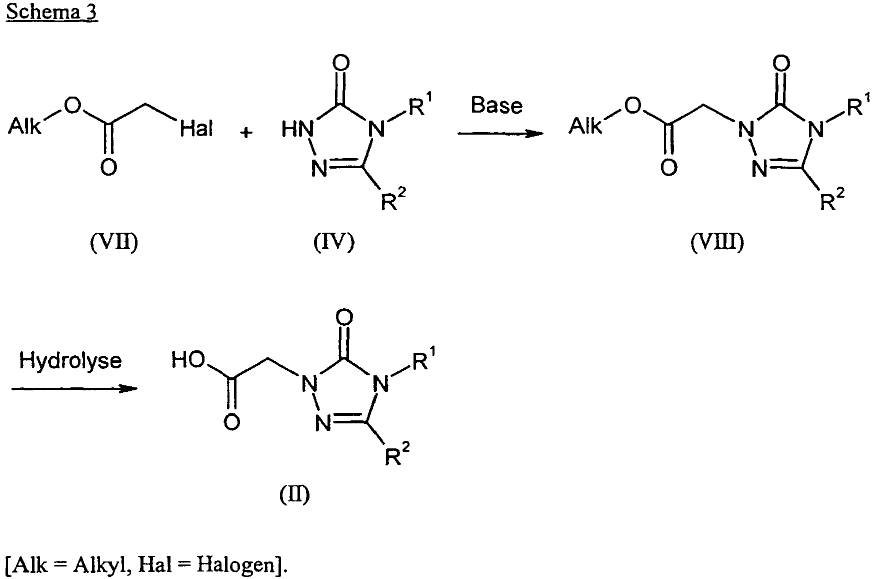

Die Verbindungen der Formel (VIII) können alternativ auch aus literaturbekannten *N*-(Alkoxycarbonyl)arylthioamiden der Formel (X) [siehe z.B. M. Amswald, W.P. Neumann, J. Org. Chem. 58 (25), 7022-7028 (1993); E.P. Papadopoulos, J. Org. Chem. 41 (6), 962-965 (1976)] durch Reaktion mit Hydrazino-Estern der Formel (IX) und nachfolgende Alkylierung an N-4 des Triazolons (XI) hergestellt werden (Schema 4):

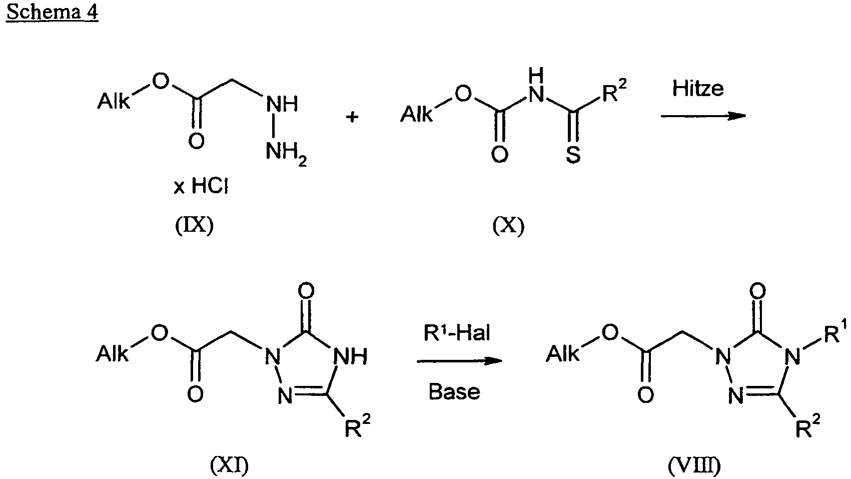

Die Verbindungen der Formel (IV) können ausgehend von Carbonsäurehydraziden der Formel (XII) durch Umsetzung mit Isocyanaten der Formel (XIII) oder Nitrophenylcarbamaten der Formel (XIV) und nachfolgende baseninduzierte Cyclisierung der intermediären Hydrazincarboxamide (XV) hergestellt werden (Schema 5):

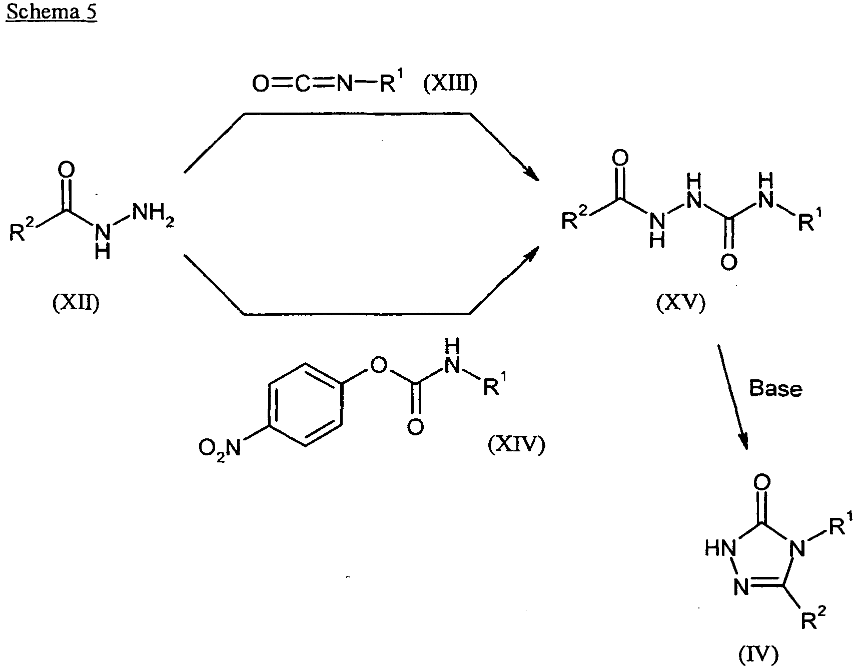

Die Verbindung, in der R¹ dem Substituenten CH₂CH(OH)CF₃ entspricht, wird dem Schema 4 zunächst folgend aus Alkylisocyanatoacetat (XIIIa) und (XII) zu (XVa) umgesetzt. Nachfolgende basische Zyklisierung führt zum Triazolon (IVa). Einführung der CF₃- Gruppe erfolgt durch Umsetzung von (IVa) mit Trifluoressigsäureanhydrid in Pyridin. Das resultierende Keton (IVb) kann dann durch Reduktion nach (IVc) überführt werden (Schema 6):

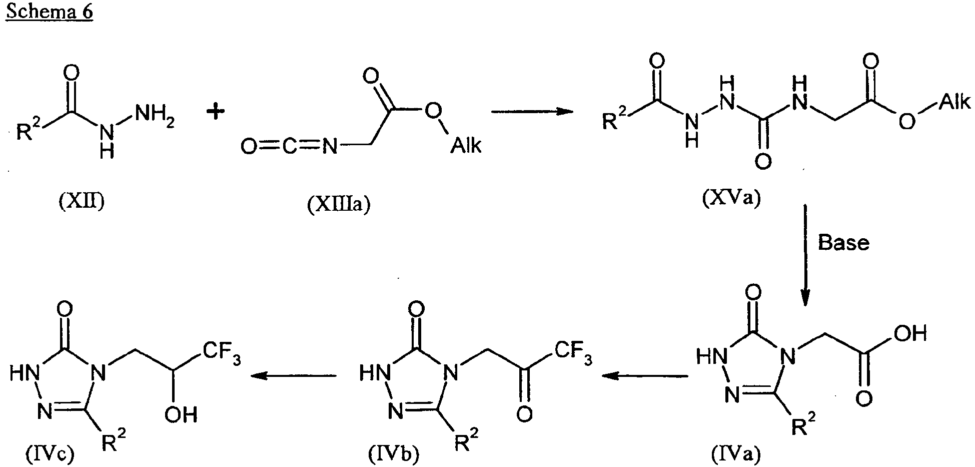

Die Verbindungen der Formeln (III), (V), (VI), (VII), (IX), (X), (XII), (XIII), (XIIIa) und (XIV) sind vielfältig kommerziell verfügbar, literaturbekannt oder nach allgemein bekannten Verfahren zugänglich.

Weitere erfindungsgemäße Verbindungen können gegebenenfalls auch hergestellt werden durch Umwandlungen von funktionellen Gruppen einzelner Substituenten, insbesondere den unter R¹ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, insbesondere Bildung von Carbonsäureamiden, sowie Einführung und Entfernung temporärer Schutzgruppen.

Die erfindungsgemäßen Verbindungen besitzen wertvolle pharmakologische Eigenschaften und können zur Vorbeugung und/oder Behandlung von verschiedenen Erkrankungen und krankheitsbedingten Zuständen bei Menschen und Tieren verwendet werden.

Bei den erfindungsgemäßen Verbindungen handelt es sich um potente, selektive duale V1a/V2-Rezeptor-Antagonisten, die die Vasopressin-Aktivität *in vitro* und *in vivo* inhibieren, und eine verbesserte Wirkung auf beiden Vasopressin-Rezeptoren aufweisen.

Die erfindungsgemäßen Verbindungen sind insbesondere für die Prophylaxe und/oder Behandlung von kardiovaskulären Erkrankungen geeignet. In diesem Zusammenhang seien als Zielindikationen beispielhaft und vorzugsweise genannt: akute und chronische Herzinsuffizienz, arterielle Hypertonie, koronare Herzerkrankung, stabile und instabile Angina pectoris, myokardiale Ischämie, Myokardinfarkt, Schock, Arteriosklerose, atriale und ventrikuläre Arrhythmien, transitorische und ischämische Attacken, Hirnschlag, entzündliche kardiovaskuläre Erkrankungen, periphere und kardiale Gefäßerkrankungen, periphere Durchblutungsstörungen, arterielle pulmonale Hypertonie, Spasmen der Koronararterien und peripherer Arterien, Thrombosen, thromboembolische Erkrankungen, Ödembildung wie zum Beispiel pulmonales Ödem, Hirnödem, renales Ödem oder Herzinsuffizienz-bedingtes Ödem, sowie Restenosen wie nach Thrombolysetherapien, percutan-transluminalen Angioplastien (PTA), transluminalen Koronarangioplastien (PTCA), Herztransplantationen und Bypass-Operationen.

Im Sinne der vorliegenden Erfindung umfasst der Begriff Herzinsuffizienz auch spezifischere oder verwandte Krankheitsformen wie Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Weiterhin eignen sich die erfindungsgemäßen Verbindungen zur Verwendung als Diuretikum für die Behandlung von Ödemen und bei Elektrolytstörungen, insbesondere bei der hypervolämischen und euvolämischen Hyponaträmie.

Die erfindungsgemäßen Verbindungen sind weiter geeignet für die Prophylaxe und/oder Behandlung der polyzystischen Nierenkrankheit (PCKD) und des Syndroms der inadäquaten ADH-Sekretion (SIADH).

Außerdem können die erfindungsgemäßen Verbindungen für die Prophylaxe und/oder Behandlung der Leberzirrhose, des Aszites, von Diabetes mellitus und diabetischen Folgeerkrankungen, wie z.B. Neuropathie und Nephropathie, von akutem und chronischem Nierenversagen sowie von chronischer Niereninsuffizienz verwendet werden.

Ferner eignen sich die erfindungsgemäßen Verbindungen für die Prophylaxe und/oder Behandlung zentralnervöser Störungen wie Angstzuständen und Depressionen, von Glaukom sowie von Krebs, insbesondere von Lungentumoren.

Darüber hinaus können die erfindungsgemäßen Verbindungen für die Prophylaxe und/oder Behandlung von entzündlichen Erkrankungen, asthmatischen Erkrankungen, chronisch-obstruktiven Atemwegserkrankungen (COPD), Schmerzzuständen, Prostatahypertrophien, Inkontinenz, Blasenentzündung, hyperaktiver Blase, Erkrankungen der Nebenniere wie zum Beispiel Phäochromozytom und Nebennierenapoplexie, Erkrankungen des Darms wie zum Beispiel Morbus Crohn und Diarrhoe, oder von Menstruationsstörungen wie zum Beispiel Dysmenorrhöen oder von Endometriose eingesetzt werden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, die mindestens eine der erfindungsgemäßen Verbindungen sowie einen oder mehrere weitere Wirkstoffe enthalten, insbesondere zur Behandlung und/oder Prophylaxe der zuvor genannten Erkrankungen. Als hierfür geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Diuretika, insbesondere Schleifendiuretika sowie Thiazide und Thiazid-ähnliche Diuretika;
- positiv-inotrop wirksame Verbindungen, wie beispielsweise Herzglycoside (Digoxin), betaadrenerge und dopaminerge Agonisten wie Isoproterenol, Adrenalin, Noradrenalin, Dopamin und Dobutamin;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, sowie PDE 3-Inhibitoren wie Amrinone und Milrinone;
- natriuretische Peptide, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Calcium-Sensitizer, wie beispielhaft und vorzugsweise Levosimendan;
- NO- und Häm-unabhängige Aktivatoren der Guanylatcyclase, wie insbesondere Cinaciguat sowie die in WO 01/19355, WO 01/19776, WO 01/19778, WO 01/19780, WO 02/070462 und WO 02/070510 beschriebenen Verbindungen;
- NO-unabhängige, jedoch Häm-abhängige Stimulatoren der Guanylatcyclase, wie insbesondere Riociguat sowie die in WO 00/06568, WO 00/06569, WO 02/42301 und WO 03/095451 beschriebenen Verbindungen;
- Inhibitoren der humanen neutrophilen Elastase (HNE), wie beispielsweise Sivelestat oder DX-890 (Reltran);
- die Signaltransduktionskaskade inhibierende Verbindungen, wie beispielsweise Tyrosinkinase-Inhibitoren, insbesondere Sorafenib, Imatinib, Gefitinib und Erlotinib;
- den Energiestoffwechsel des Herzens beeinflussende Verbindungen, wie beispielhaft und vorzugsweise Etomoxir, Dichloracetat, Ranolazine oder Trimetazidine;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Vasopeptidase-Inhibitoren, Inhibitoren der neutralen Endopeptidase, Endothelin-Antagonisten, Renin-Inhibitoren, alpha-Rezeptoren-Blocker, beta-Rezeptoren-Blocker, Mineralocorticoid-Rezeptor-Antagonisten und Rho-Kinase-Inhibitoren; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten. Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Vasopeptidase-Inhibitoren, Inhibitoren der neutralen Endopeptidase, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Vasopeptidase-Inhibitor bzw. Inhibitor der neutralen Endopeptidase (NEP) verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton, Eplerenon, Canrenon oder Kalium-Canrenoat, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 10 mg/kg, vorzugsweise etwa 0.01 bis 1 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- BOC: *tert*.-Butoxycarbonyl
- CI: chemische Ionisation (bei MS)
- DCI: direkte chemische Ionisation (bei MS)
- DME: 1,2-Dimethoxyethan
- DMF: Dimethylformamid
- DMSO: Dimethylsulfoxid
- d. Th.: der Theorie (bei Ausbeute)
- EDC: *N*'-(3-Dimethylaminopropyl)-*N*-ethylcarbodiimid-Hydrochlorid
- eq.: Äquivalent(e)
- ESI: Elektrospray-Ionisation (bei MS)
- GC/MS: Gaschromatographie-gekoppelte Massenspektrometrie
- ges.: gesättigt
- h: Stunde(n)
- HOBt: 1-Hydroxy-1*H*-benzotriazol-Hydrat
- HPLC: Hochdruck-, Hochleistungsflüssigchromatographie
- HV: Hochvakuum
- konz.: konzentriert
- LC/MS: Flüssigchromatographie-gekoppelte Massenspektrometrie
- LDA: Lithiumdiisopropylamid
- LiHMDS: Lithiumhexamethyldisilazan
- min: Minute(n)
- MS: Massenspektrometrie
- MTBE: Methyl-tert.-butylether
- NMR: Kernresonanzspektrometrie
- rac: racemisch / Racemat
- R_{f}: Retentionsfaktor (bei Dünnschichtchromatographie auf Kieselgel)
- RT: Raumtemperatur
- Rₜ: Retentionszeit (bei HPLC)
- THF: Tetrahydrofuran
- TMOF: Trimethylorthoformiat
- UV: Ultraviolett-Spektrometrie
- v/v: Volumen zu Volumen-Verhältnis (einer Lösung)

### LC/MS-, HPLC- und GC/MS-Methoden:

Methode 1: Gerätetyp MS: Micromass ZQ; Gerätetyp UPLC: Waters Alliance 2795; Säule: Phenomenex Synergi 2.5 µ MAX-RP 100A Mercury 20 mm x 4mm; Eluent A: 11 Wasser + 0.5 ml 50%ige Ameisensäure, Eluent B: 1 1 Acetonitril + 0.5 ml 50%ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.
Methode 2: Gerätetyp MS: Waters (Micromass) Quattro Micro; Gerätetyp HPLC: Agilent 1100 Serie; Säule : Thermo Hypersil GOLD 3 µ 20 x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100% A → 3.0 min 10% A → 4.0 min 10% A → 4.01 min 100% A (Flow 2.5 ml) → 5.00 min 100% A; Ofen: 50°C; Fluss: 2 ml/min; UV-Detektion: 210 nm.
Methode 3: Instrument: Micromass Quattro Premier mit Waters UPLC Acquity; Säule: Thermo Hypersil GOLD 1.9 µ 50 x 1 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 0.1 min 90% A → 1.5 min 10% A → 2.2 min 10% A Ofen: 50°C; Fluss: 0.33 ml/min; UV-Detektion: 210 nm.
Methode 4: Instrument: Waters ACQUITY SQD UPLC System; Säule: Waters Acquity UPLC HSS T3 1.8 µ 50 x 1 mm; Eluent A: 1 1 Wasser + 0.25 ml 99%ige Ameisensäure , Eluent B: 1 1 Acetonitril + 0.25 ml 99%ige Ameisensäure; Gradient: 0.0 min 90% A → 1.2 min 5% A → 2.0 min 5% A Ofen: 50 °C; Fluss: 0.40 ml/min; UV-Detektion: 210 - 400 nm.
Methode 5: Instrument: Waters ACQUITY SQD UPLC System; Säule: Waters Acquity UPLC HSS T3 1.8 µ 50 x 1 mm; Eluent A: 1 1 Wasser + 0.25 ml 99%ige Ameisensäure , Eluent B: 1 1 Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 210 - 400 nm.
Methode 6: Gerätetyp MS: Micromass ZQ; Gerätetyp HPLC: HP 1100 Series; UV DAD; Säule: Phenomenex Gemini 3 µ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.
Methode 7: Gerätetyp MS: Waters ZQ; Gerätetyp HPLC: Agilent 1100 Series; UV DAD; Säule: Thermo Hypersil GOLD 3 µ 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100% A → 3.0 min 10% A → 4.0 min 10% A → 4.1 min 100% Fluss: 2,5 ml/min, Ofen: 55°C; Fluss 2/ml; UV-Detektion: 210 nm.
Methode 8 (chirale präparative HPLC): Chirale stationäre Kieselgel-Phase basierend auf dem Selektor Poly(N-methacryloyl-D-Leucin-dicyclopropylmethylamid); Säule: 670 mm x 40 mm, Fluss: 80 ml/min, Temperatur: 24 °C; UV-Detektor 260 nM. Elutionsmittel iso-Hexan / Essigsäureethylester 30 : 70.
   Methode 8a: Elutionsmittel: iso-Hexan / Essigsäureethylester 10: 90 (v/v); Fluss: 50 ml/min.
Methode 9 (präparative HPLC): Chirale stationäre Kieselgel-Phase basierend auf dem Selektor Poly(N-methacryloyl-D-Leucin-dicyclopropylmethylamid); Säule: 250 mm x 4.6 mm, Elutionsmittel Essigsäureethylester 100%, Fluss: 1 ml/min, Temperatur: 24 °C; UV-Detektor 265 nM.
Methode 10 (präparative HPLC): Säule: Grom-Sil 120 ODS-4HE, 10 µm, SNr. 3331, 250 mm x 30 mm. Eluent A: Ameisensäure 0.1% in Wasser, Eluent B: Acetonitril; Fluss: 50 ml/min Programm: 0-3 min: 10% B; 3-27 min: Gradient bis 95% B; 27-34 min: 95% B; 34.01-38 min: 10% B.
Methode 11 (chirale präparative HPLC): Stationäre Phase Daicel Chiralcel OD-H, 5 µm, Säule: 250 mm x 20 mm; Temperatur: RT; UV-Detektion: 230 nm. Verschiedene Elutionsmittel:
   Methode 11a: Elutionsmittel: *iso*-Hexan / *iso*-Propanol 70: 30 (v/v); Fluss: 20 ml/min
   Methode 11b: Elutionsmittel: *iso*-Hexan / *iso*-Propanol 50: 50 (v/v); Fluss: 18 ml/min
   Methode 11c: Elutionsmittel: *iso*-Hexan/Methanol/Ethanol 70:15:15; (v/v/v); Fluss 20 ml/min
   Methode 11d: Elutionsmittel: *iso*-Hexan / *iso*-Propanol 75 : 25 (v/v); Fluss 15 ml/min
Methode 12 (analytische präparative HPLC): Stationäre Phase Daicel Chiralcel OD-H, Säule: 250 mm x 4 mm; Fluss: 1 ml/min; Temperatur: RT; UV-Detektion: 230 nm. Verschiedene Elutionsmittel:
   Methode 12a: Elutionsmittel: *iso*-Hexan / iso-Propanol 1: 1 (v/v);
   Methode 12b: Elutionsmittel: *iso*-Hexan / Methanol / Ethanol 70 : 15 : 15 (v/v/v)
   Methode 12c: Elutionsmittel: *iso*-Hexan / *iso*-Propanol 75:25 (v/v);
Methode 13 chirale präparative HPLC): Chirale stationäre Kieselgel-Phase basierend auf dem Selektor Poly(*N*-methacryloyl-D-Leucin-dicyclopropyl-methylamid); Säule: 600 mm x 30 mm, Eluent: Stufengradient Essigsäureethylester / Methanol 1:1 (0 - 17 min)→ Essigsäureethylester (17.01 min bis 21 min)→ Essigsäureethylester / Methanol 1:1 (21.01 min bis 25 min); Fluss: 80 ml/min, Temperatur: 24 °C; UV-Detektor 265 nM.
Methode 14 (chirale präparative HPLC): wie Methode 9 aber Fluss 2 ml / min.
Methode 15 (chirale präparative HPLC Chirale stationäre Kieselgel-Phase basierend auf dem Selektor Poly(*N*-methacryloyl-L-Isoeucin-3-pentylamid); Säule: 430 mm x 40 mm, Fluss: 80 ml/min, Temperatur: 24 °C; UV-Detektor 265 nM. Verschiedene Elutionsmittel:
   Methode 15a: 100% Essigsäureethylester
   Methode 15b: *iso*-Hexan / Essigsäureethylester 10 : 90
Methode 16 (chirale analytische HPLC): Chirale stationäre Kieselgel-Phase basierend auf dem Selektor Poly(*N*-methacryloyl-L-Isoeucin-3-pentylamid); Säule: 250 mm x 4.6 mm, Eluent 100% EE, Fluss 2 ml/min, Temperatur 24°C; UV-Detector 265 nM.
Methode 17 (chirale präparative HPLC): Chirale stationäre Kieselgel-Phase basierend auf dem Selektor Poly(*N*-methacryloyl-L-Leucin-(+)-3-pinanmethylamid); Säule: 600 mm x 30 mm, Fluss: 80 ml/min, Temperatur: 24 °C; UV-Detektor 265 nM. Verschiedene Elutionsmittel:
   Methode 17a: *iso*-Hexan / Essigsäureethylester 20 : 80
   Methode 17b: *iso*-Hexan / Essigsäureethylester 30 : 70
   Methode 17c: *iso*-Hexan / Essigsäureethylester 50 : 50
   Methode 17d: 100% Essigsäureethylester
   Methode 17e: *iso*-Hexan / Essigsäureethylester 40 : 60
   Methode 17f: *iso*-Hexan / Essigsäureethylester 10 : 90
Methode 18 (chirale analytische HPLC): Chirale stationäre Kieselgel-Phase basierend auf dem Selektor Poly(*N*-methacryloyl-L-Leucin-(+)-3-pinanmethylamid); Säule: 250 mm x 4.6 mm, Temperatur 24°C; UV-Detector 265 nM.
   Methode 18a: Eluent: *iso*-Hexan / Essigsäureethylester 50 : 50, Fluss: 2 ml/min.
   Methode 18b: Eluent : 100% Essigsäureethylester, Fluss: 2 ml/min.
   Methode 18c: Eluent: 100% Essigsäureethylester, Fluss: 1ml / min.
Methode 19 (präparative HPLC): Säule Grom-Sil 120 ODS-4HE 10 µm, 250 mm x 30 mm; Eluent: A = Wasser, B = Acetonitril; Gradient: 0.0 min 10% B, 3 min 10% B, 30 min 95% B, 42 min 95% B, 42.01 min 10% B, 45 min 10% B; Fluss: 50 ml/min; Säulentemperatur: RT; UV-Detektion: 210 nm.

### Ausgangsverbindungen und Intermediate:

### Beispiel 1A

### Ethyl-N-({2-[(4-chlorphenyl)carbonyl]hydrazinyl}carbonyl)glycinat

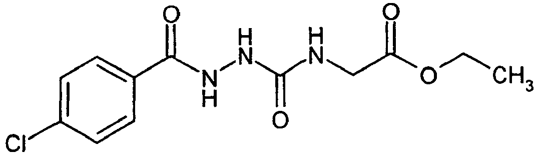

Eine Suspension von 12.95 g (75.9 mmol) 4-Chlorbenzhydrazid in 50 ml trockenem THF wurde bei 50°C vorgelegt und tropfenweise mit einer Lösung von 10.0 g (77.5 mmol) Ethyl-2-isocyanatoacetat in 100 ml trockenem THF versetzt. Zunächst bildete sich eine Lösung, dann fiel ein Niederschlag aus. Nach Ende der Zugabe wurde die Mischung noch 2 h auf 50°C weiter gerührt, dann über Nacht bei RT stehen lassen. Die Kristalle wurden durch Filtration isoliert, mit wenig Diethylether gewaschen und am HV getrocknet. 21.43 g (89 % d. Th) der Titelverbindung wurden erhalten.
LC/MS [Methode 1]: Rₜ = 1.13 min.; m/z = 300 (M+H)⁺
¹H NMR (DMSO-d₆, 400MHz): δ [ppm] = 10.29 (s, 1H), 8.21 (s, 1H), 7.91 (d, 2H), 7.57 (d, 2H), 6.88 (br.s, 1H), 4.09 (q, 2H), 3.77 (d, 2H), 1.19 (t, 3H)

### Beispiel 2A

### [3-(4-Chlorphenyl)-5-oxo-1,5-dihydro-4H-1,2,4-triazol-4-yl]essigsäure

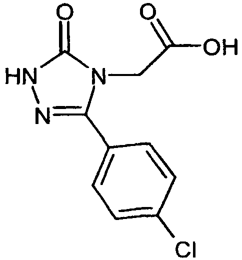

21.43 g (67.93 mmol) der Verbindung aus Beispiel 1A wurden mit 91 ml einer 3N Natronlauge versetzt und auf Rückfluss über Nacht erhitzt. Nach Abkühlen auf RT wurde die Mischung durch langsame Zugabe ca. 20%igen Salzsäure auf pH 1 eingestellt. Der ausgefallene Feststoff wurde durch Filtration isoliert, mit Wasser gewaschen und bei 60°C in Vakuum getrocknet. Ausbeute: 17.55 g (90% d. Th., ca. 88% Reinheit) der Titelverbindung.
LC/MS [Methode 1]: Rₜ = 0.94 min.; m/z = 254 (M+H)⁺
¹H NMR (DMSO-d₆, 400MHz): δ [ppm] = 13.25 (br.s, 1H), 12.09 (s, 1H), 7.65 - 7.56 (m, 4H), 4.45 (s, 2H).

### Beispiel 3A

### 5-(4-Chlorphenyl)-4-(3,3,3-trifluor-2-oxopropyl)-2,4-dihydro-3H-1,2,4-triazol-3-on (oder als Hydrat: 5-(4-Chlorphenyl)-4-(3,3,3-trifluor-2,2-dihydroxypropyl)-2,4-dihydro-3H-1,2,4-triazol-3-on)

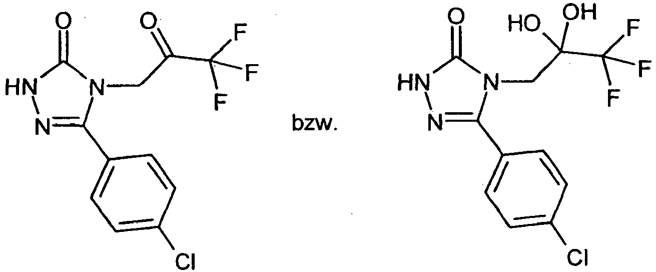

5 g (16.36 mmol) der Verbindung aus Beispiel 2A wurden unter Argon in 200 ml Pyridin gelöst, dann mit 17.18 g (81.8 mmol) Trifluoressigsäureanhydrid versetzt. Dabei stieg die Temperatur auf ca. 35°C. Nach 30 min wurde das Pyridin am Rotationsverdampfer entfernt und der Rückstand mit 1.5 L 0.5N Salzsäure verdünnt. Diese Mischung wurde auf 70°C erwärmt dann warm filtriert. Der Feststoff wurde mit etwas Wasser gewaschen. Das gesamte Filtrat wurde dreimal mit Essigsäureethylester extrahiert. Die vereinigten organischen Phasen wurden mit Wasser, dann einer gesättigter wässriger Natriumhydrogencarbonatlösung, dann mit einer gesättigter wässriger Natriumchloridlösung gewaschen, über Natriumsulfat getrocknet und am Rotationsverdampfer vom Lösungsmittel befreit. Der Rückstand wurde am HV getrocknet. Ausbeute: 3.56 g (68% d. Th.) der Titelverbindung als Hydrat.
LC/MS [Methode 1]: Rₜ = 1.51 min.; m/z = 306 (M+H)⁺ und 324 (M+H⁺)(Keton bzw. Hydrat)
¹H NMR (DMSO-d₆ ,400MHz): δ [ppm] = 12.44 (s, 1H), 7.72 (d, 2H), 7.68 (br.s, 2H), 7.61 (d, 2H), 3.98 (s, 2H).

### Beispiel 4A

### 5-(4-Chlorphenyl)-4-(3,3,3-trifluor-2-hydroxypropyl)-2,4-dihydro-3H-1,2,4-triazol-3-on

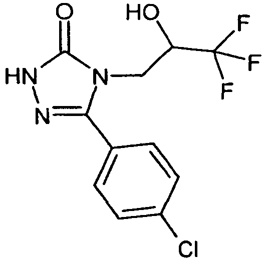

3.56 g (11 mmol) der Verbindung aus Beispiel 3A wurden in 100 ml Methanol gelöst und unter Eiskühlung mit 3.75 g Natriumborhydrid (99 mmol) versetzt (Gasentwicklung). Nach 1.5 h wurden langsam 200 ml 1M Salzsäure zugegeben. Das Methanol wurde am Rotationsverdampfer entfernt, der Rückstand mit 500 ml Wasser verdünnt und dreimal mit Essigsäureethylester extrahiert. Die vereinigten organischen Phasen wurden mit einer gesättigten wässrigen Natriumhydrogencarbonatlösung, dann mit einer gesättigten wässrigen Natriumchloridlösung gewaschen, über Natriumsulfat getrocknet und am Rotationsverdampfer vom Lösungsmittel befreit. Der Rückstand wurde am HV getrocknet. 3.04 g (90% d. Th.) der Titelverbindung wurden erhalten.
LC/MS [Methode 2]: Rₜ = 1.80 min.; m/z = 308 (M+H)⁺.
¹H NMR (DMSO-d₆ ,400MHz): δ [ppm] = 12.11 (s, 1H), 7.75 (d, 2H), 7.62 (d, 2H), 6.85 (d, 1H), 4.34 - 4.23 (m, 1H), 3.92 (dd, 1H), 3.77 (dd, 1H).

### Beispiel 5A

### {3-(4-Chlorphenyl)-5-oxo-4-(3,3,3-trifluor-2-hydroxypropyl)-4,5-dihydro-1H-1,2,4-triazol-1-yl}-essigsäuremethylester

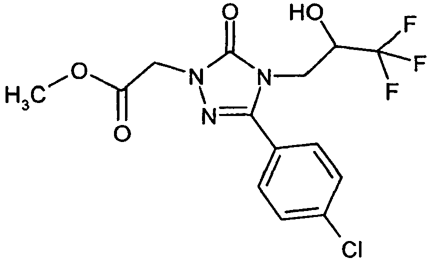

3.04 g (9.9 mmol) der Verbindung aus Beispiel 4A wurden in 100 ml Acetonitril gelöst und mit 1.07 g (9.9 mmol) Chloressigsäuremethylester, 2.73 g (19.8 mmol) Kaliumcarbonat und einer kleinen Spatelspitze Kaliumiodid versetzt. Die Reaktionsmischung wurde auf Rückfluss für 1 h erhitzt, auf RT abkühlen lassen und filtriert. Das Filtrat wurde am Rotationsverdampfer von den flüchtigen Komponenten befreit und der Rückstand am HV getrocknet. Ausbeute: 3.70 g (89% d. Th., 90% Reinheit) der Titelverbindung.
LC/MS [Methode 3]: Rₜ = 1.10 min.; m/z = 380 (M+H)⁺.
¹H NMR (DMSO-d₆, 400MHz): δ [ppm] = 7.78 (d, 2H), 7.64 (d, 2H), 6.91 (d, 1H), 4.72 (s, 2H), 4.16 - 4.35 (m, 1H), 3.99 (dd, 1H), 3.84 (dd, 1H), 3.70 (s, 3H).

Die racemische Verbindung aus Beispiel 5A konnte durch präparative HPLC an einer chiralen Phase in seinen Enantiomeren Beispiel 6A und Beispiel 7A getrennt werden, wie in WO 2007/134862 beschrieben.

Säule: Chirale Kieselgel-Phase basierend auf dem Selektor Poly(*N*-methacryloyl-L-isoleucin-3-pentylamid, 430 mm x 40 mm; Eluent: Stufengradient iso-Hexan/Essigsäureethylester 1:1 → Essigsäureethylester → iso-Hexan/Essigsäureethylester 1:1; Fluss: 50 ml/min; Temperatur: 24°C; UV-Detektion: 260 nm.

Man erhielt auf dieser Weise aus 3.6 g racemischer Verbindung aus Beispiel 5A (in 27 ml Essigsäureethylester und 27 ml I iso-Hexan gelöst und in drei Portionen durch die Säule getrennt) 1.6 g des zuerst eluierenden Enantiomers 1 (Beispiel 6A) sowie 1.6 g des später eluierenden Enantiomers 2 (Beispiel 7A).

### Beispiel 6A

### {3-(4-Chlorphenyl)-5-oxo-4-[(2S)-3,3,3-trifluor-2-hydroxypropyl]-4,5-dihydro-1H-1,2,4-triazol-1-yl}-essigsäuremethylester

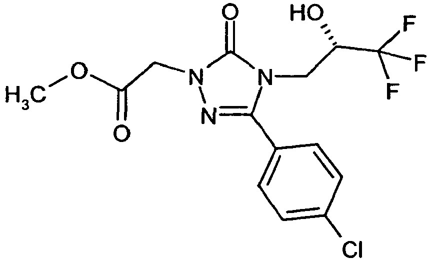

Zuerst eluierendes Enantiomer aus der Racemat-Trennung von Beispiel 5A.

Rₜ = 3.21 min [Säule: Chirale Kieselgel-Phase basierend auf dem Selektor Poly(*N*-methacryloyl-L-isoleucin-3-pentylamid, 250 mm x 4.6 mm; Eluent: *iso*-Hexan/Essigsäureethylester 1:1; Fluss: 1 ml/min; UV-Detektion: 260 nm].

### Beispiel 7A

### {3-(4-Chlorphenyl)-5-oxo-4-[(2R)-3,3,3-trifluor-2-hydroxypropyl]-4,5-dihydro-1H-1,2,4-triazol-1-yl}-essigsäuremethylester

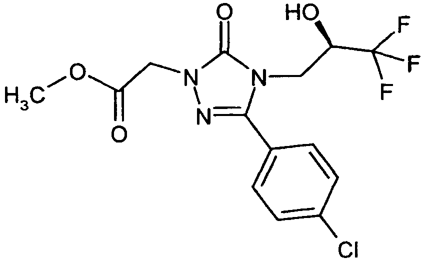

Zuletzt eluierendes Enantiomer aus der Racemat-Trennung von Beispiel 5A.

Rₜ = 4.48 min [Säule: Chirale Kieselgel-Phase basierend auf dem Selektor Poly(*N*-methacryloyl-L-isoleucin-3-pentylamid, 250 mm x 4.6 mm; Eluent: *iso*-Hexan/Essigsäureethylester 1:1; Fluss: 1 ml/min; UV-Detektion: 260 nm].

### Beispiel 8A

### {3-(4-Chlorphenyl)-5-oxo-4-[(2S)-3,3,3-trifluor-2-hydroxypropyl]-4,5-dihydro-1H-1,2,4-triazol-1-yl}-essigsäure

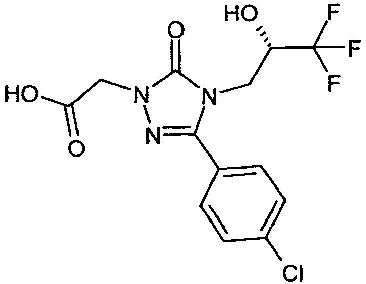

Der enantiomerenreine Ester aus Beispiel 6A (1.6 g, 4.21 mmol) wurde in 77 ml Methanol gelöst und mit 17 ml einer 1 M Lösung von Lithiumhydroxid in Wasser versetzt. Die Mischung wurde 1 h bei RT gerührt und dann am Rotationsverdampfer vom Methanol befreit. Der Rückstand wurde mit 100 ml Wasser verdünnt und mit 1 N Salzsäure auf pH 1 - 2 angesäuert. Das ausgefallene Produkt wurde abfiltriert, mit Wasser und Cyclohexan nacheinander gewaschen und filtriert. Nach Trocknung am HV wurde die Titelverbindung (1.1 g, 71% d. Th.) erhalten.
[α]_{D}²⁰ = +3.4° (Methanol, c = 0.37 g/100 ml)
LC/MS [Methode 1]: Rₜ = 1.51 min; m/z = 366 (M+H)⁺
¹H-NMR (400 MHz, DMSO-d₆): δ [ppm] = 3.84 (dd, 1H), 4.00 (dd, 1H), 4.25 (m, 1H), 4.58 (s, 2H), 6.91 (d, 1H), 7.63 (d, 2H), 7.78 (d, 2H), 13.20 (br. s, 1H).

### Beispiel 9A

### {3-(4-Chlorphenyl)-5-oxo-4-[(2R)-3,3,3-trifluor-2-hydroxypropyl]-4,5-dihydro-1H-1,2,4-triazol-1-yl}-essigsäure

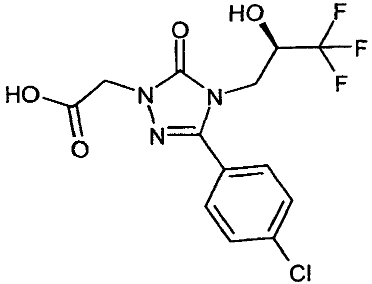

Analog zu Beispiel 8A wurde aus Beispiel 7A die Titelverbindung erhalten.
[α]_{D}²⁰ = -4.6° (Methanol, c = 0.44 g/100 ml)
LC/MS [Methode 1]: Rₜ = 1.53 min; m/z = 366 (M+H)⁺
¹H-NMR (400 MHz, DMSO-d₆): δ [ppm] = 3.84 (dd, 1H), 4.00 (dd, 1H), 4.25 (m, 1H), 4.58 (s, 2H), 6.91 (d, 1H), 7.63 (d, 2H), 7.78 (d, 2H), 13.20 (br. s, 1H).

### Beispiel 10A

### Methyl-{3-(4-chlorphenyl)-5-oxo-4-[(1E)-3,3,3-trifluorprop-1-en-1-yl]-4,5-dihydro-1H-1,2,4-triazol-1-yl}acetat

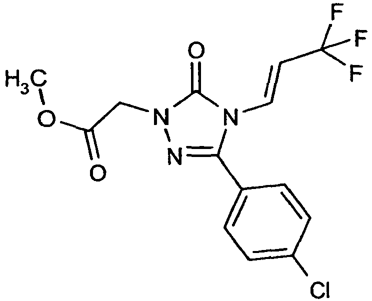

280 mg (0.74 mmol) der Verbindung aus Beispiel 7A wurden bei RT zusammen mit 108.1 mg (0.89 mmol) 4-Dimethylaminopyridin in 5.3 ml Pyridin vorgelegt, portionsweise mit 0.31 ml (1.84 mmol) Trifluormethansulfonsäureanhydrid versetzt und 12 h gerührt. Das Pyridin wurde am Rotationsverdampfer entfernt. Der Rückstand wurde in Acetonitril und 1N Salzsäure aufgenommen und über die präparative HLPC (Methode 10) gereinigt. Erhalten wurden 230 mg (86 % d. Th.) der Titelverbindung.
LC/MS [Methode 4]: Rₜ = 1.14 min; m/z = 362 (M+H)⁺
¹H-NMR (400MHz, DMSO-d₆): δ [ppm]= 3.72 (s, 3H), 4.78 (s, 2H), 6.85 (dd, 1H), 7.18 (d, 1H), 7.68 (s, 4H).

### Beispiel 11A

### {3-(4-Chlorphenyl)-5-oxo-4-[(1E)-3,3,3-trifluorprop-1-en-1-yl]-4,5-dihydro-1H-1,2,4-triazol-1-yl}essigsäure

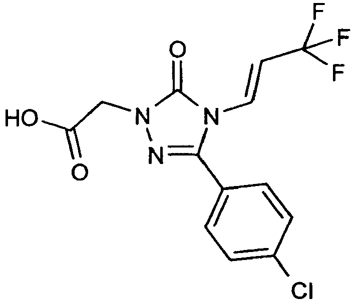

260 mg (0.72 mmol) der Verbindung aus Beispiel 10A wurden in 5 ml Methanol gelöst und mit 2.87 ml (2.87 mmol) einer 1 M Lösung von Lithiumhydroxid in Wasser versetzt. Die Mischung wurde 1 h bei RT gerührt, dann mit 1N Salzsäure angesäuert und mit DMSO verdünnt. Die gesamte Lösung wurde über die präparative HLPC (Methode 10) gereinigt. Erhalten wurden 215 mg (86 % d. Th.) der Titelverbindung.
LC/MS [Methode 4]: Rₜ = 1.03 min.; m/z = 348 (M+H)⁺
¹H-NMR (400MHz, DMSO-d₆): δ [ppm]= 4.64 (s, 2H), 6.79 - 6.92 (m, 1H), 7.19 (dd, 1H), 7.68 (s, 4H), 13.31 (br. s, 1H).

### Beispiel 12A

### 2-Amino-2-[3-(trifluormethyl)phenyl]propanamid

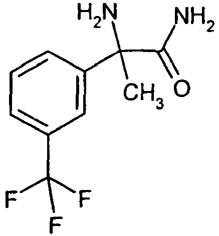

138 ml Wasser, 108 ml 25%ige wässrige Ammoniaklösung und 173 ml Ethanol wurden vorgelegt und dann mit 108 g (574.0 mmol)1-[3-(Trifluormethyl)phenyl]ethanon, 30 g (574 mmol) Natriumcyanid und 31 g (631 mmol) Ammoniumchlorid versetzt.

Diese Mischung wurde für 20h im Autoklav bei 70°C rühren gelassen. Am Rotationsverdampfer wurde vom Ethanol befreit und 4 x mit je 500 ml Ether extrahiert. Die vereinigten organischen Phasen wurden mit Magnesiumsulfat und Aktivkohle versetzt und über Kieselgur abgesaugt. Das Filtrat wurde am Rotationsverdampfer eingeengt. Anschließend wurde der Rückstand durch Chromatographie an 2 kg Kieselgel 60 (Laufmittel: Cyclohexan-Essigester 3:1 1 bis 1: 1) gereinigt.

Das so isolierte Zwischenprodukt 2-Amino-2-[3-(trifluoromethyl)phenyl]propannitril (56 g, 46 % d.Th.) wurde unter Eiskühlung langsam mit 500 ml konzentrierter Salzsäure versetzt. Die Suspension wurde über Nacht bei RT gerührt. Am Rotationsverdampfer wurde das Volumen auf 150 ml reduziert. Es wurden 250 ml Aceton zugegeben und alle flüchtigen Komponenten am Rotationsverdampfer entfernt. Der verbleibende feste Brei wurde unter Eiskühlung mit 125 ml konzentrierter wässriger Ammoniaklösung versetzt. Es wurde 30 Minuten im Eisbad gerührt. Die Kristalle wurden abgesaugt und 2x mit je 50 ml Eiswasser, dann Pentan gewaschen. Das Produkt wurde im Hochvakuum getrocknet. Erhalten wurden 43 g (32% d. Th.) der Titelverbindung.
MS (ESIpos): m/z = 233 [M+H]⁺.
H-NMR (400MHz, CDCl₃): δ [ppm]= 1.82 (s, 3H), 5.54 (br.s, 1H), 7.26 (br.s, 1H), 7.48 (t, 1H), 7.55 (d, 2H), 7.75 (d, 1H), 7.83 (s, 1H).

### Beispiel 13A

### tert.-Butyl-{1-amino-1-oxo-2-[3-(trifluormethyl)phenyl]propan-2-yl}carbamat

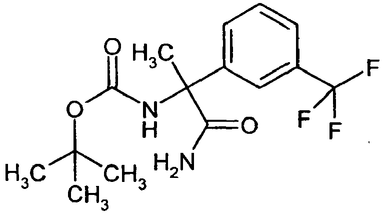

43.0 g (185 mmol) 2-Amino-2-[3-(trifluormethyl)phenyl]propanamid wurden in 245 ml DMF und 245 ml tert.-Butanol zusammen mit 53.6 g (638 mmol) Natriumhydrogencarbonat bei RT vorgelegt und dann mit 99.5 g (456 mmol) Di-tert.-butyldicarbonat versetzt. Es wurde bei 60°C für 3 Tage nachgerührt. Zur Aufarbeitung wurde mit Essigsäureethylester verdünnt und nacheinander zweimal mit Wasser, zweimal mit 1M Salzsäure und einmal mit gesättigter wässriger Natriumchloridlösung gewaschen. Die organische Phase wurde über Natriumsulfat getrocknet und im Vakuum eingeengt. Der Rückstand wurde in DMSO aufgenommen und über die präparative HPLC (Methode 7) getrennt. Die Produktfraktion wurde am Rotationsverdampfer eingeengt. Der Rückstand wurde im Hochvakuum getrocknet. Erhalten wurden 30.0 g (50 % d. Th.) der Titelverbindung.
LC/MS [Methode 2]: Rₜ = 2.11 min; m/z = 333 (M+H)⁺
¹H-NMR (400MHz, DMSO-d₆): δ [ppm]= 1.82 (s, 3H), 7.09 (br.s, 1H), 7.27 - 7.40 (m, 2H), 7.53 - 7.65 (m, 2H), 7.65 - 7.73 (m, 2H).

Die beiden Enantiomere konnten durch HPLC an einer chiralen Phase [Methode 13] getrennt werden: siehe Beispiele 14A und 15A.

### Beispiel 14A

### tert.-Butyl-{(2R)-1-amino-1-oxo-2-[3-(trifluoromethyl)phenyl]propan-2-yl}carbamat (Enantiomer I)

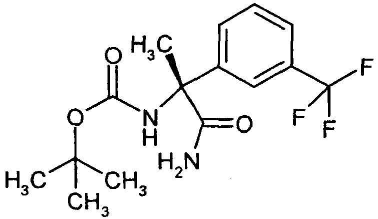

Zuerst eluiertes Enantiomer (12.1 g) aus der Enantiomerentrennung von 21.5 g der Verbindung aus Beispiel 13A nach Methode 13.
Chirale analytische HPLC [Methode 14]: Rₜ = 2.89 min.

### Beispiel 15A

### tert.-Butyl-{(2S)-1-amino-1-oxo-2-[3-(trifluormethyl)phenyl]propan-2-yl}carbamat (Enantiomer II)

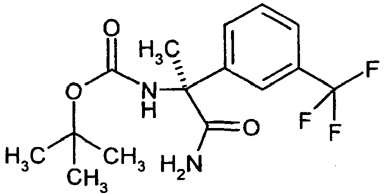

Zuletzt eluiertes Enantiomer (12.1 g) aus der Enantiomerentrennung von 21.5 g der Verbindung aus Beispiel 13A nach Methode 13.
Chirale analytische HPLC [Methode 14]: Rₜ = 4.55 min.

### Beispiel 16A

### (2R)-2-Amino-2-[3-(trifluormethyl)phenyl]propanamid-Hydrochlorid

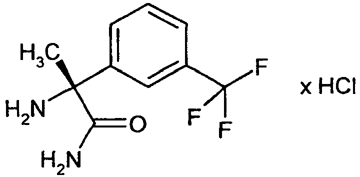

12 g (36.1 mmol) tert.-Butyl-{(2*R*)-1-amino-1-oxo-2-[3-(trifluormethyl)phenyl]propan-2-yl}carbamat aus Beispiel 14A wurden bei RT in 20 ml Dichlormethan vorgelöst, dann mit 50 ml 4M Chlorwasserstofflösung in Dioxan versetzt und 1 h nachgerührt. Es wurde im Vakuum eingeengt und der Rückstand im Hochvakuum getrocknet. Der Rückstand wurde mit 100 ml Dichlormethan versetzt und 10 Minuten im Ultraschallbad gehalten. Der Feststoff wurde abgesaugt, mit etwas Dichlormethan nachgewaschen und im Hochvakuum getrocknet. Erhalten wurden 8.14 g (84% d. Th.) der Titelverbindung.
LC/MS [Methode 2]: Rₜ = 0.51 min; m/z = 233 (M+H)⁺
¹H-NMR (400MHz, DMSO-d₆): δ [ppm]= 1.95 (s, 3H), 7.69 - 7.94 (m, 6H), 8.85 (br.s, 3H).

### Beispiel 17A

### (2S)-2-Amino-2-[3-(trifluormethyl)phenyl]propanamid-Hydrochlorid

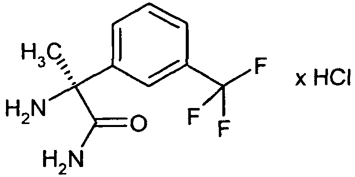

11.5 g (34.6 mmol) tert.-Butyl-{(2S)-1-amino-1-oxo-2-[3-(trifluormethyl)phenyl]propan-2-yl}carbamat aus Beispiel 15A wurden bei RT in 20 ml Dichlormethan vorgelöst, dann mit 4M Chlorwasserstofflösung in Dioxan versetzt und 1 h nachgerührt. Es wurde im Vakuum auf ein 1/3 des Volumens eingeengt, wobei das Produkt bereits kristallin ausfiel. Es wurde mit 100 ml Dichlormethan verdünnt und 10 Minuten im Ultraschallbad gehalten. Der Feststoff wurde abgesaugt und mit etwas Dichlormethan nachgewaschen und im Hochvakuum getrocknet. Erhalten wurden 7.56 g (82% d. Th.) der Titelverbindung.
LC/MS [Methode 2]: Rₜ = 0.55 min; m/z = 233 (M+H)⁺
¹H-NMR (400MHz, DMSO-d₆): δ [ppm]= 1.94 (s, 3H), 7.67 - 7.80 (m, 3H), 7.80 - 7.91 (m, 3H), 8.79 (br.s, 3H).

### Beispiel 18A

### 2-Amino-2-[2-fluor-3-(trifluormethyl)phenyl]propanamid-Hydrochlorid

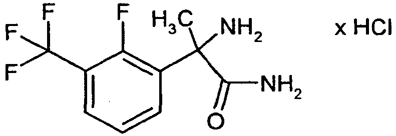

5 g (24.3 mmol) 1-[2-Fluor-3-(trifluormethyl)phenyl]ethanon wurden mit 1.248 g (25.5 mmol) Natriumcyanid, 1.427 g (26.7 mmol) Ammoniumchlorid und 3.6 ml 25%iger wässriger Ammoniak-Lösung zusammen in 6 ml Wasser und 7.5 ml Ethanol bei 70°C für 17 h gerührt. Die dunkelbraune Lösung wurde auf RT abgekühlt und am Rotationsverdampfer auf 1/3 des Volumens reduziert. Der Rückstand wurde 3x mit Diethylether extrahiert. Die vereinigten organischen Phasen wurden mit Magnesiumsulfat und Aktivkohle versetzt, 30 min verrührt und dann filtriert. Das Filtrat wurde mit 8 ml 4M Chlorwasserstofflösung in Dioxan versetzt, 5 min verrührt und am Rotationsverdampfer von den flüchtigen Komponenten befreit. Der Rückstand wurde mit 20 ml konzentrierter Salzsäure versetzt und über Nacht verrührt. Es wurde mit Wasser auf 300 ml verdünnt und 3x mit je 50 ml Dichlormethan gewaschen. Die wässrige Phase wurde mit 35%iger wässriger Ammoniak-Lösung alkalisch gestellt (ca. pH 9-10) und 3x mit je 75 ml Dichlormethan extrahiert. Die vereinigten organischen Phasen wurden über Natriumsulfat getrocknet und im Vakuum eingeengt. Der Rückstand wurde in 150 ml Diethylether aufgenommen und mit 8 ml 4M Chlorwasserstofflösung in Dioxan versetzt. Es wurde im Vakuum eingeengt und im Hochvakuum getrocknet. Erhalten wurden 1.97 g (24 % d. Th., 86 % Reinheit) der Titelverbindung.
LC/MS [Methode 3]: Rₜ = 0.25 min; m/z = 251 (M+H)⁺
¹H-NMR (400MHz, DMSO-d₆): δ [ppm]= 1.93 (s, 3H), 7.49 - 7.77 (m, 3H), 7.84 - 8.04 (m, 2H), 8.74 (br.s, 3H).

### Beispiel 19A

### 2-Amino-2-[3-(trifluormethyl)phenyl]butanamid-Hydrochlorid

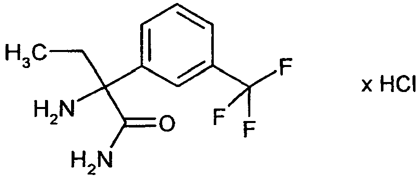

9.8 g (48.5 mmol) 1-[3-(Trifluormethyl)phenyl]propan-1-on wurden zusammen mit 3.8 g (77.6 mmol) Natriumcyanid, 4.4 g (82.4 mmol) Ammoniumchlorid und 10 ml 35%iger wässriger Ammoniak-Lösung in 25 ml Wasser und 30 ml Ethanol bei 70°C für 17 h nachgerührt. Die Lösung wurde auf RT abgekühlt. Das Volumen wurde am Rotationsverdampfer auf 1/3 reduziert. Der Rückstand wurde 3x mit Diethylether extrahiert. Die vereinigten organischen Phasen wurden mit Magnesiumsulfat und Aktivkohle versetzt, 30 Minuten verrührt und dann filtriert. Das Filtrat wurde mit 20 ml 4M Chlorwasserstofflösung in Dioxan versetzt und der ausgefallene Feststoff abgesaugt. Der Feststoff wurde mit 40 ml konzentrierter Salzsäure versetzt und über Nacht verrührt. Die Mischung wurde mit Wasser auf 300 ml verdünnt. Es wurde 3x mit je 50 ml Dichlormethan gewaschen. Die wässrige Phase wurde mit 35%iger wässriger Ammoniak-Lösung alkalisch gestellt (ca. pH 9-10) und 3x mit je 75 ml Dichlormethan extrahiert. Die vereinigten organischen Phasen wurden über Natriumsulfat getrocknet, dann mit 10 ml 4M Chlorwasserstofflösung in Dioxan versetzt und am Rotationsverdampfer vom Lösemittel befreit. Der Feststoff wurde im Hochvakuum getrocknet, dann noch mal in Wasser gelöst und über präparative HPLC (Methode 7) gereinigt. Die Produktfraktion wurde am Rotationsverdampfer vom Lösemittel befreit und anschließend im Hochvakuum getrocknet. Erhalten wurden 190 mg (1.4 % d. Th.) der Titelverbindung.
LC/MS [Methode 2]: Rₜ = 0.78 min; m/z = 247 (M+H)⁺
¹H-NMR (400MHz, DMSO-d₆): δ [ppm]= 0.76 (t, 3H), 1.79 - 1.93 (dq, 1H), 1.97 - 2.10 (dq, 1H), 7.11 (br.s, 1H), 7.38 (br.s, 1H), 7.51 - 7.62 (m, 2H), 7.82 (d, 1H), 7.87 (s, 1H).

### Beispiel 20A

### 2-Amino-2-cyclopropyl-2-[3-(trifluormethyl)phenyl]acetamidhydrochlorid

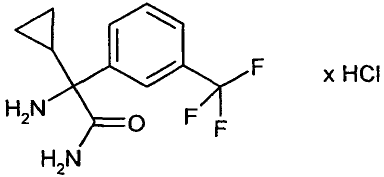

1.6 g (7.5 mmol) Cyclopropyl[3-(trifluormethyl)phenyl]methanon wurden zusammen mit 384 mg (7.8 mmol) Natriumcyanid, 440 mg (8.2 mmol) Ammoniumchlorid und 1 ml 35%iger wässriger Ammoniak-Lösung in 3 ml Wasser und 3 ml Ethanol bei 70°C für 17 h nachgerührt. Die Lösung wurde auf RT abgekühlt und am Rotationsverdampfer auf 1/3 des Volumens reduziert. Der Rückstand wurde 3x mit Diethylether extrahiert. Die vereinigten organischen Phasen wurden mit Magnesiumsulfat und Aktivkohle versetzt, 30 min verrührt und dann filtriert. Das Filtrat wurde mit 10 ml 4M-Chlorwasserstofflösung in Dioxan versetzt und im Vakuum eingeengt. Der Rückstand wurde mit 20 ml konzentrierter Salzsäure versetzt und über Nacht verrührt. Es wurde mit Wasser auf 100 ml verdünnt und 3x mit je 50 ml Dichlormethan gewaschen. Die wässrige Phase wurde mit 35%iger wässriger Ammoniak-Lösung alkalisch gestellt (ca. pH 9-10) und dreimal mit je 75 ml Dichlormethan extrahiert. Die vereinigten organischen Phasen wurden über Natriumsulfat getrocknet und mit 10 ml 4M Chlorwasserstofflösung in Dioxan versetzt. Es wurde von allen flüchtigen Bestandteilen am Rotationsverdampfer befreit. Der Rückstand wurde im Hochvakuum getrocknet dann wieder in Wasser gelöst und über präparative HPLC (Methode 10) gereinigt. Die Produktfraktion wurde am Rotationsverdampfer vom Lösemittel befreit und anschließend im Hochvakuum getrocknet. Erhalten wurden 24 mg (1 % d. Th.) der Titelverbindung mit ca. 80 % Reinheit.
LC/MS [Methode 2]: Rₜ = 0.95 min; m/z =259 (M+H)⁺
¹H-NMR (400MHz, DMSO-d₆): δ [ppm]= 0.54 - 0.67 (m, 1H), 0.74 (m, 2H), 0.80 - 0.98 (m, 1H), 1.68 - 1.87 (m, 1H), 7.54 (s, 1H), 7.73 - 7.80 (m, 1H), 7.82 - 7.97 (m, 4H), 8.46 - 8.72 (m, 3H).

### Beispiel 21A

### tert-Butyl-[(2R)-1-amino-2-(2-chlorphenyl)-1-oxopropan-2-yl]carbamat

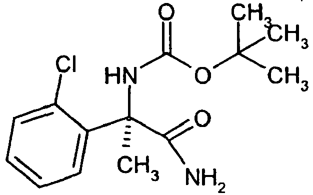

In 10 ml 10%-iger wässriger Natriumhydrogencarbonatlösung wurden 500 mg (2.12 mmol) (2*R*)-2-Amino-2-(2-chlorphenyl)propansäure-Hydrochlorid (Firma Netchem, New Brunswick NJ 08901, USA, Artikel-Nr: 506093-HCl) gelöst. Es wurden 10 ml Dioxan und 511 µl (2.22 mmol) Di-tert-butyldicarbonat hinzugefügt und die Reaktionsmischung über Nacht bei RT gerührt. Durch Zugabe von 1N Salzsäure wurde der pH-Wert auf 2 gestellt, dann das Produkt dreimal mit Essigsäureethylester extrahiert. Die vereinigten organischen Phasen wurden über Natriumsulfat getrocknet und das Lösungsmittel am Rotationsverdampfer entfernt. Der Rückstand wurde im HV getrocknet und entspricht dem Zwischenprodukt (2*R*)-2-[(tert-Butoxycarbonyl)amino]-2-(2-chlorphenyl)propansäure (322 mg, 51% d. Th. LC-MS [Methode 3]: Rₜ = 1,08 min. m/z: ES pos.: 322 (M+Na)⁺, ES neg.: 298 (M-H)⁻.

100 mg (0.334 mmol) der so erhaltenen (2R)-2-[(tert-Butoxycarbonyl)amino]-2-(2-chlorphenyl)propansäure und 81 mg (0.6 mmol) HOBt wurden im 3 ml Dimethylformamid vorgelegt, 115 mg (0.6 mmol) EDC zugegeben und die Reaktionsmischung 20 Minuten bei RT gerührt. Sie wurde dann mit 2 ml 32%iger wässriger Ammoniak-Lösung versetzt und über Nacht bei RT weitergerührt. Die Mischung wurde mit 1N Salzsäure auf pH 2 gestellt und über die präparative HPLC (Methode 10) getrennt. Die Produktfraktion wurde am Rotationsverdampfer vom Lösemittel befreit und anschließend im Hochvakuum getrocknet. Erhalten wurden 59 mg (59 % d. Th.) der Titelverbindung.
LC/MS [Methode 3]: Rₜ = 1.02 min; m/z = 299 [M+H]⁺.
¹H-NMR (400MHz, DMSO-d₆): δ [ppm]= 1.26 (br.s, 7H), 1.84 (s, 3H), 6.46 - 6.70 (m, 1H), 6.85 (br.s, 1H), 7.25 - 7.44 (m, 4H), 7.64 (d, 1H).

### Beispiel 22A

### (2R)-2-Amino-2-(2-chlorphenyl)propanamidhydrochlorid

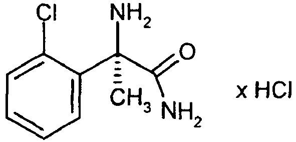

58 mg (0.194 mmol) tert.-Butyl-[(2*R*)-1-amino-2-(2-chiorphenyl)-1-oxopropan-2-yl]carbamat aus Beispiel 21A wurden mit 2 ml Dichlormethan und 2 ml 4M Chlorwasserstofflösung in Dioxan versetzt und bei RT 2 h verrührt. Es wurden alle flüchtigen Bestandteile am Rotationsverdampfer entfernt und der weiße Feststoff im Hochvakuum getrocknet. Erhalten wurden 50 mg (46 % d. Th.) der Titelverbindung.
LC/MS [Methode 2]: Rₜ = 0.22 min; m/z = 199 (M+H)⁺
¹H-NMR (400MHz, DMSO-d₆): δ [ppm]= 1.89 (s, 3H), 7.30 (s, 1H), 7.47 - 7.57 (m, 3H), 7.61 (s, 1H), 7.68 - 7.77 (m, 1H), 8.40 (br.s, 3H).

### Beispiel 23A

### 5-Methyl-5-[3-(trifluormethyl)phenyl]imidazolidin-2,4-dion (Racemat)

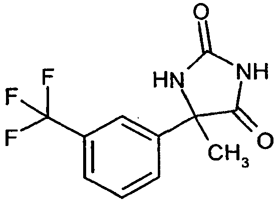

Eine Mischung von 25 g (133 mmol) 3-Trifluormethylacetophenon, 10.4 g (159 mmol) Kaliumcyanid und 63.8 g (664 mmol) Ammoniumcarbonat in 300 ml Wasser und 300 ml Ethanol wurde über Nacht bei 60°C gerührt. Das Ethanol wurde am Rotationsverdampfer entfernt. Das Produkt fiel in der verbleibende wässrige Mischung aus. Es wurde abfiltriert, dreimal mit Wasser gewaschen und im HV getrocknet. Man erhielt 31 g (90% d. Th.) der Titelverbindung.
LC/MS [Methode 3]: Rt = 0.90 min; m/z = 259 (M+H)+
1H-NMR (400MHz, DMSO-d6): δ [ppm]= 1.69 (s, 3H), 7.62 - 7.70 (m, 1H), 7.71 - 7.76 (m, 1H), 7.78 (s, 1H), 7.83 (d, 1H), 8.75 (s, 1H), 10.91 (br.s, 1H).

Die beiden Enantiomere konnten durch Chromatographie an einer chiralen Phase (Methode 8) getrennt werden: siehe Beispiele 24A und 25A.

### Beispiel 24A

### (5R)-5-Methyl-5-[3-(trifluormethyl)phenyl]imidazolidin-2,4-dion

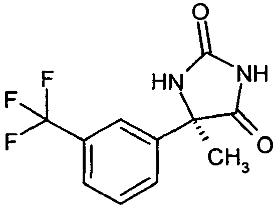

Zuerst eluiertes Enantiomer (14.6 g) aus der Trennung von 30.3 g der Verbindung aus Beispiel 23A nach Methode 8.
Chirale analytische HPLC [Methode 9]: Rₜ = 2.9 min.
[α]_{D}²⁰ = - 102.2° (Methanol, c = 0.53 g/100 ml)

Die absolute Konfiguration wurde durch Hydrolyse zu Beispiel 26A und Vergleich mit der käuflichen Aminosäure bestimmt (siehe Beispiel 27A).

### Beispiel 25A

### (5S)-5-Methyl-5-[3-(trifluormethyl)phenyl]imidazolidin-2,4-dion

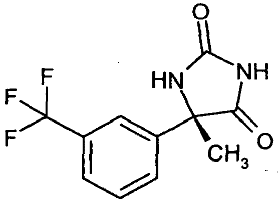

Zuletzt eluiertes Enantiomer (13.8 g) aus der Trennung von 30.3 g der Verbindung aus Beispiel 23A nach Methode 8.
Chirale analytische HPLC [Methode 9]: Rₜ = 5.4 min.
[α]_{D}²⁰ = + 102.4° (Methanol, c = 0.53 g/100 ml)

### Beispiel 26A

### 2-Amino-2-[3-(trifluormethyl)phenyl]propansäure (Racemat)

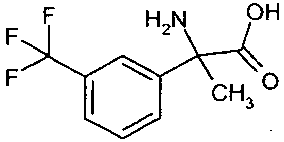

300 mg (1.16 mmol) der Verbindung aus Beispiel 23A wurden in 3 ml 1N Natronlauge 3 Tage auf Rückfluss erhitzt. Nach Abkühlung auf RT wurde die Reaktionsmischung durch vorsichtiger Zugabe von 6N Salzsäure sauer gestellt (pH 1-2). Dabei fiel das Produkt teilweise als Gel aus. Die Mischung wurde mit 200 ml Wasser verdünnt und zweimal mit Essigsäureethylester gewaschen. Die wässrige Phase wurde am Rotationsverdampfer vom Wasser befreit. Der Rückstand wurde in Methanol verrührt und die erhaltene Suspension filtriert. Das Filtrat wurde am Rotationsverdampfer vom Methanol befreit. Der Rückstand wurde in einem Gemisch Acetonitril / Wasser 2:1 gelöst und durch präparative HPLC (Methode 10) gereinigt. Es wurden 205 mg (76% d. Th.) der Titelverbindung erhalten.
LC/MS [Methode 3]: Rₜ = 0.34 min; m/z = 234 (M+H)⁺
¹H-NMR (400MHz, DMSO-d₆): δ [ppm]= 1.67 (s, 3H), 7.56 - 7.68 (m, 2H), 7.79 (d, 1H), 7.86 (s, 1H), 8.20 (br.s, 2H).

### Beispiel 27A

### (2R)-2-Amino-2-[3-(trifluormethyl)phenyl]propansäure

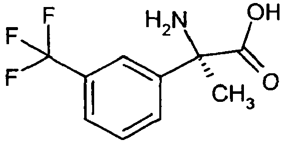

14.6 g (56.7 mmol) der Verbindung aus Beispiel 24A wurden in 400 ml 2N-Natronlauge 23 h auf Rückfluss erhitzt. Die Reaktionsmischung wurde auf 0°C gekühlt (Eisbad) und langsam mit 6N Salzsäure bis pH 1 versetzt. Der ausgefallene Feststoff wurde abfiltriert. Das Filtrat wurde am Rotationsverdampfer zur Trockne eingeengt. Der Rückstand wurde in 300 ml Methanol verrührt und die erhaltene Suspension filtriert. Dieses Filtrat wurde am Rotationsverdampfer eingeengt. Der Rückstand wurde in Wasser aufgenommen und durch präparative HPLC (Methode 7) gereinigt. Das erhaltene Produkt wurde im HV getrocknet (12 g, 91 % d. Th.)
LC/MS [Methode 3]: Rₜ = 0.33 min; m/z = 234 (M+H)⁺
[α]_{D}²⁰ = - 44.1° (Methanol, c = 0.50 g/100 ml).
¹H-NMR (400MHz, DMSO-d₆): δ [ppm]= 1.67 (s, 3H), 7.55 - 7.68 (m, 2H), 7.79 (d, 1H), 7.86 (s, 1H), 8.19 (br.s, 3H).

75 mg dieser Aminosäure wurden mit einem Überschuss einer 4N Chlorwasserstoff-Lösung in Dioxan behandelt, am Rotationsverdampfer von den flüchtigen Komponenten befreit und im HV getrocknet. Das so erhaltene Hydrochlorid zeigt folgende analytische Daten:
[α]_{D}²⁰ = - 63.8° (Methanol, c = 0.51 g/100 ml).
¹H-NMR (400MHz, DMSO-d₆): δ [ppm]= 1.91 (s, 3H), 7.75 (t, 1H), 7.84 (d, 1H), 7.86 - 7.94 (m, 2H), 9.18 (br.s, 3H).

Dieser Drehwert ist vergleichbar mit dem Drehwert, der für das käufliche (2R)-2-Amino-2-[3-(trifluormethyl)phenyl]propansäure-Hydrochlorid (Netchem, New Brunswick NJ 08901, USA, Artikel-Nr: 506085-HCl) bestimmt wurde: [α]_{D}²⁰ = - 44.1° (Methanol, c = 0.50 g/100 ml). Daher wurde für Beispiel 24A und für Beispiel 26A die (R)-Konfiguration angegeben, und für Beispiel 25A und Beispiel 27A die (S)-Konfiguration.

### Beispiel 28A

### (2S)-2-Amino-2-[3-(trifluormethyl)phenyl]propansäure

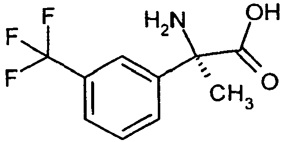

Analog zu Beispiel 26A wurden durch Hydrolyse von 13.1 g (50.7 mmol) der Verbindung aus Beispiel 25A 8.22 g (69 % d. Th.) der Titelverbindung erhalten.
LC/MS [Methode 2]: Rₜ = 0.92 min; m/z = 234 (M+H)⁺
[α]_{D}²⁰ = + 47.0° (Methanol, c = 0.50 g/100 ml).
¹H-NMR (400MHz, DMSO-d₆): δ [ppm]= 1.67 (s, 3H), 7.55 - 7.68 (m, 2H), 7.79 (d, 1H), 7.86 (s, 1H), 8.19 (br.s, 3H).

Diese Aminosäure wurde in Acetonitril mit einem Überschuss an 1N Salzsäure behandelt. Dann wurden die flüchtigen Komponenten am Rotationsverdampfer entfernt und der Rückstand im HV getrocknet. Das so erhaltene Hydrochlorid zeigt folgende analytische Daten:
[α]_{D}²⁰ = - 63.8° (Methanol, c = 0.51 g/100 ml).
¹H-NMR (400MHz, DMSO-d₆): δ [ppm]= 1.91 (s, 3H), 7.75 (t, 1H), 7.84 (d, 1H), 7.86 - 7.94 (m, 2H), 9.18 (br.s, 3H).

### Beispiel 29A

### 2-Amino-3,3,3-trifluor-2-[3-(trifluormethyl)phenyl]-propansäure (Mischung von Enantiomeren)

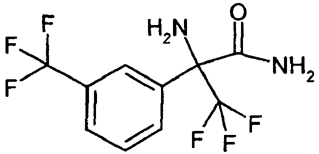

In Analogie zu H. Wang et al., Organic Letters 2006, 8 (7), 1379-1381 wurden 2.50 g (10.3 mmol) 2,2,2-Trifluoracetophenon mit 2.50 g (20.7 mmol) (R)-tert.-Butylsulfinamid in 21 ml n-Hexan vorgelegt und mit 4.40 g (4.57 ml, 15.5 mmol) Titan(IV)isopropylat versetzt. Die Reaktionsmischung wurde über Nacht bei RT gerührt, dann wurde die Reaktion unter Eisbad-Kühlung durch Zugabe von 9 ml Wasser gestoppt. Nach 5 min wurde die ganze Mischung über Celite filtriert. Das Filtrat wurde am Rotationsverdampfer eingeengt. Der Rückstand (2.86 g) wurde in 17 ml n-Hexan gelöst und bei RT mit 1.66 ml (12.4 mmol) Trimethylsilylcyanid versetzt. Die Reaktionsmischung wurde drei Tage bei RT gerührt, dann mit 30 ml 10%iger wässriger Ammoniumchloridlösung versetzt und dreimal mit Essigsäureethylester extrahiert. Die vereinigten organischen Phasen wurden über Natriumsulfat getrocknet und am Rotationsverdampfer vom Lösungsmittel befreit. Der Rückstand (3.04 g) wurde ohne Reinigung und Analytik weiter umgesetzt. Dafür wurde unter Eiskühlung die Gesamtmenge in 23 ml konz. Schwefelsäure gelöst, dann bei RT 3 h gerührt. Die Reaktionsmischung wurde auf Eis gegossen und dreimal mit Essigsäureethylester extrahiert. Die vereinigten organischen Phasen wurden über Natriumsulfat getrocknet und am Rotationsverdampfer eingeengt. So wurde der Rückstand A erhalten. Die saure wässrige Phase wurde mit 20%iger Natronlauge auf pH 7 gestellt und erneut dreimal mit Essigsäureethylester extrahiert. Die vereinigten organischen Phasen wurden über Natriumsulfat getrocknet und am Rotationsverdampfer eingeengt. So wurde der Rückstand B erhalten. Die beiden Rückstände A und B wurden vereinigt und über präparative HPLC (Methode 10) getrennt. Die geeignete Fraktion wurde am Rotationsverdampfer eingeengt und die verbliebene wässrige Phase wurde mit 2M Natronlauge auf pH 14 gestellt, dann dreimal mit Dichlormethan extrahiert. Die vereinigten Dichlormethan-Phasen wurden über Natriumsulfat getrocknet und am Rotationsverdampfer eingeengt. Das Öl entsprach der Titelverbindung (136 mg, 5% d. Th).
1H-NMR (400MHz, DMSO-d6): δ [ppm]= 7.49 (br.s, 1H), 7.59 (br.s, 1H), 7.67 (t, 1H), 7.78 (d, 1H), 7.97 (d, 1H), 8.02 (s, 1H).

### Beispiel 30A

### {4-[(2S)-2-{[tert-Butyl(dimethyl)silyl]oxy}-3,3,3-trifluorpropyl]-3-(4-chlorphenyl)-5-oxo-4,5-dihydro-1H-1,2,4-triazol-1-yl}essigsäure

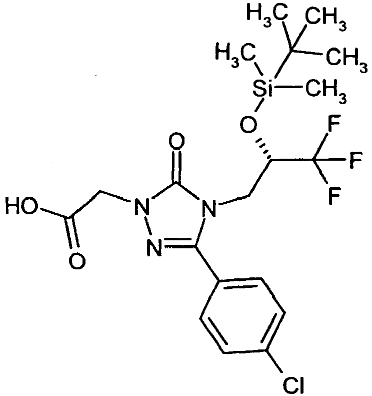

300 mg (0.82 mmol) der Verbindung aus Beispiel 8A wurden mit 2.46 ml (1.5 eq) einer Lösung, die 0.5M tert.-Butyldimethylsilylchlorid und 1 M Imidazol in DMF enthält, versetzt. Die Reaktionsmischung wurde über Nacht bei RT gerührt. Dann wurden erneut 1.5 eq. der obigen Lösung zugegeben und die Mischung 24 h gerührt. Dieser Vorgang wurde wiederholt, bis insgesamt 6 eq. tert.-Butyldimethylsilylchlorid zugegeben worden sind. Die Reaktionsmischung wurde dann mit 6 ml einer 2M wässrigen Natriumcarbonatlösung versetzt und 30 min gerührt. Durch Zugabe von 1M Salzsaüre wurde der pH-Wert auf 4 gestellt und die Mischung dreimal mit Dichlormethan extrahiert. Die vereinigten organischen Phasen wurden mit Wasser gewaschen, dann über Natriumsulfat getrocknet und am Rotationsverdampfer eingeengt. Der Rückstand wurde im HV getrocknet. Man erhielt die Titelverbindung: 407 mg (93 % d. Th.).
LC/MS [Methode 5]: Rₜ = 1.33 min; m/z = 480 (M+H)⁺
¹H-NMR (400 MHz, DMSO-d₆): δ [ppm] = - 0.11 (s, 3H), 0.06 (s, 3H), 0.80 (s, 9H), 4.01 (dd, 1H), 4.13 (dd, 1H), 4.54 - 4.63 (m, 1H), 4.60 (s, 2H), 7.69 (d, 2H), 7.80 (d, 2H).

### Beispiele 31A

### N-Cyclopropyl-2-{[2-(trifluormethoxy)phenyl]carbonyl}hydrazincarboxamid

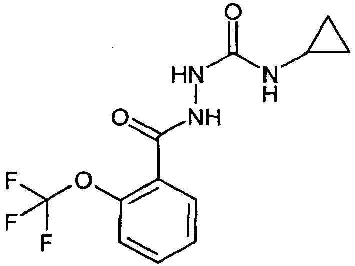

2.00 g (9.09 mmol) 2-Trifluormethoxybenzhydrazid wurden in trockenem THF (50 ml) bei 60°C gelöst, anschließend tropfte man 0.79 g (9.09 mmol) Cyclopropylisocyanat gelöst in 10 ml trockenem Tetrahydrofuran hinzu. Es wurde 18 h bei 60°C gerührt. Nach Abkühlen auf RT wurde mit ca. 50 ml Diethylether verrührt. Der farblose Feststoff wurde abgesaugt, mit Diethylether gewaschen und im Hochvakuum getrocknet. 2.57 g (93% d.Th.) der Zielverbindung wurden so erhalten.
LC-MS [Methode 6] Rₜ = 1.43 min; MS [ESIpos]: m/z = 304 (M+H)⁺.
¹H-NMR (400MHz, CDCl₃): δ [ppm]= 0.61 - 0.69 (m, 2H), 0.77 - 0.85 (m, 2H), 2.60 - 2.68 (m, 1H), 5.45 (br.s, 1H), 7.34 (d, 1H), 7.42 (t, 1H), 7.52 - 7.62 (m, 2H), 7.99 (dd, 1H), 8.63 (br.s, 1H).

### Beispiel 32A

### 4-Cyclopropyl-5-[2-(trifluormethoxy)phenyl]-2,4-dihydro-3H-1,2,4-triazol-3-on

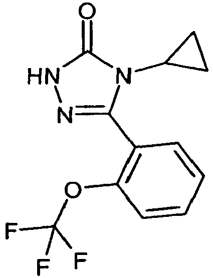

2.53 g (8.3 mmol) der Verbindung aus Beispiel 31A wurden in 15 ml 3M Natronlauge suspendiert und 96 h zum Rückfluss erhitzt. Nach dem Abkühlen stellte man mit halbkonzentrierter Salzsäure auf pH 10 ein. Der ausgefallene Feststoff wurde abgesaugt, mit Wasser neutral gewaschen und anschließend in Methanol verrührt. Die Mischung wurde filtriert, das Filtrat am Rotationsverdampfer eingeeengt und der Rückstand im Hochvakuum getrocknet. Man erhielt so 1.81 g (55% d. Th., 72% Reinheit) der gewünschten Verbindung, die so direkt weiter umgesetzt wurden.
LC-MS [Methode 6] Rₜ = 1.76 min; MS [ESIpos]: m/z = 286 (M+H)⁺.

### Beispiel 33A

### Methyl-{4-cyclopropyl-5-oxo-3-[2-(trifluormethoxy)phenyl]-4,5-dihydro-1H-1,2,4-triazol-1-yl}acetat

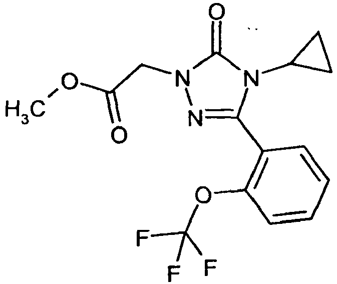

1.81 g (4.60 mmol) der Verbindung aus Beispiel 32A wurden in 15 ml Acetonitril gelöst und mit 1.64 g Cäsiumcarbonat (5.03 mmol) versetzt. Bei RT fügte man anschließend 0.48 ml (5.48 mmol) Chloressigsäuremethylester hinzu. Das Gemisch wurde 2 h unter Rückfluss erhitzt, anschließend bei RT mit 20 ml Essigsäureethylester verdünnt und mit 10 ml Wasser gewaschen. Die wässrige Phase wurde noch zweimal mit je 10 ml Essigsäureethylester extrahiert, über Magnesiumsulfat getrocknet, filtriert und im Vakuum eingeengt. Man erhielt so 1.46 mg (82% d. Th.) der Zielverbindung.
LC-MS [Methode 3] Rₜ = 1.05 min; MS [ESIpos]: m/z = 358 (M+H)⁺.
¹H-NMR (400MHz, CDCl₃): δ [ppm]= 0.58 - 0.66 (m, 2H), 0.78 - 0.85 (m, 2H), 2.95 (spt, 1H), 3.78 (s, 3H), 4.64 (s, 2H), 7.37 - 7.45 (m, 2H), 7.53 - 7.63 (m, 2H).

### Beispiel 34A

### {4-Cyclopropyl-5-oxo-3-[2-(trifluormethoxy)phenyl]-4,5-dihydro-1H-1,2,4-triazol-1-yl}essigsäure

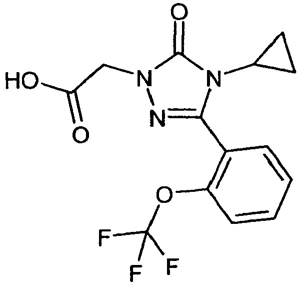

1.46 g (4.09 mmol) der Verbindung aus Beispiel 33A wurden in 8 ml Methanol gelöst und bei RT mit 4.9 ml (4.9 mmol) 1N Lithiumhydroxidlösung versetzt. Nach 30 min wurde das Lösungsmittel unter vermindertem Druck entfernt und der Rückstand in 20 ml Wasser und 20 ml Essigsäureethylester aufgenommen. Nach Trennen der Phasen wurde die wässrige Phase mit 1N Salzäure angesäuert und zweimal mit je 15 ml Essigsäureethylester extrahiert. Die vereinigten organischen Phasen wurden über Magnesiumsulfat getrocknet, filtriert, im Vakuum eingeengt und der Rückstand im Hochvakuum getrocknet. Man erhielt so 1.25 g (85% d. Th.) der Zielverbindung, die ohne weitere Reinigung weiter umgesetzt wurde.
LC-MS [Methode 6] Rₜ = 1.82 min; MS [ESIpos]: m/z = 344 (M+H)⁺.
¹H-NMR (400MHz, CDCl₃): δ [ppm]= 0.60 - 0.66 (m, 2H), 0.77 - 0.86 (m, 2H), 2.96 (spt, 1H), 4.67 (s, 2H), 7.37 - 7.45 (m, 2H), 7.55 - 7.63 (m, 2H).

### Ausführungsbeispiele:

### Beispiel 1

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propansäure (Diastereomerengemisch)

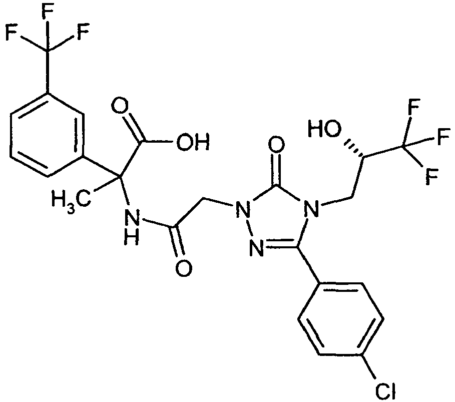

56 mg der Verbindung aus Beispiel 8A (0.15 mmol) wurden mit 29 mg (0.15 mmol) EDC und 21 mg (0.15 mmol) HOBt in 2.2 ml DMF 20 min bei RT gerührt, dann mit 50 mg (0.18 mmol) der Verbindung aus Beispiel 26A und 53 µl (0.31 mmol) N,N-Diisopropylethylamin versetzt. Die Mischung wurde 20 min bei RT gerührt, dann mit 1 ml 1N Salzsäure versetzt und komplett durch präparative HPLC (Methode 10) getrennt. Es wurden 54 mg (61% d. Th.) der Titelverbindung erhalten.
LC-MS [Methode 6]: Rₜ = 2.78 min; MS [ESIpos]: m/z = 581 (M+H)⁺
¹H-NMR (400MHz, DMSO-d₆): δ [ppm]= 1.85 (s, 3H), 3.82 (dd, 1H), 3.96 (br.d, 1H), 4.19 - 4.35 (m, 1H), 4.58 (s, 2H), 6.92 (d, 1H), 7.54 - 7.70 (m, 4H), 7.71 - 7.82 (m, 4H), 8.80 (s, 1H), 13.11 (br.s, 1H).

### Beispiel 2

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propanamid (Diastereomerengemisch)

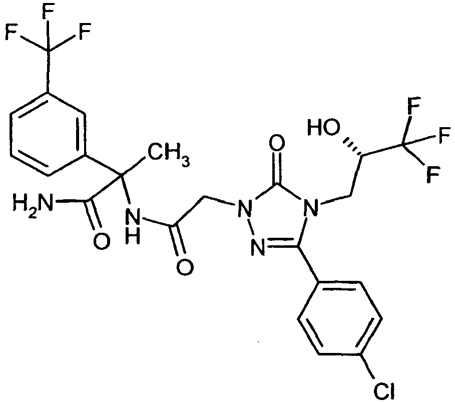

54 mg der Verbindung aus Beispiel 1 (90 µmol) und 24 mg (179 µmol) HOBt wurden in 1.3 ml DMF vorgelegt und mit 34 mg (179 µmol) EDC versetzt. Die Mischung wurde 20 min bei RT gerührt dann mit 5 ml einer Ammoniak-Lösung (35% in Wasser) versetzt und noch 20 min weitergerührt. 1 ml 1N Salzsäure wurde zugegeben und die komplette Mischung durch präparative HPLC (Methode 10) getrennt. Die geeignete Fraktion wurde am Rotationsverdampfer von den Lösungsmitteln befreit und der Rückstand am HV getrocknet. Es wurden 49 mg (94% d. Th.) der Titelverbindung erhalten.
LC-MS [Methode 6]: Rₜ = 2.28 min; MS [ESIpos]: m/z = 580 (M+H)⁺
¹H-NMR (400MHz, DMSO-d₆): δ [ppm]= 1.88 (d (1 s pro Diastereomer, 3H), 3.74 - 3.89 (dd, 1H), 3.94 (dd, 1H), 4.26 (m, 1H), 4.48 - 4.69 (m, 2H), 6.90 (t (1 d pro Diastereomer), 1H), 7.33 (br.s, 1H), 7.41 (br.d (1 br.s pro Diastereomer), 1H), 7.52 - 7.69 (m, 4H), 7.68 - 7.83 (m, 4H), 8.63 (s, 1H).

Die Diastereomeren aus Beispiel 2 konnten durch präparative Chromatographie an einer chiralen Phase (Methode 17b) getrennt werden: siehe Beispiel 3 und Beispiel 4.

### Beispiel 3

### (2S)-2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propanamid

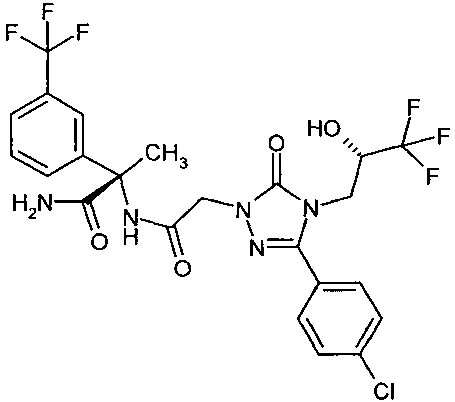

Zuerst eluiertes Diastereomer (19 mg) aus der Trennung von 49 mg der Verbindung aus Beispiel 2 nach Methode 17b.
Chirale analytische HPLC (Methode 18b): Rₜ = 1.73 min.

Alternativ kann die Titelverbindung nach folgendem Verfahren hergestellt werden:
3.50 g der Verbindung aus Beispiel 8A (9.57 mmol) und 2.04 g (14.36 mmol) HOBt wurden in 100 ml DMF vorgelegt und mit 2.75 g (14.36 mmol) EDC versetzt. Die Mischung wurde 15 min bei RT gerührt bevor 2.83 g (10.5 mmol) der Verbindung aus Beispiel 17A und 2.0 ml (11.5 mmol) N,N-Diisopropylethylamin zugegeben wurden. Die Reaktionsmischung wurde über Nacht bei RT gerührt, dann mit 1 L Wasser verdünnt und dreimal mit je 400 ml Essigsäureethylester extrahiert. Die vereinigten organischen Phasen wurden nacheinander zweimal mit 1N Salzsäure, einmal mit Wasser, zweimal mit einer gesättigten wässrigen Natriumhydrogencarbonatlösung und einmal mit einer gesättigten wässrigen Natriumchloridlösung gewaschen, dann über Natriumsulfat getrocknet und am Rotationsverdampfer vom Lösungsmittel befreit. Der Rückstand wurde durch präparative HPLC (Methode 10) gereinigt. Es wurden 4.09 g (74% d. Th.) der Titelverbindung erhalten.
LC-MS [Methode 3]: Rₜ = 1.20 min; MS [ESIpos]: m/z = 580 (M+H)⁺
¹H-NMR (400MHz, DMSO-d6): δ [ppm]= 1.88 (s, 3H), 3.82 (dd, 1H), 3.91 - 4.01 (m, 1H), 4.26 (br.s, 1H), 4.50 - 4.63 (m [AB], 2H), 6.91 (d, 1H), 7.33 (s, 1H), 7.42 (s, 1H), 7.54 - 7.60 (m, 1H), 7.60 - 7.66 (m, 3H), 7.69 - 7.80 (m, 4H), 8.63 (s, 1H).

### Beispiel 4

### (2R)-2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propanamid

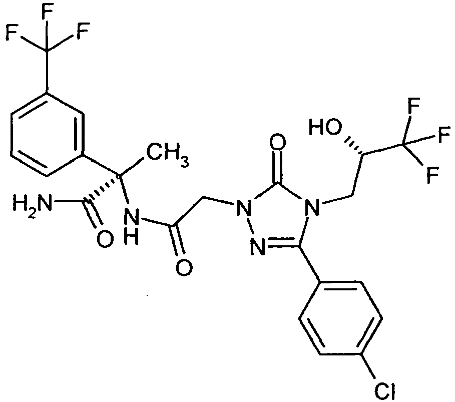

Zuletzt eluiertes Diastereomer (16 mg) aus der Trennung von 49 mg der Verbindung aus Beispiel 2 nach Methode 17b.
Chirale analytische HPLC (Methode 18b): Rₜ = 2.45 min.

Alternativ kann die Titelverbindung nach dem folgenden Verfahren hergestellt werden (A):
6.00 g der Verbindung aus Beispiel 8A (16.4 mmol) und 3.32 g (24.6 mmol) HOBt wurden in 160 ml DMF vorgelegt und mit 4.72 g (24.6 mmol) EDC versetzt. Die Mischung wurde 15 min bei RT gerührt bevor 4.85 g (18.0 mmol) der Verbindung aus Beispiel 16A und 3.4 ml (19.7 mmol) N,N-Diisopropylethylamin zugegeben wurden. Die Reaktionsmischung wurde über Nacht bei RT gerührt, dann mit 1.2 L Wasser verdünnt und dreimal mit je 400 ml Essigsäureethylester extrahiert. Die vereinigten organischen Phasen wurden nacheinander zweimal mit 1N Salzsäure, einmal mit Wasser, zweimal mit gesättigter wässriger Natriumhydrogencarbonatlösung und einmal mit gesättigter wässriger Natriumchloridlösung gewaschen, dann über Natriumsulfat getrocknet und am Rotationsverdampfer vom Lösungsmittel befreit. Der Rückstand wurde durch präparative HPLC (Methode 10) gereinigt. Es wurden 6.67 g (70% d. Th.) der Titelverbindung erhalten.
LC-MS [Methode 3]: Rₜ = 1.22 min; MS [ESIpos]: m/z = 580 (M+H)⁺
¹H-NMR (400MHz, DMSO-d₆): δ [ppm]= 1.88 (s, 3H), 3.78 - 3.87 (m, 1H), 3.92 - 3.99 (m, 1H), 4.26 (br.s, 1H), 4.53 - 4.63 (m [AB], 2H), 6.90 (d, 1H), 7.33 (s, 1H), 7.41 (s, 1H), 7.53 - 7.60 (m, 1H), 7.60 - 7.66 (m, 3H), 7.68 - 7.79 (m, 4H), 8.64 (s, 1H).

Alternativ kann die Titelverbindung nach dem folgenden Verfahren hergestellt werden (B):
1.80 g (4.92 mmol) der Verbindung aus Beispiel 8A (4.92 mmol) und 700 mg (5.41 mmol) HOBt wurden in 30 ml DMF vorgelegt und mit 944 mg (5.41 mmol) EDC versetzt. Die Mischung wurde 20 min bei RT gerührt dann auf eine Suspension von 1.46 g (5.41 mmol) der Verbindung aus Beispiel 27A und 1.03 ml (5.91 mmol) N,N-Diisopropylethylamin in 30 ml DMF zugetropft. Die Reaktionsmischung wurde 1 h bei RT gerührt, dann mit 500 ml 0.5N Salzsäure verdünnt und dreimal mit Essigsäureethylester extrahiert. Die vereinigten organischen Phasen wurden dreimal mit Wasser, dann einmal mit einer gesättigten wässrigen Natriumchloridlösung gewaschen und über Natriumsulfat getrocknet. Das Lösungsmittel wurde am Rotationsverdampfer entfernt. Der Rückstand wurde im HV getrocknet. Das so erhaltene Produkt (3.44 g), das der Verbindung aus Beispiel 6 in ca. 70 % Reinheit entspricht (4.21 mmol), wurde ohne Reinigung weiter umgesetzt: die Gesamtmenge wurde mit 1.02 g (7.57 mmol) HOBt in 40 ml DMF gelöst und mit 1.45 g (7.57 mmol) EDC versetzt. Die so erhaltene Lösung wurde 30 min bei RT gerührt, dann auf eine vorgelegte Ammoniak-Lösung (35% in Wasser, 45 ml) zugetropft. Diese Mischung wurde 20 min gerührt, dann am Rotationsverdampfer eingeengt. Zum Rückstand wurden 500 ml Wasser zugegeben. Die Lösung wurde dreimal mit je 250 ml Essigsäureethylester extrahiert. Die vereinigten organischen Phasen wurden nacheinander dreimal mit 1M Salzsäure, einmal mit Wasser, zweimal mit einer gesättigten wässrigen Natriumhydrogencarbonatlösung und einmal mit einer gesättigten wässrigen Natriumchloridlösung gewaschen, dann über Natriumsulfat getrocknet und am Rotationverdampfer vom Lösungsmittel befreit. Der Rückstand wurde durch präparative HPLC (Methode 7) gereinigt. Es wurden 2.30 g (3.97 mmol, 80% d. Th.) der Titelverbindung erhalten.

### Beispiel 5

### (2R)-2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propansäure

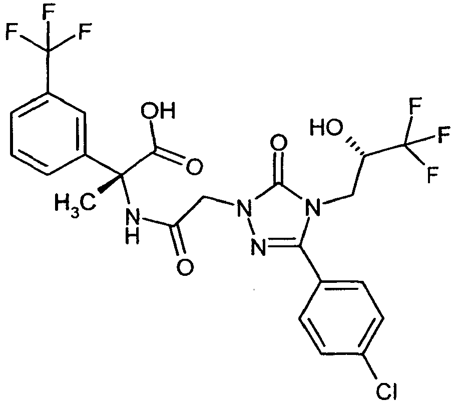

250 mg der Verbindung aus Beispiel 8A (0.68 mmol) und 92 mg (0.68 mmol) HOBt wurden in 5 ml DMF vorgelegt und mit 131 mg (0.68 mmol) EDC versetzt. Die Mischung wurde 20 min bei RT gerührt dann auf eine Lösung von 221 mg (0.82 mmol) (2R)-2-Amino-2-[3-(trifluormethyl)phenyl]propionsäure-Hydrochlorid (Firma Netchem, New Brunswick NJ 08901, USA, Artikel-Nr: 506085-HCl) und 119 µl (0.68 mmol) N,N-Diisopropylethylamin in 2 ml DMF zugetropft. Die Reaktionsmischung wurde 20 min bei RT gerührt, dann mit 1 ml 1N Salzsäure versetzt und komplett durch präparative HPLC (Methode 10) gereinigt. Die geeignete Fraktion wurde am Rotationsverdampfer von den Lösungsmitteln befreit und der Rückstand am HV getrocknet. Es wurden 260 mg (65% d. Th.) der Titelverbindung erhalten.
LC-MS [Methode 3]: Rₜ = 1.23 min; MS [ESIpos]: m/z = 581 (M+H)⁺
¹H-NMR (400MHz, DMSO-d₆): δ [ppm]= 1.85 (s, 3H), 3.76 - 3.88 (m, 1H), 3.90 - 4.01 (m, 1H), 4.26 (br.s, 1H), 4.51 - 4.67 (m, 2H), 6.92 (d, 1H), 7.55 - 7.71 (m, 4H), 7.71 - 7.83 (m, 4H), 8.80 (s, 1H), 13.10 (s, 1H).

### Beispiel 6

### (2S)-2-[({3-(4-Chlorphenyl)-5-oxo-4-[(2R)-3,3,3-trifluor-2-hydroxypropyl]-4,5-dihydro-1H-1,2,4-triazol-1-yl}acetyl)amino]-2-[3-(trifluormethyl)phenyl]propanamid

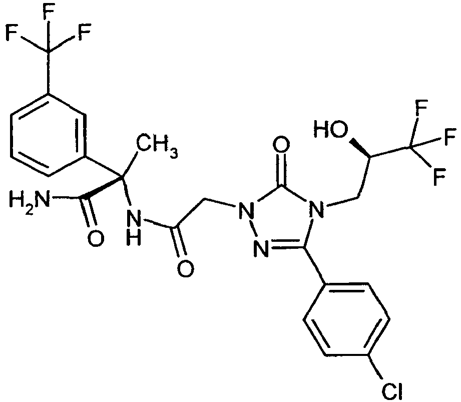

318 mg der Verbindung aus Beispiel 9A (0.87 mmol) wurden in 5 ml DMF gelöst und mit 250 mg (1.30 mmol) EDC und 176 mg (1.30 mmol) HOBt versetzt. Nach 30 min Rühren bei RT wurden 269 mg (1 mmol) der Verbindung aus Beispiel 17A, dann 303 µl (1.74 mmol) N,N-Diisopropylethylamin zugegeben. Die Mischung wurde 1 h bei RT gerührt, dann komplett durch präparative HPLC (Methode 10) getrennt. Die geeignete Fraktion wurde am Rotationsverdampfer von den Lösungsmitteln befreit und der Rückstand am HV getrocknet. Es wurden 244 mg (47% d. Th.) der Titelverbindung erhalten.
LC-MS [Methode 3]: Rₜ = 1.22 min; MS [ESIpos]: m/z = 580 (M+H)⁺
¹H-NMR (400MHz, DMSO-d₆): δ [ppm]= 1.88 (s, 3H), 3.77 - 3.87 (dd, 1H), 3.90 - 4.00 (dd, 1H), 4.26 (m., 1H), 4.52 - 4.64 (m [AB], 2H), 6.90 (d, 1H), 7.33 (s, 1H), 7.41 (s, 1H), 7.53 - 7.60 (m, 1H), 7.60 - 7.67 (m, 3H), 7.68 - 7.80 (m, 4H), 8.64 (s, 1H).

### Beispiel 7

### 2-({[3-(4-Chlorphenyl)-4-cyclopropyl-5-oxo-4,5-dihydro-1H-1,2,4-triazol-1-yl]acetyl}amino)-2-[3-(trifluormethyl)phenyl]propansäure

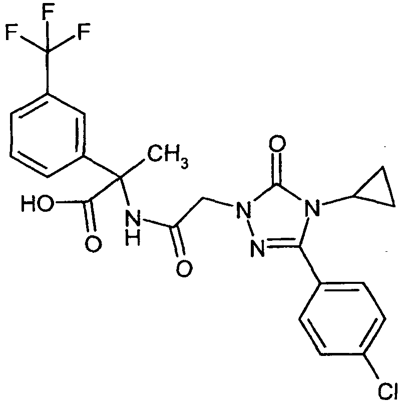

18.2 mg (62 µmol) [3-(4-Chlorphenyl)-4-cyclopropyl-5-oxo-4,5-dihydro-1*H*-1,2,4-triazol-1-yl]-essigsäure (Herstellung siehe WO 2007/134862, Beispiel 88A) wurden in 900 µl DMF gelöst und mit 8.4 mg (62 µmol) HOBt, dann 11.8 mg (62 µmol) EDC versetzt und 20 min bei RT gerührt. Anschließend wurden 20 mg (74 µmol) der Verbindung aus Beispiel 26A und 22 µl (124 µmol) N,N-Diisopropylethylamin zugegeben. Die Mischung wurde 20 min bei RT weiter gerührt, dann komplett durch präparative HPLC (Methode 10) getrennt. Es wurden 12 mg (38% d. Th.) der Titelverbindung erhalten.
LC-MS [Methode 1]: Rₜ = 1.92 min; MS [ESIpos]: m/z = 509 (M+H)⁺
¹H-NMR (400MHz, DMSO-d₆): δ [ppm]= 0.51 - 0.63 (m, 2H), 0.83 - 0.94 (m, 2H), 1.84 (s, 3H), 3.11 - 3.22 (m, 1H), 4.51 (s, 2H), 7.55 - 7.63 (m, 3H), 7.66 (d, 1H), 7.70 - 7.88 (m, 4H), 8.75 (s, 1H), 13.10 (br.s, 1H).

### Beispiel 8

### 2-({[3-(4-Chlorphenyl)-4-cyclopropyl-5-oxo-4,5-dihydro-1H-1,2,4-triazol-1-yl]acetyl}amino)-2-[3-(trifluormethyl)phenyl]propanamid

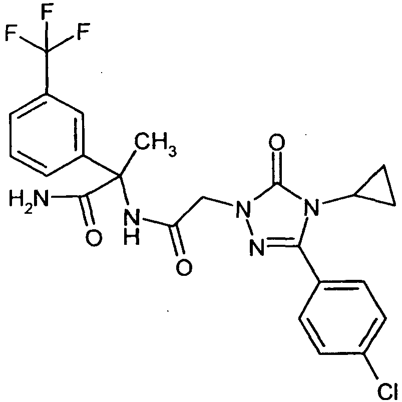

18.0 mg (36 µmol) der Verbindung aus Beispiel 7 wurden in 520 µl DMF gelöst und mit 9.6 mg (71 µmol) HOBt, dann 14 mg (71 µmol) EDC versetzt und 20 min bei RT gerührt. Anschließend wurden 5 ml Ammoniak (35% in Wasser) zugegeben. Die Mischung wurde 20 min bei RT weiter gerührt, dann komplett durch präparative HPLC (Methode 10) getrennt. Es wurden 9 mg (50% d. Th.) der Titelverbindung erhalten.
LC-MS [Methode 6]: Rₜ = 2.17 min; MS [ESIpos]: m/z = 508 (M+H)⁺
¹H-NMR (400MHz, DMSO-d₆): δ [ppm]= 0.49 - 0.65 (m, 2H), 0.84 - 0.93 (m, 2H), 1.87 (s, 3H), 3.17 (dt, 1H), 4.42 - 4.57 (m [AB], 2H), 7.33 (s, 1H), 7.41 (s, 1H), 7.53 - 7.65 (m, 4H), 7.67 - 7.75 (m, 2H), 7.76 - 7.86 (m, 2H), 8.55 (s, 1H).

### Beispiel 9

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[2-fluor-3-(trifluormethyl)phenyl]propanamid (Diastereomerengemisch)

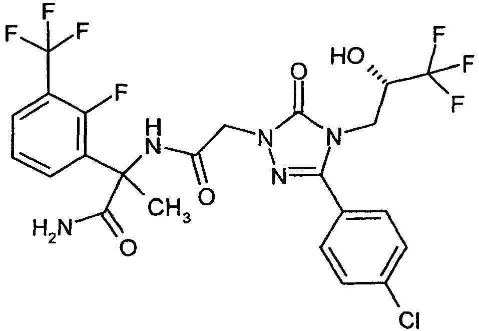

100 mg der Verbindung aus Beispiel 8A (0.27 mmol) wurden zusammen mit 109 mg (ca. 0.33 mmol) der Verbindung aus Beispiel 18A, 79 mg (0.41 mmol) EDC und 55 mg (0.41 mmol) HOBt mit 3 ml DMF vorgelegt, dann mit 57 µl (0.33 mmol) N,N-Diisopropylethylamin versetzt. Die Mischung wurde 1 h bei RT gerührt, dann komplett durch präparative HPLC (Methode 10) getrennt. Es wurden 90 mg (55% d. Th.) der Titelverbindung erhalten.
LC-MS [Methode 3]: Rₜ = 1.21 min; MS [ESIpos]: m/z = 598 (M+H)⁺
¹H-NMR (400MHz, DMSO-d₆): δ [ppm]= 1.90 (s, 3H), 3.74 - 3.86 (m, 1H), 3.88 - 4.00 (m, 1H), 4.25 (br.s, 1H), 4.42 - 4.58 (m, 2H), 6.89 (d, 1H), 7.22 (s, 1H), 7.38 (t, 1H), 7.44 (s, 1H), 7.59 - 7.65 (m, 2H), 7.69 (t, 1H), 7.73 - 7.79 (m, 2H), 7.85 (t, 1H), 8.54 (d, 1H).

Die Diastereomeren aus Beispiel 9 konnten durch präparative Chromatographie an einer chiralen Phase (Methode 15) getrennt werden: siehe Beispiel 10 und Beispiel 11.

### Beispiel 10

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[2-fluor-3-(trifluormethyl)phenyl]propanamid (Diastereomer I)

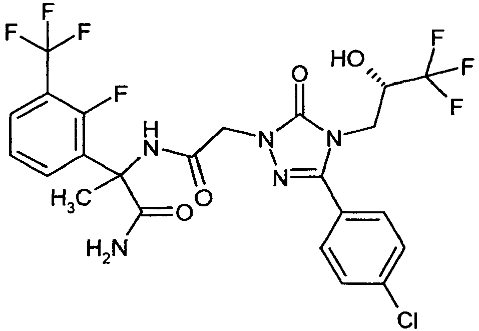

Zuerst eluiertes Diastereomer (31 mg) aus der Trennung von 85 mg der Verbindung aus Beispiel 9 nach Methode 15.
LC-MS [Methode 3]: Rₜ = 1.21 min; MS [ESIpos]: m/z = 598 (M+H)⁺
Analytische chirale HPLC [Methode 16]: Rₜ = 3.57 min.
¹H-NMR (400MHz, DMSO-d₆): δ [ppm]= 1.90 (s, 3H), 3.74 - 3.86 (m, 1H), 3.89 - 4.00 (m, 1H), 4.24 (br.s, 1H), 4.42 - 4.57 (m [AB], 2H), 6.89 (d, 1H), 7.22 (s, 1H), 7.38 (t, 1H), 7.44 (s, 1H), 7.59 - 7.65 (m, 2H), 7.69 (t, 1H), 7.72 - 7.79 (m, 2H), 7.83 (t, 1H), 8.55 (s, 1H).

### Beispiel 11

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[2-fluor-3-(trifluormethyl)phenyl]propanamid (Diastereomer II)

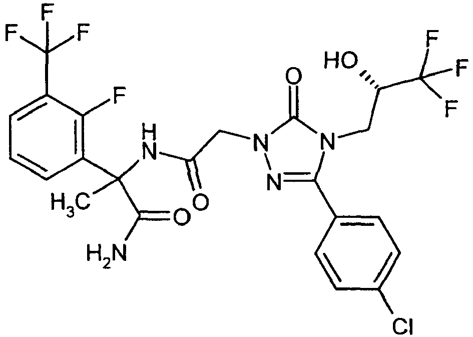

Zuletzt eluiertes Diastereomer (30 mg) aus der Trennung von 85 mg der Verbindung aus Beispiel 9 nach Methode 15.
LC-MS [Methode 3]: Rₜ = 1.20 min; MS [ESIpos]: m/z = 598 (M+H)⁺
Analytische chirale HPLC [Methode 16]: Rₜ = 4.19 min.
¹H-NMR (400MHz, DMSO-d₆): δ [ppm]= 1.90 (s, 3H), 3.74 - 3.86 (m, 1H), 3.89 - 3.97 (m, 1H), 4.25 (br.s, 1H), 4.42 - 4.57 (m [AB], 2H), 6.89 (d, 1H), 7.22 (s, 1H), 7.38 (t, 1H), 7.45 (s, 1H), 7.59 - 7.65 (m, 2H), 7.69 (t, 1H), 7.72 - 7.79 (m, 2H), 7.85 (t, 1H), 8.54 (s, 1H).

### Beispiel 12

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]butanamid (Diastereomerengemisch)

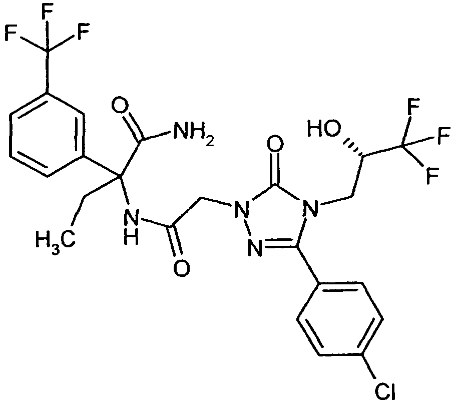

160 mg der Verbindung aus Beispiel 8A (0.44 mmol) wurden zusammen mit 126 mg (0.66 mmol) EDC und 89 mg (0.66 mmol) HOBt in 4 ml DMF 20 min gerührt, dann mit 136 mg (0.48 mmol) der Verbindung aus Beispiel 19A und 99 µl (0.57 mmol) N,N-Diisopropylethylamin versetzt. Die Mischung wurde 2 h bei RT gerührt, dann komplett durch präparative HPLC (Methode 10) getrennt. Die geeignete Fraktion wurde am Rotationsverdampfer von den Lösungsmitteln befreit und der Rückstand am HV getrocknet. Es wurden 59 mg (22% d. Th.) der Titelverbindung erhalten.
LC-MS [Methode 3]: Rₜ = 1.24 min; MS [ESIpos]: m/z = 594 (M+H)⁺

Die Diastereomeren aus Beispiel 12 konnten durch präparative Chromatographie an einer chiralen Phase (Methode 17a) getrennt werden: siehe Beispiel 13 und Beispiel 14.

### Beispiel 13

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]butanamid (Diastereomer I)

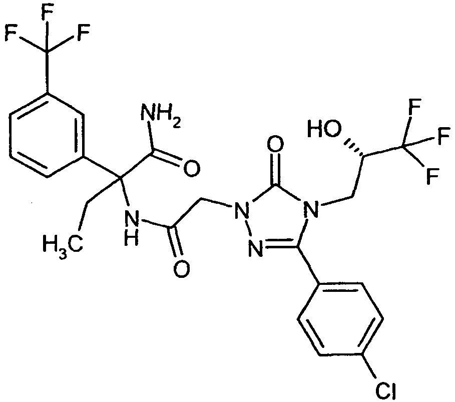

Zuerst eluierendes Diastereomer (29 mg) aus der Trennung von 59 mg der Verbindung aus Beispiel 12 nach Methode 17a.
Chirale analytische HPLC [Methode 18a]: Rₜ = 5.2 min.
LC-MS [Methode 5]: Rₜ = 1.09 min; MS [ESIpos]: m/z = 594 (M+H)⁺
¹H-NMR (400MHz, DMSO-d₆): δ [ppm]= 0.79 (t, 3H), 2.42 - 2.64 (m, 2H), 3.76 - 3.86 (m, 1H), 3.91 - 3.99 (m, 1H), 4.25 (br.s, 1H), 4.55 - 4.66 (m [AB], 2H), 6.89 (d, 1H), 7.40 (d, 2H), 7.52 - 7.58 (m, 1H), 7.58 - 7.65 (m, 3H), 7.71 (d, 1H), 7.73 - 7.80 (m, 3H), 8.43 (s, 1H).

### Beispiel 14

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]butanamid (Diastereomer II)

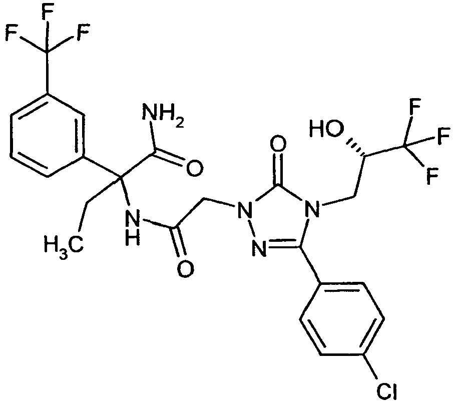

Zuletzt eluierendes Diastereomer (26 mg) aus der Trennung von 59 mg der Verbindung aus Beispiel 12 nach Methode 17a.
Chirale analytische HPLC [Methode 18a]: Rₜ = 9.1 min
LC-MS [Methode 5]: Rₜ = 1.09 min; MS [ESIpos]: m/z = 594 (M+H)⁺
¹H-NMR (400MHz, DMSO-d₆): δ [ppm]= 0.80 (t, 3H), 2.56 - 2.64 (m, 2H), 3.76 - 3.87 (m, 1H), 3.89 - 4.00 (m, 1H), 4.26 (br.s, 1H), 4.61 (s, 2H), 6.89 (d, 1H), 7.40 (d, 2H), 7.52 - 7.58 (m, 1H), 7.59 - 7.65 (m, 3H), 7.67 - 7.78 (m, 4H), 8.44 (s, 1H).

### Beispiel 15

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-cyclopropyl-2-[3-(trifluormethyl)phenyl]acetamid (Diastereomerengemisch)

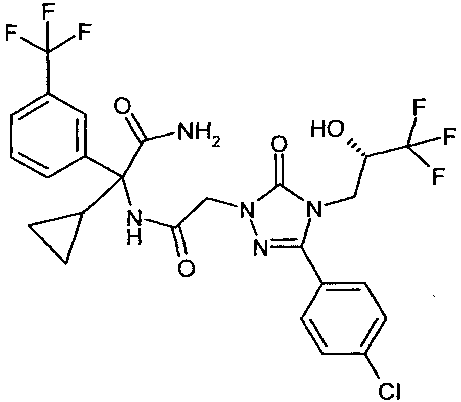

27 mg der Verbindung aus Beispiel 8A (74 µmol) wurden zusammen mit 24 mg (81 µmol) der Verbindung aus Beispiel 20A, 20 mg (0.10 mmol) EDC und 14 mg (0.10 mmol) HOBt mit 550 µl DMF vorgelegt, dann mit 26 µl (0.15 mmol) N,N-Diisopropylethylamin versetzt. Die Mischung wurde 2 h bei RT gerührt, dann komplett durch präparative HPLC (Methode 10) getrennt. Es wurden 24 mg (25% d. Th.) der Titelverbindung erhalten.
LC-MS [Methode 3]: Rₜ = 1.26 min; MS [ESIpos]: m/z = 606 (M+H)⁺
¹H-NMR (400MHz, DMSO-d₆): δ [ppm]= 0.30 - 0.44 (m, 2H), 0.45 - 0.62 (m, 2H), 1.80 - 1.88 (m, 1H), 3.77 - 3.85 (dd, 1H), 3.91 - 3.98 (dd, 1H), 4.25 (m., 1H), 4.53 - 4.61 (m [AB], 2H), 6.89 (2d, 1H), 7.16 (br.s, 1H), 7.34 (s, 1H), 7.49 - 7.56 (m, 1H), 7.57 - 7.66 (m, 3H), 7.70 - 7.80 (m, 3H), 7.87 (br.s, 1H), 8.32 (s, 1H).

### Beispiel 16

### (2R)-2-(2-Chlorphenyl)-2-[({3-(4-chlorphenyl)-5-oxo-4-[(2S)-3,3,3-trifluor-2-hydroxypropyl]-4,5-dihydro-1H-1,2,4-triazol-1-yl}acetyl)amino]propansäure

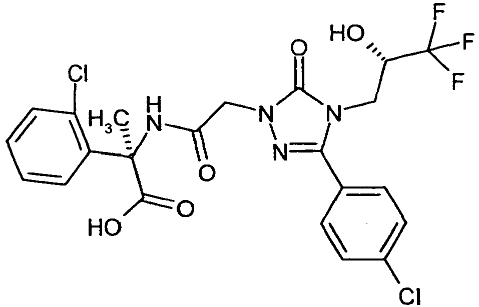

134 mg der Verbindung aus Beispiel 8A (0.37 mmol) wurden mit 52 mg (0.37 mmol) HOBt in 5 ml DMF bei RT vorgelegt. 70 mg (0.37 mmol) EDC wurden zugegeben und die Mischung 20 min bei RT gerührt. Die entstandene Lösung wurde zu einer Suspension von 95 mg (0.40 mmol) (2R)-2-Amino-2-(2-chlorphenyl)propionsäure-Hydrochlorid (Firma Netchem, New Brunswick NJ 08901, USA, Artikel-Nr: 506093-HCl) und 159 µl (0.91 mmol) N,N-Diisopropylethylamin in 3 ml DMF zugetropft und die resultierende Mischung 4 h bei RT gerührt. Nach Zugabe von 2 ml 1N Salzsäure wurde die komplette Reaktionsmischung durch präparative HPLC (Methode 10) getrennt. Es wurden 63 mg (31% d. Th.) der Titelverbindung erhalten.
LC-MS [Methode 3]: Rₜ = 1.12 min; MS [ESIpos]: m/z = 547 (M+H)⁺
¹H-NMR (400MHz, DMSO-d₆): δ [ppm]= 1.96 (s, 3H), 3.71 - 3.86 (m, 1H), 3.87 - 3.99 (m, 1H), 4.26 (br.s, 1H), 4.36 - 4.59 (m [AB], 2H), 6.91 (d, 1H), 7.22 - 7.39 (m, 3H), 7.55 - 7.66 (m, 3H), 7.69 - 7.82 (m, 2H), 8.45 (s, 1H), 13.53 (br.s, 1H).

### Beispiel 17

### (2R)-2-(2-Chlorphenyl)-2-[({3-(4-chlorphenyl)-5-oxo-4-[(2S)-3,3,3-trifluor-2-hydroxypropyl]-4,5-dihydro-1H-1,2,4-triazol-1-yl}acetyl)amino]propanamid

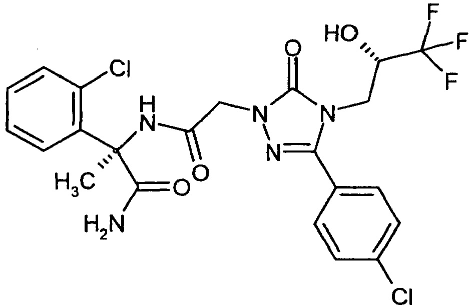

64 mg der Verbindung aus Beispiel 8A (0.17 mmol) wurden zusammen mit 45 mg (0.19 mmol) der Verbindung aus Beispiel 22A, 47 mg (0.24 mmol) EDC und 33 mg (0.24 mmol) HOBt mit 2 ml DMF vorgelegt, dann mit 36 µl (0.21 mmol) N,N-Diisopropylethylamin versetzt. Die Mischung wurde 1 h bei RT gerührt, dann komplett durch präparative HPLC (Methode 10) getrennt. Es wurden 46 mg (48% d. Th.) der Titelverbindung erhalten.
LC-MS [Methode 4]: Rₜ = 0.96 min; MS [ESIpos]: m/z = 546 (M+H)⁺
¹H-NMR (400MHz, DMSO-d₆): δ [ppm]= 1.91 (s, 3H), 3.73 - 4.00 (m, 2H), 4.26 (br.s, 1H), 4.35 - 4.55 (m [AB], 2H), 6.80 (s, 1H), 6.91 (d, 1H), 7.24 - 7.36 (m, 3H), 7.40 (s, 1H), 7.56 - 7.70 (m, 3H), 7.72 - 7.83 (m, 2H), 8.37 (s, 1H).

### Beispiel 18

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-3,3,3-trifluor-2-[3-(trifluormethyl)phenyl]propanamid (Diastereomerengemisch)

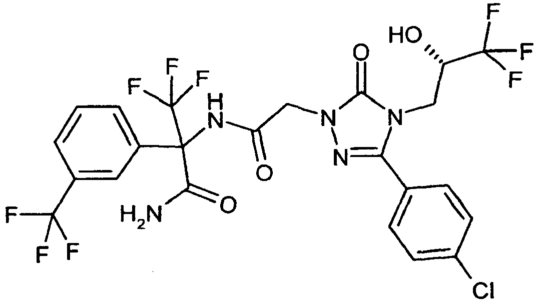

100 mg der Verbindung aus Beispiel 30A (0.21 mmol) wurden in 1.5 ml Dichlormethan gelöst und mit 33.4 mg (0.25 mmol) Ghosez Reagenz (1-Chlor-N,N,2-trimethylprop-1-en-1-amin) versetzt. Diese Lösung wurde 10 min bei RT gerührt, dann mit einer Lösung von 65.6 mg (0.23 mmol) der Verbindung aus Beispiel 29A und 27 µl (0.33 mmol) Pyridin in 1.5 ml Dichlormethan versetzt. Die Mischung wurde 2h bei RT gerührt. Noch 72 mg (0.25 mmol) der Verbindung aus Beispiel 29A wurden zugegeben. Die Mischung wurde über Nacht gerührt, dann am Rotationsverdampfer von den flüchtigen Komponenten befreit. Zur Abspaltung der tert.-Butyl-dimethylsilyl-Schutzgruppe wurde der Rückstand in 5 ml THF aufgenommen und mit 0.5 ml Wasser, dann mit 1 ml einer 1M Tetra-n-butylammoniumfluorid-Lösung in THF versetzt. Die erhaltene Lösung wurde 75 min bei RT gerührt. Das THF wurde am Rotationsverdampfer entfernt und der Rückstand durch präparative HPLC (Methode 10) getennt. Es wurden 71 mg (53% d. Th.) der Titelverbindung erhalten.
LC-MS [Methode 5]: Rₜ = 1.12 min; MS [ESIpos]: m/z = 634 (M+H)⁺
¹H-NMR (400MHz, DMSO-d₆): δ [ppm]= 3.76 - 3.88 (m, 1H), 3.91 - 4.01 (m, 1H), 4.25 (m, 1H), 4.72 - 4.87 (m, 2H), 6.92 (d, 1H), 7.60 - 7.82 (m, 8H), 7.91 (d, 1H), 8.00 (s, 1H), 9.43 (s, 1H).

Eine analytische HPLC an einer chiralen Phase (Methode 18a) zeigt 66 % d.e. (Zuerst eluiertes Diastereomer, Rₜ = 10 min, 83%; zuletzt eluiertes Diastereomer, Rₜ = 16 min, 17%).

### Beispiel 19

### (2R)-2-[({3-(4-Chlorphenyl)-5-oxo-4-[(1E)-3,3,3-trifluorprop-1-en-1-yl]-4,5-dihydro-1H-1,2,4-triazol-1-yl}acetyl)amino]-2-[3-(trifluormethyl)phenyl]propanamid

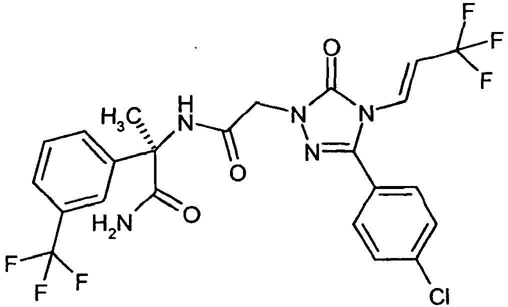

35 mg der Verbindung aus Beispiel 11A (0.10 mmol) wurden zusammen mit 32.5 mg (0.12 mmol) der Verbindung aus Beispiel 16A, 23 mg (0.12 mmol) EDC und 17 mg (0.12 mmol) HOBt mit 1.1 ml DMF vorgelegt, dann mit 26 µl (0.15 mmol) N,N-Diisopropylethylamin versetzt. Die Mischung wurde 30 min bei RT gerührt, dann mit 1 ml 1N Salzsäure versetzt und komplett durch präparative HPLC (Methode 10) getrennt. Die geeignete Fraktion wurde am Rotationsverdampfer von den Lösungsmitteln befreit und der Rückstand am HV getrocknet. Es wurden 55 mg (97% d. Th.) der Titelverbindung erhalten.
LC-MS [Methode 4]: Rₜ = 1.15 min; MS [ESIpos]: m/z = 562 (M+H)⁺
¹H-NMR (400MHz, DMSO-d₆): δ [ppm]= 1.88 (s, 3H), 4.58 - 4.71 (m [AB], 2H), 6.84 (dq, 1H), 7.16 (dq, 1H), 7.32 (s, 1H), 7.41 (s, 1H), 7.53 - 7.61 (m, 1H), 7.60 - 7.70 (m, 5H), 7.71 - 7.78 (m, 2H), 8.68 (s, 1H).

### Beispiel 20

### 2-[({4-Cyclopropyl-5-oxo-3-[2-(trifluormethoxy)phenyl]-4,5-dihydro-1H-1,2,4-triazol-1-yl}acetyl)amino]-2-[3-(trifluormethyl)phenyl]propanamid (Enantiomerengemisch)

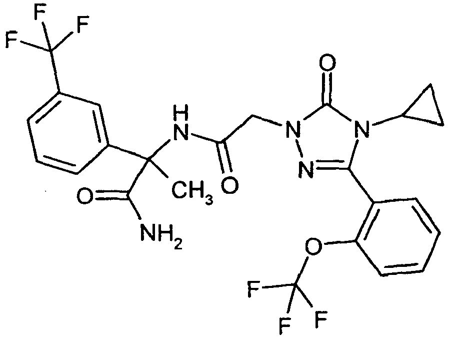

145 mg (0.40 mmol) der Verbindung aus Beispiel 34A wurden in 2 ml DMF gelöst, mit 115 mg (0.60 mmol) EDC sowie 81 mg (0.60 mmol) HOBt versetzt und 10 Minuten bei Raumtemperatur nachgerührt. Anschließend fügte man 102 mg (0.44 mmol) der Verbindung aus Beispiel 12A hinzu und ließ das Gemisch 12 h bei Raumtemperatur rühren. Das Rohgemisch wurde direkt durch präparative HPLC [Methode 19] gereinigt. Man erhielt so 136 mg (58% d. Th.) der Zielverbindung.
LC-MS [Methode 1] Rₜ = 1.86 min; MS [ESIpos]: m/z = 558 (M+H)⁺
¹H-NMR (400MHz, CDCl₃): δ [ppm]= 0.61 - 0.68 (m, 2H), 0.80 - 0.87 (m, 2H), 2.01 (s, 3H), 2.97 (spt, 1H), 4.48 und 4.55 (2d, 2H), 5.42 (br.s, 1H), 5.70 (br.s, 1H), 7.39 - 7.53 (m, 3H), 7.54 - 7.70 (m, 5H), 7.80 (s, 1H).

Die Enantiomeren aus Beispiel 20 konnten durch präparative Chromatographie an einer chiralen Phase (Methode 11) getrennt werden: siehe Beispiel 21 und Beispiel 22

### Beispiel 21

### 2-[({4-Cyclopropyl-5-oxo-3-[2-(trifluormethoxy)phenyl]-4,5-dihydro-1H-1,2,4-triazol-1-yl}acetyl)amino]-2-[3-(trifluormethyl)phenyl]propanamid (Enantiomer I)

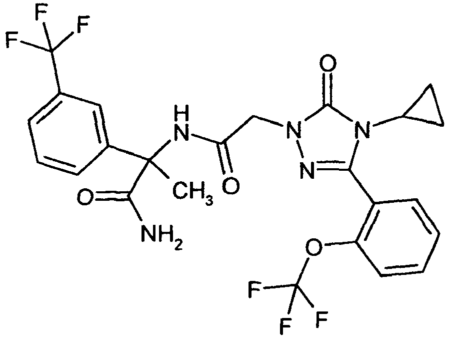

Zuerst eluierendes Enantiomer (36 mg) aus der Trennung von 119 mg der Verbindung aus Beispiel 20 nach Methode 11.
Analytische chirale HPLC [Methode 12]: Rₜ = 4.23 min.

### Beispiel 22

### 2-[({4-Cyclopropyl-5-oxo-3-[2-(trifluormethoxy)phenyl]-4,5-dihydro-1H-1,2,4-triazol-1-yl}acetyl)amino]-2-[3-(trifluormethyl)phenyl]propanamid (Enantiomer II)

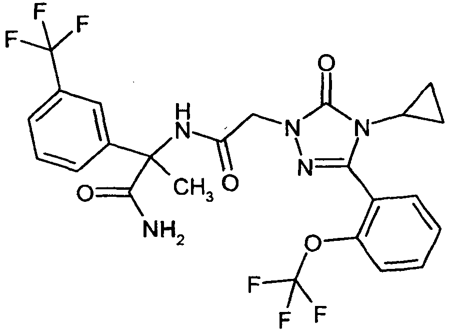

Zuletzt eluierendes Enantiomer (41 mg) aus der Trennung von 119 mg der Verbindung aus Beispiel 20 nach Methode 11.
Analytische chirale HPLC [Methode 12]: Rₜ = 5.04 min.

### Beispiel 23

### 2-({[3-(4-Chlorphenyl)-4-(2-fluorbenzyl)-5-oxo-4,5-dihydro-1H-1,2,4-triazol-1-yl]acetyl}amino)-2-[3-(trifluormethyl)phenyl]propanamid (Enantiomerengemisch)

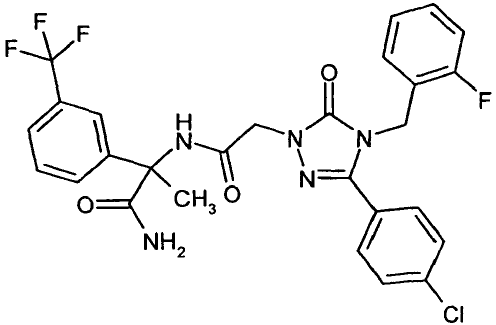

109 mg (0.28 mmol) [3-(4-Chlorphenyl)-4-(2-fluorbenzyl)-5-oxo-4,5-dihydro-1*H*-1,2,4-triazol-1-yl]-essigsäure (Herstellung siehe WO 2007/134862, Beispiel 156A) wurden in 2 ml DMF gelöst mit 79 mg (0.41 mmol) EDC sowie 56 mg (0.41 mmol) HOBt versetzt und 10 Minuten bei Raumtemperatur nachgerührt. Anschließend fügte man 70 mg (0.30 mmol) der Verbindung aus Beispiel 12A hinzu und ließ das Gemisch 20 h bei Raumtemperatur rühren. Das Rohgemisch wurde direkt durch präparative HPLC [Methode 19] gereinigt. Man erhielt so 109 mg (69% d. Th.) der Zielverbindung.
LC-MS [Methode 1] Rₜ = 2.10 min; MS [ESIpos]: m/z = 576 (M+H)⁺
¹H-NMR (400MHz, CDCl₃): δ [ppm]= 2.05 (s, 2H), 4.54 (d, 1H), 4.61 (d, 1H), 5.03 (s, 2H), 5.41 (br.s, 1H), 5.55 (br.s, 1H), 7.03 (t, 1H), 7.09 (t, 1H), 7.14 - 7.21 (m, 1H), 7.23 - 7.31 (m, 1H), 7.36 - 7.45 (m, 4H), 7.46 - 7.53 (m, 1H), 7.54 - 7.66 (m, 2H), 7.69 (s, 1H), 7.97 (s, 1H).

Die Enantiomeren aus Beispiel 23 konnten durch präparative Chromatographie an einer chiralen Phase (Methode 17a) getrennt werden: siehe Beispiel 24 und Beispiel 25.

### Beispiel 24

### 2-({[3-(4-Chlorphenyl)-4-(2-fluorbenzyl)-5-oxo-4,5-dihydro-1H-1,2,4-triazol-1-yl]acetyl}amino)-2-[3-(trifluormethyl)phenyl]propanamid (Enantiomer I)

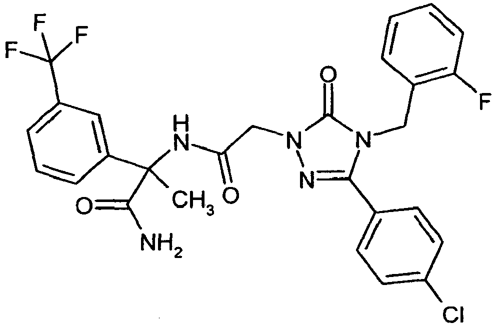

Zuerst eluierendes Enantiomer (11 mg) aus der Trennung von 108 mg der Verbindung aus Beispiel 23 nach Methode 17a.
Analytische chirale HPLC [Methode 18a]: Rₜ = 2.12 min.

### Beispiel 25

### 2-({[3-(4-Chlorphenyl)-4-(2-fluorbenzyl)-5-oxo-4,5-dihydro-1H-1,2,4-triazol-1-yl]acetyl}amino)-2-[3-(trifluormethyl)phenyl]propanamid (Enantiomer II)

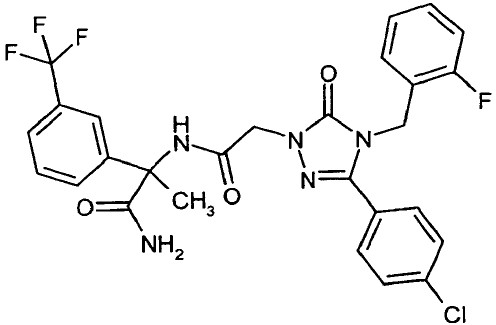

Zuletzt eluierendes Enantiomer (31 mg) aus der Trennung von 108 mg der Verbindung aus Beispiel 23 nach Methode 17a.
Analytische chirale HPLC [Methode 18a]: Rₜ = 2.48 min.

### B. Bewertung der pharmakologischen Wirksamkeit

Die pharmakologische Wirkung der erfindungsgemäßen Verbindungen kann in folgenden Assays gezeigt werden:

### Abkürzungen:

- EDTA: Ethylendiamintetraessigsäure
- DMEM: Dulbecco's Modified Eagle Medium
- FCS: Fötales Kälberserum
- HEPES: 4-(2-Hydroxyethyl)-1-piperazinethansulfonsäure
- SmGM: Smooth Muscle Cell Growth Media
- Tris-HCl: 2-Amino-2-(hydroxymethyl)-1,3-propandiol-Hydrochlorid
- UtSMC: Uterine Smooth Muscle Cells

### B-1. Zellulärer in vitro-Test zur Bestimmung der Vasopressin-Rezeptor-Aktivität

Die Identifizierung von Agonisten und Antagonisten der V1a und V2 Vasopressin Rezeptoren aus Mensch und Ratte sowie die Quantifizierung der Wirksamkeit der hier beschriebenen Substanzen erfolgte mit Hilfe von rekombinanten Zelllinien. Diese Zellen leiten sich ursprünglich von einer Ovarepithelzelle des Hamsters (Chinese Hamster Ovary, CHO K1, ATCC: American Type Culture Collection, Manassas, VA 20108, USA) ab. Die Testzelllinien exprimieren konstitutiv eine modifizierte Form des Calcium-sensitiven Photoproteins Aequorin, das nach Rekonstitution mit dem Co-Faktor Coelenterazin bei Erhöhungen der freien Calcium-Konzentration im Licht emittiert (Rizzuto R., Simpson A.W., Brini M., Pozzan T.; Nature 358 (1992) 325-327). Zusätzlich sind die Zellen stabil transfiziert mit den V1a oder V2 Rezeptoren des Menschen oder der Ratte. Im Falle der Gs-koppelnden V2-Rezeptoren sind die Zellen mit einem weiteren Gen, das für das promiskuitive G_{α16}-Protein kodiert (Amatruda T.T., Steele D.A., Slepak V.Z., Simon M.I., Proc. Na. Acad. Sci. USA 88 (1991), 5587-5591), entweder unabhängig oder als Fusionsgen stabil transfiziert. Die resultierenden Vasopressin Rezeptor-Testzellen reagieren auf Stimulation der rekombinant exprimierten Vasopressin-Rezeptoren mit einer intrazellulären Freisetzung von Calcium-Ionen, die durch die resultierende Aequorin-Lumineszenz mit einem geeignetem Luminometer quantifiziert werden kann (Milligan G., Marshall F., Rees S., Trends in Pharmaco. Sci. 17 (1996) 235-237).

Testablauf: Die Zellen werden am Tag vor dem Test in Kulturmedium (DMEM, 10% FCS, 2 mM Glutamine, 10 mM HEPES) in 384-Loch-Mikrotiterplatten ausplattiert und in einem Zellinkubator

(96% Luftfeuchtigkeit, 5% v/v Kohlendioxid, 37°C) gehalten. Am Testtag wird das Kulturmedium durch eine Tyrodelösung (140 mM Natriumchlorid, 5 mM Kaliumchlorid, 1 mM Magnesiumchlorid, 2 mM Calciumchlorid, 20 mM Glucose, 20 mM HEPES), das zusätzlich den Co-Faktor Coelenterazin (50 µM) enthält, ausgetauscht und die Mikrotiterplatte anschließend für weitere 3-4 Stunden inkubiert. Die Testsubstanzen werden in verschiedenen Konzentrationen für 10 bis 20 Minuten in den Löchern der Mikrotiterplatte vorgelegt, ehe der Agonist [Arg8]-Vasopressin hinzugegeben wird und das resultierende Lichtsignal sofort im Luminometer gemessen wird. Die Berechnung der IC50-Werte erfolgt mit Hilfe des Computerprogramms GraphPad PRISM (Version 3.02).

In der folgenden Tabelle sind repräsentative IC₅₀-Werte für die erfindungsgemäßen Verbindungen an der mit dem humanen V1a- bzw. V2-Rezeptor transfizierten Zelllinie aufgerührt:

**Tabelle 1:**

| **Beispiel Nr.** | **IC₅₀ hV1a [µM]** | **IC₅₀ hV2 [µM]** |
|---|---|---|
| 1 | 0.118 | 0.012 |
| 3 | 0.106 | 0.028 |
| 4 | 0.0022 | 0.0037 |
| 14 | 0.006 | 0.0064 |
| 17 | 0.012 | 0.22 |
| 19 | 0.013 | 0.014 |
| 25 | 0.006 | 0.016 |

### B-2. Zellulärer in vitro Test zum Nachweis der Wirkung von Vasopressin V1a Rezeptor Antagonisten auf die Regulation pro-fibrotischer Gene

Die als Kardiomyozyten-Typ beschriebene Zelllinie H9C2 (American Type Culture Collection ATCC-Nr. CRL-1446), die aus Herzgewebe der Ratte isoliert wurde, exprimiert endogen den Vasopressin V1A Rezeptor AVPR1A in hoher Kopienzahl, während die AVPR2 Expression nicht nachweisbar ist. Für Zell-Tests zur Hemmung der AVPR1A Rezeptor abhängigen Regulation der Genexpression durch Rezeptor-Antagonisten wird wie folgt verfahren:

H9C2 Zellen werden in 1.0 ml Medium Opti-MEM (Invitrogen Corp. Carlsbad CA, USA, Kat. Nr. 11058-021) mit 2% FCS und 1% Penicillin/Streptomycin Lösung (Invitrogen Kat. Nr. 10378-016) mit einer Zelldichte von 100000 Zellen/Well in 12-Well Mikrotiterplatten für die Zellkultur ausgesät und in einem Zellinkubator (96% Luftfeuchtigkeit, 5% v/v Kohlendioxid, 37°C) gehalten. Nach 24 Stunden werden in je drei Wells (Triplikate) Vehikel-Lösung (Negativ-Kontrolle), Vasopressin-Lösung [Arg8]-Vasopressin Acetat (Sigma Kat. Nr. V9879) oder Testsubstanzen (gelöst in Vehikel Wasser mit 20 Vol% Ethanol) und Vasopressin-Lösung gegeben. In der Zellkultur ist die finale Vasopressin-Konzentration 0.05 µM. Die Testsubstanz Lösung wird in kleinen Volumina zur Zellkultur gegeben, so dass eine finale Konzentration von 0.1 % Ethanol im Zelltest nicht überschritten wird. Nach einer Inkubationszeit von 6 Stunden wird der Kulturüberstand abgesaugt, die adhärenten Zellen werden in 250 µl RLT-Puffer (Qiagen, Ratingen, Kat. Nr. 79216) lysiert, und die RNA wird aus diesem Lysat mit dem RNeasy Kit (Qiagen, Kat. Nr. 74104) isoliert. Anschliessend erfolgt der DNAse-Verdau (Invitrogen Kat. Nr. 18068-015), die cDNA Synthese (Promaga ImProm-II Reverse Transcription System Kat. Nr. A3800) und die RTPCR mit dem pPCR MasterMix RT-QP2X-03-075 von Eurogentec, Seraing, Belgien. Alle Prozeduren erfolgen gemäß den Arbeitsprotokollen der Test-Reagenzien Hersteller. Die Primer-Sets für die RTPCR werden anhand der mRNA Gensequenzen (NCBI Genbank Entrez Nucleotide Data Base) mit Hilfe des Programms Primer3Plus mit 6-FAM - TAMRA markierten Sonden ausgewählt. Die RTPCR zur Bestimmung der relativen mRNA Expression in den Zellen der verschiedenen Test-Ansätze erfolgt mit dem Applied Biosystems ABI Prism 7700 Sequence Detector im 96-Well oder 384-Well Mikrotiterplatten-Format gemäß der Betriebsanleitung des Gerätes. Die relative Genexpression wird durch den delta-delta Ct-Wert dargestellt [Applied Biosystems, User Bulletin No. 2 ABI Prism 7700 SDS December 11, 1997 (updated 10/2001)] mit dem Bezug auf die Expressionsstärke des Gens Ribosomales Protein L-32 (Genbank Acc. No. NMR_013226) und den Schwellen-Ct-Wert Ct = 35.

### B-3. In vivo-Test zum Nachweis der kardiovaskulären Wirkung: Blutdruckmessung an narkotisierten Ratten (Vasopressin, Challenge' Modell)

Bei männlichen Sprague-Dawley-Ratten (250-350 g Körpergewicht) werden in Ketamin/Xylazin/Pentobarbital-Injektionsnarkose Polyethylenschläuche (PE-50; Intramedic®), die mit Heparin-haltiger (500 I.E./ml) isotoner Natriumchlorid-Lösung vorgefüllt sind, in die Vena jugularis und die Vena femoralis eingeführt und anschließend eingebunden. Über einen venösen Zugang wird mit Hilfe einer Spritze Arginin-Vasopressin injiziert, über den zweiten venösen Zugang werden die Testsubstanzen appliziert. Zur Ermittlung des systolischen Blutdruckes wird ein Druckkatheter (Millar SPR-320 2F) in die A. carotis eingebunden. Der arterielle Katheter wird an einen Druckwandler angeschlossen, der seine Signale in einen mit einer geeigneten Regi-strierungssoftware ausgestatteten Messcomputer einspeist. In einem typischen Experiment werden dem Versuchstier 3-4 aufeinanderfolgende Bolusinjektionen im Abstand von 10-15 min mit einer definierten Menge Arginin-Vasopressin (30 ng/kg) in isotoner Natriumchlorid-Lösung verabreicht und, nachdem der Blutdruck wieder Ausgangswerte erreicht hat, die zu testende Substanz als Bolus mit anschließender Dauerinfusion in einem geeigneten Lösungsmittel appliziert. Hiernach wird in definierten Abständen (10-15 min) erneut die gleiche Menge Vasopressin wie zu Anfang verabreicht. Anhand der Blutdruckwerte wird ermittelt, inwieweit die Testsubstanz der blutdrucksteigemden Wirkung des Vasopressins entgegenwirkt. Kontrolltiere erhalten nur Lösungsmittel statt der Testsubstanz.

Die erfindungsgemäßen Verbindungen bewirken nach intravenöser Applikation im Vergleich zu den Lösungsmittelkontrollen eine Hemmung des durch Arginin-Vasopressin verursachten Blutdruckanstiegs.

### B-4. In vivo-Test zum Nachweis der kardiovaskulären Wirkung: Diurese-Untersuchungen an wachen Ratten in Stoffwechselkäfigen

Wistar Ratten (220-400 g Körpergwicht) werden mit freiem Zugang zu Futter (Altromin) und Trinkwasser gehalten. Während des Versuches werden die Tiere für 4 bis 8 Stunden einzeln in für Ratten dieser Gewichtsklasse geeigneten Stoffwechselkäfigen (Tecniplast Deutschland GmbH, D-82383 Hohenpeißenberg) mit freiem Zugang zu Trinkwasser gehalten. Am Versuchsbeginn wird den Tieren die zu prüfende Substanz in einem Volumen von 1 bis 3 ml/kg Körpergewicht eines geeigneten Lösemittels mittels einer Schlundsonde in den Magen verabreicht. Als Kontrolle dienende Tiere erhalten nur Lösemittel. Kontrollen und Substanztestungen werden am selben Tag parallel durchgeführt. Kontrollgruppen und Substanzdosisgruppen bestehen aus jeweils 4 bis 8 Tieren. Während des Versuchs wird der von den Tieren ausgeschiedene Urin kontinuierlich in einem Auffangbehälter am Käfigboden gesammelt. Für jedes Tier wird gesondert das Urinvolumen pro Zeiteinheit bestimmt und die Konzentration der im Urin ausgeschiedenen Natrium- bzw. Kalium-Ionen mittels flammenphotometrischer Standardmethoden gemessen. Um eine ausreichende Urinmenge zu erhalten, wird den Tieren zu Versuchsbeginn per Schlundsonde eine definierte Menge Wasser zugeführt (typischerweise 10 ml pro Kilogramm Körpergewicht). Vor Versuchsbeginn und nach Versuchsende wird das Körpergewicht der einzelnen Tiere bestimmt.

Die erfindungsgemäßen Verbindungen bewirken nach oraler Applikation im Vergleich mit Kontrolltieren eine gesteigerte Ausscheidung von Urin, die im wesentlichen auf einer gesteigerten Wasserauscheidung (Aquarese) beruht.

### B-5. In vivo-Test zum Nachweis der kardiovaskulären Wirkung: Hämodynamische Untersuchungen an narkotisierten Hunden

Männliche oder weibliche Mischlingshunde (Mongrels, Marshall BioResources, USA) mit einem Gewicht zwischen 20 und 30 kg werden mit Pentobarbital (30 mg/kg iv, Narcoren®, Merial, Deutschland) werden für die operativen Eingriffe und die hämodynamische und funktionellen Untersuchungstermine jeweils annarkotisiert. Alcuroniumchlorid (Alloferin®, ICN Pharmaceuticals, Deutschland, 3 mg/Tier iv) dient dabei zusätzlich als Muskelrelaxans. Die Hunde werden intubiert und mit einem Sauerstoff-Raumluft-Gemisch (40/60%) beatmet (etwa 5-6L/min). Die Beatmung erfolgt mit einem Beatmungsgerät der Firma Draeger (Sulla 808) und wird überwacht mit einem Kohlendioxid-Analysator (Engström).

Die Narkose wird aufrechterhalten durch eine ständige Infusion von Pentobarbital (50µg/kg/min); als Analgetikum dient Fentanyl (10 µg/kg/h). Eine Alternative zu Pentobarbital besteht in der Verwendung von Isofluran (1-2 Vol%).

Die Hunde werden in vorbereitenden Eingriffen mit einem Herzschrittmacher ausgestattet.
- Zum Zeitpunkt 21 Tage vor der ersten Medikamenten-Testung =(Versuchsbeginn) wird eine Herzschrittmacher der Fa. Biotronik (Logos®) in eine subcutane Hauttasche implantiert und über eine Schrittmacher-Elektrode, die durch die Vena jugularis externa unter Durchleuchtung bis in den rechten Ventrikel vorgeschoben wird, mit dem Herzen in Kontakt gebracht.
- Zum gleichen Zeitpunkt der Schrittmacher-Implantation erfolgt durch retrogrades Vorschieben eine 7F-Biopsie-Zange (Fa. Cordis) über eine Schleuse (Avanti+®; Fa. Cordis) in der Arteria femoralis und nach atraumatischer Passager der Aortenklappe eine definierte Läsion der Mitralklappe unter echokardiographischer und Durchleuchtungskontrolle. Im Anschluss werden alle Zugänge entfernt und der Hund wacht spontan aus der Narkose auf.
- Nach 7 weiteren Tagen (=14 Tage vor der ersten Medikamenten-Testung) wird der o.g. Schrittmacher aktiviert und das Herz mit einer Frequenz von 220 Schlägen pro Minute stimuliert.

Die eigentlichen Experimente zur Medikamenten-Testung erfolgen 14 und 28 Tagen nach Beginn der Schrittmacher-Stimulation nach folgender Instrumentierung:
- Blasenkatheter zur Blasenentlastung bzw. zur Messung des Urinflusses
- EKG-Ableitungen an den Extremitäten (zur EKG Messung)
- Einführen eines NaCl-gefüllten Fluidmedic-PE-300-Schlauches in die A. femoralis. Dieser ist verbunden mit einem Drucksensor (Braun Melsungen, Melsungen, Deutschland) zur Messung des systemischen Blutdrucks
- Einführen eines Millar Tip Katheters (Typ 350 PC, Millar Instruments, Houston, USA) über den linken Vorhof oder über eine in der A. carotis eingebundene Schleuse zur Messung der Herz-Hämodynamik
- Einführen eines Swan-Ganz-Katheters (CCOmbo 7.5F, Edwards, Irvine, USA) über die V. jugularis in die A. pulmonalis zur Messung von Herzzeitvolumen, Sauerstoffsättigung, Pulmonalartiendrücken und zentralvenösem Druck.
- Platzieren einer Braunüle in den Venae cephalicae zur Infusion von Pentobarbital, der Flüssigkeitssubstitution und zur Blutentnahme (Bestimmung der Substanz-Plasmaspiegeln oder sonstiger klinischer Blutwerte)
- Platzieren einer Braunüle in der Venae saphenae zur Infusion von Fentanyl und zur Substanzapplikation
- Infusion von Vasopressin (Fa. Sigma) in aufsteigender Dosierung bis zu einer Dosis von 4 mU/kg/min. Unter dieser Dosierung erfolgt dann die Testung pharmakologischer Substanzen.

Die Primärsignale werden eventuell verstärkt (Gould Verstärker, Gould Instrument Systems, Valley View, USA) bzw. Edwards-Vigilance- Monitor (Edwards, Irvine, USA) und anschließend zur Auswertung in das Ponemah-System (DataSciences Inc, Minneapolis, USA) eingespeist. Die Signale werden kontinuierlich über den gesamten Versuchszeitraum aufgezeichnet, von dieser Software digital weiterarbeitet und über 30 s gemittelt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 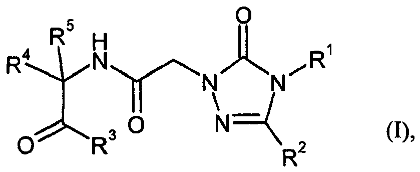 in welcher
R¹ für (C₁-C₆)-Alkyl, (C₂-C₆)-Alkenyl, (C₂-C₆)-Alkinyl oder (C₃-C₇)-Cycloalkyl steht,
wobei (C₁-C₆)-Alkyl, (C₂-C₆)-Alkenyl und (C₂-C₆)-Alkinyl mit 1 bis 3 Substituenten unabhängig voneinander ausgewählt aus der Gruppe Halogen, Cyano, Oxo, Hydroxy, Trifluormethyl, (C₃-C₇)-Cycloalkyl, (C₁-C₆)-Alkoxy, Trifluormethoxy und Phenyl substituiert sein können,
worin (C₃-C₇)-Cycloalkyl mit 1 oder 2 Substituenten unabhängig voneinander ausgewählt aus der Gruppe (C₁-C₄)-Alkyl, Oxo, Hydroxy, (C₁-C₄)-Alkoxy und Amino substituiert sein kann,
und
worin (C₁-C₆)-Alkoxy mit 1 oder 2 Substituenten unabhängig voneinander ausgewählt aus der Gruppe Amino, Hydroxy, (C₁-C₄)-Alkoxy, Hydroxycarbonyl und (C₁-C₄)-Alkoxycarbonyl substituiert sein kann
und
worin Phenyl mit 1 bis 3 Substituenten unabhängig voneinander ausgewählt aus der Gruppe Halogen, Cyano, Nitro, (C₁-C₄)-Alkyl, Trifluormethyl, Hydroxy, Hydroxymethyl, (C₁-C₄)-Alkoxy, Trifluormethoxy, (C₁-C₄)-Alkoxymethyl, Hydroxycarbonyl und (C₁-C₄)-Alkoxycarbonyl substituiert sein kann,
und
wobei (C₃-C₇)-Cycloalkyl mit 1 oder 2 Substituenten unabhängig voneinander ausgewählt aus der Gruppe Fluor, (C₁-C₄)-Alkyl, (C₁-C₄)-Alkoxy, Hydroxy, Amino und Oxo substituiert sein kann,
R² für Phenyl, Naphthyl, Thienyl, Benzothienyl, Furyl oder Benzofuryl steht,
wobei Phenyl, Naphthyl, Thienyl, Benzothienyl, Furyl und Benzofuryl mit 1 bis 3 Substituenten unabhängig voneinander ausgewählt aus der Gruppe Halogen, Cyano, Nitro, (C₁-C₄)-Alkyl, Trifluormethyl, Hydroxy, (C₁-C₄)-Alkoxy, Trifluormethoxy und Phenyl substituiert sein können,
worin Phenyl mit 1 oder 2 Substituenten unabhängig von einander ausgewählt aus der Gruppe Halogen, Cyano, Nitro, (C₁-C₄)-Alkyl, Trifluormethyl, Hydroxy, (C₁-C₄)-Alkoxy, Trifluormethoxy, Hydroxy-(C₁-C₄)-alkyl und (C₁-C₄)-Alkylthio substituiert sein kann,
R³ für Hydroxy oder -NR⁶R⁷ steht,
wobei
R⁶ für Wasserstoff oder (C₁-C₄)-Alkyl steht,
R⁷ für Wasserstoff, (C₁-C₄)-Alkyl oder (C₃-C₇)-Cycloalkyl steht,
R⁴ für Phenyl steht,
wobei Phenyl mit 1 bis 3 Substituenten unabhängig voneinander ausgewählt aus der Gruppe Halogen, Cyano, Nitro, (C₁-C₄)-Alkyl, Difluormethyl, Trifluormethyl, Hydroxy, (C₁-C₄)-Alkoxy, Difluormethoxy, Trifluormethoxy und Phenyl substituiert sein kann,
worin Phenyl mit 1 oder 2 Substituenten unabhängig von einander ausgewählt aus der Gruppe Halogen, Cyano, Nitro, (C₁-C₄)-Alkyl, Trifluormethyl, Hydroxy, (C₁-C₄)-Alkoxy, Trifluormethoxy, Hydroxy-(C₁-C₄)-alkyl und (C₁-C₄)-Alkylthio substituiert sein kann,
R⁵ für Trifluormethyl, (C₁-C₄)-Alkyl oder (C₃-C₇)-Cycloalkyl steht,
sowie ihre Salze, Solvate und Solvate der Salze.

2. Verbindung der Formel (I) nach Anspruch 1, in welcher
R¹ für (C₁-C₆)-Alkyl, (C₂-C₆)-Alkenyl oder (C₃-C₆)-Cycloalkyl steht,
wobei (C₁-C₆)-Alkyl und (C₂-C₆)-Alkenyl mit 1 bis 3 Substituenten unabhängig voneinander ausgewählt aus der Gruppe Fluor, Chlor, Cyano, Oxo, Hydroxy, Trifluormethyl, Cyclopropyl, Cyclobutyl, Methoxy, Ethoxy, Trifluormethoxy und Phenyl substituiert sein können,
worin Phenyl mit 1 bis 3 Substituenten unabhängig voneinander ausgewählt aus der Gruppe Fluor, Chlor, Cyano, Methyl, Ethyl, Trifluormethyl, Hydroxy, Hydroxymethyl, Methoxy, Ethoxy, Trifluormethoxy, Methoxymethyl, Ethoxymethyl, Hydroxycarbonyl, Methoxycarbonyl und Ethoxycarbonyl substituiert sein kann,
und
wobei (C₃-C₆)-Cycloalkyl mit 1 oder 2 Substituenten unabhängig voneinander ausgewählt aus der Gruppe Fluor, Methyl, Ethyl, Methoxy, Ethoxy, Hydroxy, Amino und Oxo substituiert sein kann,
R² für Phenyl oder Thienyl steht,
wobei Phenyl und Thienyl mit 1 oder 2 Substituenten unabhängig voneinander ausgewählt aus der Gruppe Fluor, Chlor, Cyano, Methyl, Ethyl, Trifluormethyl, Hydroxy, Methoxy, Ethoxy und Trifluormethoxy substituiert sein können,
R³ für Hydroxy oder -NR⁶R⁷ steht,
wobei
R⁶ für Wasserstoff oder (C₁-C₄)-Alkyl steht,
R⁷ für Wasserstoff, (C₁-C₄)-Alkyl oder (C₃-C₅)-Cycloalkyl steht,
R⁴ für Phenyl steht,
wobei Phenyl mit 1 bis 3 Substituenten unabhängig voneinander ausgewählt aus der Gruppe Fluor, Chlor, Cyano, Methyl, Ethyl, Difluormethyl, Trifluormethyl, Methoxy, Ethoxy, Difluormethoxy und Trifluormethoxy substituiert sein kann,
R⁵ für Trifluormethyl, Methyl, Ethyl, iso-Propyl oder Cyclopropyl steht,
sowie ihre Salze, Solvate und Solvate der Salze.

3. Verbindung der Formel (I) nach Anspruch 1 oder 2, in welcher
R¹ für (C₁-C₆)-Alkyl, (C₂-C₆)-Alkenyl oder Cyclopropyl steht,
wobei (C₁-C₆)-Alkyl und (C₂-C₆)-Alkenyl mit 1 oder 2 Substituenten unabhängig voneinander ausgewählt aus der Gruppe Fluor, Oxo, Hydroxy, Trifluormethyl, Cyclopropyl und Phenyl substituiert sein können,
worin Phenyl mit 1 oder 2 Substituenten unabhängig voneinander ausgewählt aus der Gruppe Fluor, Chlor, Methyl und Methoxy substituiert sein kann,
R² für Phenyl steht,
wobei Phenyl mit 1 oder 2 Substituenten unabhängig voneinander ausgewählt aus der Gruppe Fluor, Chlor, Methyl, Methoxy und Trifluormethoxy substituiert sein kann,
R³ für Hydroxy oder -NR⁶R⁷ steht,
wobei
R⁶ für Wasserstoff oder Methyl steht,
R⁷ für Wasserstoff, Methyl oder Cyclopropyl steht,
R⁴ für Phenyl steht,
wobei Phenyl mit 1 oder 2 Substituenten unabhängig voneinander ausgewählt aus der Gruppe Fluor, Chlor, Methyl, Trifluormethyl, Methoxy und Trifluormethoxy substituiert sein kann,
R⁵ für Methyl oder Ethyl steht,
sowie ihre Salze, Solvate und Solvate der Salze.

4. Verfahren zur Herstellung von Verbindungen der Formel (I), wie in den Ansprüchen 1 bis 3 definiert, **dadurch gekennzeichnet, dass** man
[A] eine Verbindung der Formel (II) 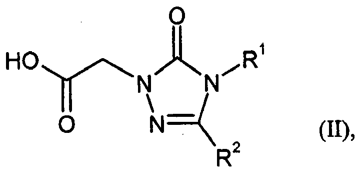 in welcher R¹ und R² jeweils die in den Ansprüchen 1 bis 3 angegebenen Bedeutungen haben,
in einem inerten Lösungsmittel unter Aktivierung der Carbonsäure-Funktion mit einer Verbindung der Formel (III) 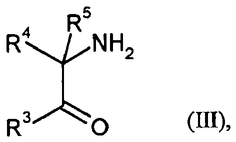 in welcher R³, R⁴ und R⁵ jeweils die in den Ansprüchen 1 bis 3 angegebenen Bedeutungen haben,
kuppelt
oder
[B] eine Verbindung der Formel (IV) 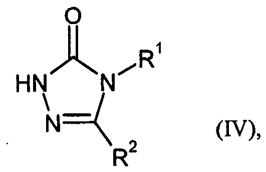 in welcher R¹ und R² jeweils die in den Ansprüchen 1 bis 3 angegebenen Bedeutungen haben,
in einem inerten Lösungsmittel in Gegenwart einer Base mit einer Verbindung der Formel (V) 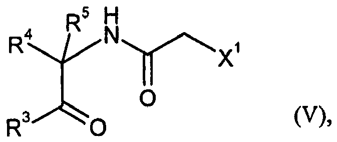 in welcher R³, R⁴ und R⁵ jeweils die in den Ansprüchen 1 bis 3 angegebenen Bedeutungen haben
und
X¹ für eine Abgangsgruppe, wie beispielsweise Halogen, Mesylat oder Tosylat, steht,
umsetzt,
oder
[C] eine Verbindung der Formel (I-A) 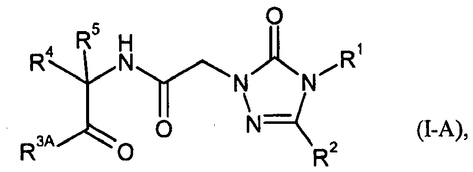 in welcher R¹, R², R⁴ und R⁵ jeweils die in den Ansprüchen 1 bis 3 angegebenen Bedeutungen haben
und
R^{3A} für Hydroxy steht,
in einem inerten Lösungsmittel unter Aktivierung der Carbonsäure-Funktion mit einem Amin der Formel (VI) 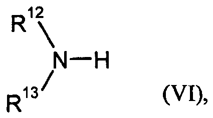 in welcher R¹² und R¹³ jeweils die in den Ansprüchen 1 bis 3 angegebenen Bedeutungen haben
umsetzt
und die resultierenden Verbindungen der Formel (I) gegebenenfalls mit den entsprechenden (i) Lösungsmitteln und/oder (ii) Basen oder Säuren in ihre Solvate, Salze und/oder Solvate der Salze überführt.

5. Verbindung der Formel (I), wie in einem der Ansprüche 1 bis 3 definiert, zur Behandlung und/oder Prophylaxe von Krankheiten.

6. Verbindung der Formel (I), wie in einem der Ansprüche 1 bis 3 definiert, zur Verwendung in einem Verfahren zur Behandlung und/oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

7. Verwendung einer Verbindung der Formel (I), wie in einem der Ansprüche 1 bis 3 definiert, zur Herstellung eines Arzneimittels zur Behandlung und/oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

8. Arzneimittel enthaltend eine Verbindung der Formel (I), wie in einem der Ansprüche 1 bis 3 definiert, in Kombination mit einem inerten, nicht-toxischen, pharmazeutisch geeigneten Hilfsstoff.

9. Arzneimittel enthaltend eine Verbindung der Formel (I), wie in einem der Ansprüche 1 bis 3 definiert, in Kombination mit einem oder mehreren weiteren Wirkstoffen ausgewählt aus der Gruppe bestehend aus Diuretika, Angiotensin AII-Antagonisten, ACE-Hemmer, beta-Rezeptoren-Blocker, Mineralocorticoid-Rezeptor-Antagonisten, organische Nitrate, NO-Donatoren und positiv-inotrop wirksame Substanzen.

10. Arzneimittel nach Anspruch 8 oder 9 zur Behandlung und/oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

11. Arzneimittel nach Anspruch 8 oder 9 zur Behandlung und/oder Prophylaxe von akutem und chronischem Nierenversagen sowie von chronischer Niereninsuffizienz.

## Claims

1. Compound of the formula (I) 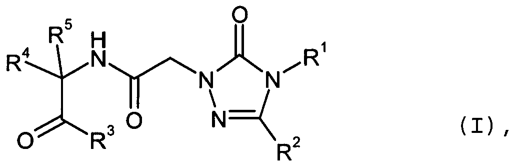 in which
R¹ represents (C₁-C₆)-alkyl, (C₂-C₆) -alkenyl, (C₂-C₆)-alkynyl or (C₃-C₇)-cycloalkyl,
where (C₁-C₆)-alkyl, (C₂-C₆) -alkenyl and (C₂-C₆)-alkynyl may be substituted by 1 to 3 substituents independently of one another selected from the group consisting of halogen, cyano, oxo, hydroxyl, trifluoromethyl, (C₃-C₇) -cycloalkyl, (C₁-C₆)-alkoxy, trifluoromethoxy and phenyl,
where (C₃-C₇)-cycloalkyl may be substituted by 1 or 2 substituents independently of one another selected from the group consisting of (C₁-C₄)-alkyl, oxo, hydroxyl, (C₁-C₄)-alkoxy and amino
and
where (C₁-C₆)-alkoxy may be substituted by 1 or 2 substituents independently of one another selected from the group consisting of amino, hydroxyl, (C₁-C₄)-alkoxy, hydroxycarbonyl and (C₁-C₄)-alkoxycarbonyl
and
where phenyl may be substituted by 1 to 3 substituents independently of one another selected from the group consisting of halogen, cyano, nitro, (C₁-C₄)-alkyl, trifluoromethyl, hydroxyl, hydroxymethyl, (C₁-C₄)-alkoxy, trifluoromethoxy, (C₁-C₄)-alkoxymethyl, hydroxycarbonyl and (C₁-C₄)-alkoxy-carbonyl,
and
where (C₃-C₇)-cycloalkyl may be substituted by 1 or 2, substituents independently of one another selected from the group consisting of fluorine, (C₁-C₄)-alkyl, (C₁-C₄)-alkoxy, hydroxyl, amino and oxo,
R² represents phenyl, naphthyl, thienyl, benzothienyl, furyl or benzofuryl,
where phenyl, naphthyl, thienyl, benzothienyl, furyl and benzofuryl may be substituted by 1 to 3 substituents independently of one another selected from the group consisting of halogen, cyano, nitro, (C₁-C₄)-alkyl, trifluoromethyl, hydroxyl, (C₁-C₄)-alkoxy, trifluoromethoxy and phenyl,
where phenyl may be substituted by 1 or 2 substituents independently of one another selected from the group consisting of halogen, cyano, nitro, (C₁-C₄)-alkyl, trifluoromethyl, hydroxyl, (C₁-C₄)-alkoxy, trifluoromethoxy, hydroxy- (C₁-C₄) -alkyl and (C₁-C₄)-alkylthio,
R³ represents hydroxyl or -NR⁶R⁷,
where
R⁶ represents hydrogen or (C₁-C₄)-alkyl,
R⁷ represents hydrogen, (C₁-C₄)-alkyl or (C₃-C₇) -cycloalkyl,
R⁴ represents phenyl,
where phenyl may be substituted by 1 to 3 substituents independently of one another selected from the group consisting of halogen, cyano, nitro, (C₁-C₄)-alkyl, difluoromethyl, trifluoromethyl, hydroxyl, (C₁-C₄)-alkoxy, difluoromethoxy, trifluoromethoxy and phenyl,
where phenyl may be substituted by 1 or 2 substituents independently of one another selected from the group consisting of halogen, cyano, nitro, (C₁-C₄)-alkyl, trifluoromethyl, hydroxyl, (C₁-C₄)-alkoxy, trifluoromethoxy, hydroxy- (C₁-C₄) -alkyl and (C₁-C₄)-alkylthio,
R⁵ represents trifluoromethyl, (C₁-C₄)-alkyl or (C₃-C₇)-cycloalkyl,
and also its salts, solvates and solvates of the salts.

2. Compound of the formula (I) according to Claim 1 in which
R¹ represents (C₁-C₆)-alkyl, (C₂-C₆)-alkenyl or (C₃-C₆)-cycloalkyl,
where (C₁-C₆) -alkyl and (C₂-C₆) -alkenyl may be substituted by 1 to 3 substituents independently of one another selected from the group consisting of fluorine, chlorine, cyano, oxo, hydroxyl, trifluoromethyl, cyclopropyl, cyclobutyl, methoxy, ethoxy, trifluoromethoxy and phenyl,
where phenyl may be substituted by 1 to 3 substituents independently of one another selected from the group consisting of fluorine, chlorine, cyano, methyl, ethyl, trifluoromethyl, hydroxyl, hydroxymethyl, methoxy, ethoxy, trifluoromethoxy, methoxymethyl, ethoxymethyl, hydroxycarbonyl, methoxycarbonyl and ethoxycarbonyl,
and
where (C₃-C₆)-cycloalkyl may be substituted by 1 or 2 substituents independently of one another selected from the group consisting of fluorine, methyl, ethyl, methoxy, ethoxy, hydroxyl, amino and oxo,
R² represents phenyl or thienyl,
where phenyl and thienyl may be substituted by 1 or 2 substituents independently of one another selected from the group consisting of fluorine, chlorine, cyano, methyl, ethyl, trifluoromethyl, hydroxyl, methoxy, ethoxy and trifluormethoxy,
R³ represents hydroxyl or -NR⁶R⁷,
where
R⁶ represents hydrogen or (C₁-C₄)-alkyl,
R⁷ represents hydrogen, (C₁-C₄)-alkyl or (C₃-C₅)-cycloalkyl,
R⁴ represents phenyl,
where phenyl may be substituted by 1 to 3 substituents independently of one another selected from the group consisting of fluorine, chlorine, cyano, methyl, ethyl, difluoromethyl, trifluoromethyl, methoxy, ethoxy, difluoromethoxy and trifluoromethoxy,
R⁵ represents trifluoromethyl, methyl, ethyl, isopropyl or cyclopropyl,
and also its salts, solvates and solvates of the salts.

3. Compound of the formula (I) according to Claim 1 or 2 in which
R¹ represents (C₁-C₆)-alkyl, (C₂-C₆)-alkenyl or cyclopropyl,
where (C₁-C₆)-alkyl and (C₂-C₆)-alkenyl may be substituted by 1 or 2 substituents independently of one another selected from the group consisting of fluorine, oxo, hydroxyl, trifluoromethyl, cyclopropyl and phenyl,
where phenyl may be substituted by 1 or 2 substituents independently of one another selected from the group consisting of fluorine, chlorine, methyl and methoxy,
R² represents phenyl,
where phenyl may be substituted by 1 or 2 substituents independently of one another selected from the group consisting of fluorine, chlorine, methyl, methoxy and trifluoromethoxy,
R³ represents hydroxyl or -NR⁶R⁷,
where
R⁶ represents hydrogen or methyl,
R⁷ represents hydrogen, methyl or cyclopropyl,
R⁴ represents phenyl,
where phenyl may be substituted by 1 or 2 substituents independently of one another selected from the group consisting of fluorine, chlorine, methyl, trifluoromethyl, methoxy and trifluoromethoxy,
R⁵ represents methyl or ethyl,
and also its salts, solvates and solvates of the salts.

4. Process for preparing compounds of the formula (I) as defined in any of Claims 1 to 3 **characterized in that**
[A] a compound of the formula (II) 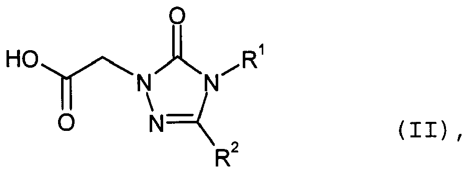 in which R¹ and R² each have the meanings given in any of Claims 1 to 3
is coupled in an inert solvent with activation of the carboxylic acid function with a compound of the formula (III) 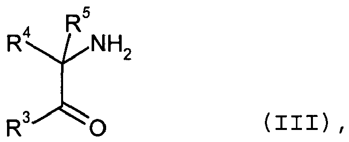 in which R³, R⁴ and R⁵ each have the meanings given in any of Claims 1 to 3,
or
[B] a compound of the formula (IV) 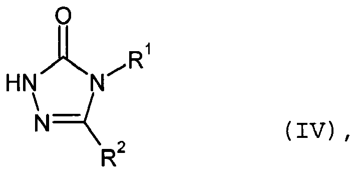 in which R¹ and R² each have the meanings given in any of Claims 1 to 3,
is reacted in an inert solvent in the presence of a base with a compound of the formula (V) 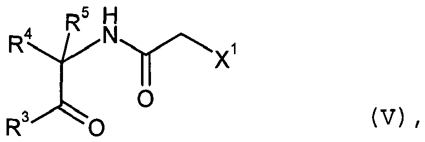 in which R³, R⁴ and R⁵ each have the meanings given in any of Claims 1 to 3
and
X¹ represents a leaving group such as, for example, halogen, mesylate or tosylate,
or
[C] a compound of the formula (I-A) 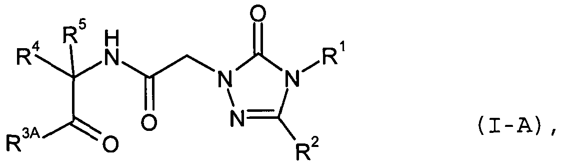 in which R¹, R², R⁴ and R⁵ each have the meanings given in any of Claims 1 to 3
and
R^{3A} represents hydroxyl,
is reacted in an inert solvent with activation of the carboxylic acid function with an amine of the formula (VI) 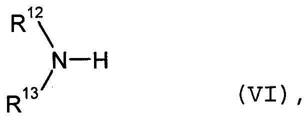 in which R¹² and R¹³ each have the meanings given in any of Claims 1 to 3
and the resulting compounds of the formula (I) are optionally converted with the appropriate (i) solvents and/or (ii) bases or acids into their solvates, salts and/or solvates of the salts.

5. Compound of the formula (I) as defined in any of Claims 1 to 3 for the treatment and/or prophylaxis of diseases.

6. Compound of the formula (I) as defined in any of Claims 1 to 3 for use in a method for the treatment and/or prophylaxis of acute and chronic heart failure, hypervolemic and euvolemic hyponatremia, cirrhosis of the liver, ascites, edema and the syndrome of inadequate ADH secretion (SIADH).

7. Use of a compound of the formula (I) as defined in any of Claims 1 to 3 for preparing a medicament for the treatment and/or prophylaxis of acute and chronic heart failure, hypervolemic and euvolemic hyponatremia, cirrhosis of the liver, ascites, edema and the syndrome of inadequate ADH secretion (SIADH).

8. Medicament comprising a compound of the formula (I) as defined in any of Claims 1 to 3 in combination with an inert non-toxic pharmaceutically suitable auxiliary.

9. Medicament comprising a compound of the formula (I) as defined in any of Claims 1 to 3 in combination with one or more further active substances selected from the group consisting of diuretics, angiotensin AII antagonists, ACE inhibitors, beta-receptor blockers, mineralocorticoid receptor antagonists, organic nitrates, NO donators and positive-inotropic active substances.

10. Medicament according to Claim 8 or 9 for the treatment and/or prophylaxis of acute and chronic heart failure, hypervolemic and euvolemic hyponatremia, cirrhosis of the liver, ascites, edema and the syndrome of inadequate ADH secretion (SIADH).

11. Medicament according to Claim 8 or 9 for the treatment and/or prophylaxis of acute and chronic kidney failure and also of chronic renal insufficiency.

## Revendications

1. Composé de formule (I) 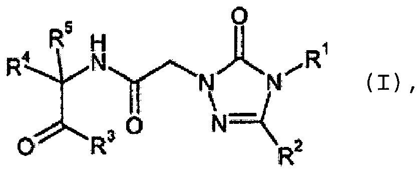 dans laquelle
R¹ représente un groupe alkyle en C₁-C₆, alcényle en C₂-C₆, alcynyle en C₂-C₆ ou cycloalkyle en C₃-C₇,
les groupes alkyle en C₁-C₆, alcényle en C₂-C₆ et alcynyle en C₂-C₆ pouvant être substitués par 1 à 3 substituants choisis chacun indépendamment dans l'ensemble constitué par halogéno, cyano, oxo, hydroxy, trifluorométhyle, cycloalkyle en C₃-C₇, alcoxy en C₁-C₆, trifluorométhoxy et phényle,
où cycloalkyle en C₃-C₇ peut être substitué par 1 ou 2 substituants choisis indépendamment l'un de l'autre dans l'ensemble constitué par alkyle en C₁-C₄, oxo, hydroxy, alcoxy en C₁-C₄ et amino,
et
où alcoxy en C₁-C₆ peut être substitué par 1 ou 2 substituants choisis indépendamment l'un de l'autre dans l'ensemble constitué par amino, hydroxy, alcoxy en C₁-C₄, hydroxycarbonyle et alcoxy(C₁-C₄)carbonyle
et
où phényle peut être substitué par 1 à 3 substituants choisis chacun indépendamment dans l'ensemble constitué par halogéno, cyano, nitro, alkyle en C₁-C₄, trifluorométhyle, hydroxy, hydroxyméthyle, alcoxy en C₁-C₄, trifluorométhoxy, alcoxy(C₁-C₄)méthyle, hydroxycarbonyle et alcoxy(C₁-C₄)carbonyle,
et
cycloalkyle en C₃-C₇ pouvant être substitué par 1 ou 2 substituants choisis indépendamment l'un de l'autre dans l'ensemble constitué par fluoro, alkyle en C₁-C₄, alcoxy en C₁-C₄, hydroxy, amino et oxo,
R² représente un groupe phényle, naphtyle, thiényle, benzothiényle, furyle ou benzofuryle,
les groupes phényle, naphtyle, thiényle, benzothiényle, furyle et benzofuryle pouvant être substitués par 1 à 3 substituants choisis chacun indépendamment dans l'ensemble constitué par halogéno, cyano, nitro, alkyle en C₁-C₄, trifluorométhyle, hydroxy, alcoxy en C₁-C₄, trifluorométhoxy et phényle,
où phényle peut être substitué par 1 ou 2 substituants choisis indépendamment l'un de l'autre dans l'ensemble constitué par halogéno, cyano, nitro, alkyle en C₁-C₄, trifluorométhyle, hydroxy, alcoxy en C₁-C₄, trifluorométhoxy, hydroxy-alkyle(C₁-C₄) et alkyl(C₁-C₄)-thio,
R³ représente un groupe hydroxy ou -NR⁶R⁷,
R⁶ représentant un atome d'hydrogène ou un groupe alkyle en C₁-C₄,
R⁷ représentant un atome d'hydrogène, un groupe alkyle en C₁-C₄ ou cycloalkyle en C₃-C₇,
R⁴ représente un groupe phényle,
le groupe phényle pouvant être substitué par 1 à 3 substituants choisis chacun indépendamment dans l'ensemble constitué par halogéno, cyano, nitro, alkyle en C₁-C₄, difluorométhyle, trifluorométhyle, hydroxy, alcoxy en C₁-C₄, difluorométhoxy, trifluorométhoxy et phényle,
où phényle peut être substitué par 1 ou 2 substituants choisis indépendamment l'un de l'autre dans l'ensemble constitué par halogéno, cyano, nitro, alkyle en C₁-C₄, trifluorométhyle, hydroxy, alcoxy en C₁-C₄, trifluorométhoxy, hydroxy-alkyle(C₁-C₄) et alkyl(C₁-C₄)-thio,
R⁵ représente un groupe trifluorométhyle, alkyle en C₁-C₄ ou cycloalkyle en C₃-C₇,
ainsi que ses sels, produits de solvatation et produits de solvatation des sels.

2. Composé de formule (I) selon la revendication 1, dans lequel
R¹ représente un groupe alkyle en C₁-C₆, alcényle en C₂-C₆ ou cycloalkyle en C₃-C₆,
les groupes alkyle en C₁-C₆ et alcényle en C₂-C₆ pouvant être substitués par 1 à 3 substituants choisis chacun indépendamment dans l'ensemble constitué par fluoro, chloro, cyano, oxo, hydroxy, trifluorométhyle, cyclopropyle, cyclobutyle, méthoxy, éthoxy, trifluorométhoxy et phényle,
où phényle peut être substitué par 1 à 3 substituants choisis chacun indépendamment dans l'ensemble constitué par fluoro, chloro, cyano, méthyle, éthyle, trifluorométhyle, hydroxy, hydroxyméthyle, méthoxy, éthoxy, trifluorométhoxy, méthoxyméthyle, éthoxy-méthyle, hydroxycarbonyle, méthoxycarbonyle et éthoxycarbonyle,
et
cycloalkyle en C₃-C₆ pouvant être substitué par 1 ou 2 substituants choisis indépendamment l'un de l'autre dans l'ensemble constitué par fluoro, méthyle, éthyle, méthoxy, éthoxy, hydroxy, amino et oxo,
R² représente un groupe phényle ou thiényle,
les groupes phényle et thiényle pouvant être substitués par 1 ou 2 substituants choisis indépendamment l'un de l'autre dans l'ensemble constitué par fluoro, chloro, cyano, méthyle, éthyle, trifluorométhyle, hydroxy, méthoxy, éthoxy et trifluorométhoxy,
R³ représente un groupe hydroxy ou -NR⁶R⁷,
R⁶ représentant un atome d'hydrogène ou un groupe alkyle en C₁-C₄,
R⁷ représentant un atome d'hydrogène, un groupe alkyle en C₁-C₄ ou cycloalkyle en C₃-C₅,
R⁴ représente un groupe phényle,
le groupe phényle pouvant être substitué par 1 à 3 substituants choisis chacun indépendamment dans l'ensemble constitué par fluoro, chloro, cyano, méthyle, éthyle, difluorométhyle, trifluoro-méthyle, méthoxy, éthoxy, difluorométhoxy et trifluorométhoxy,
R⁵ représente un groupe trifluorométhyle, méthyle, éthyle, isopropyle ou cyclopropyle,
ainsi que ses sels, produits de solvatation et produits de solvatation des sels.

3. Composé de formule (I) selon la revendication 1 ou 2, dans lequel
R¹ représente un groupe alkyle en C₁-C₆, alcényle en C₂-C₆ ou cyclopropyle,
les groupes alkyle en C₁-C₆ et alcényle en C₂-C₆ pouvant être substitués par 1 ou 2 substituants choisis indépendamment l'un de l'autre dans l'ensemble constitué par fluoro, oxo, hydroxy, trifluorométhyle, cyclopropyle et phényle,
où phényle peut être substitué par 1 ou 2 substituants choisis indépendamment l'un de l'autre dans l'ensemble constitué par fluoro, chloro, méthyle et méthoxy,
R² représente un groupe phényle,
le groupe phényle pouvant être substitué par 1 ou 2 substituants choisis indépendamment l'un de l'autre dans l'ensemble constitué par fluoro, chloro, méthyle, méthoxy et trifluorométhoxy,
R³ représente un groupe hydroxy ou -NR⁶R⁷,
R⁶ représentant un atome d'hydrogène ou le groupe méthyle,
R⁷ représentant un atome d'hydrogène, le groupe méthyle ou cyclopropyle,
R⁴ représente un groupe phényle,
le groupe phényle pouvant être substitué par 1 ou 2 substituants choisis indépendamment l'un de l'autre dans l'ensemble constitué par fluoro, chloro, méthyle, trifluorométhyle, méthoxy et trifluorométhoxy,
R⁵ représente le groupe méthyle ou éthyle,
ainsi que ses sels, produits de solvatation et produits de solvatation des sels.

4. Procédé pour la préparation des composés de formule (I), tels que définis dans les revendications 1 à 3, **caractérisé en ce que**
[A] on combine un composé de formule (II) 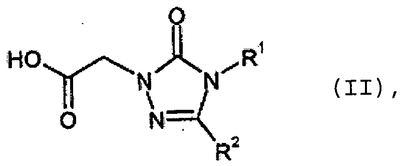 dans laquelle R¹ et R² ont chacun les significations indiquées dans les revendications 1 à 3,
dans un solvant inerte, avec activation de la fonction carboxy,
avec un composé de formule (III) 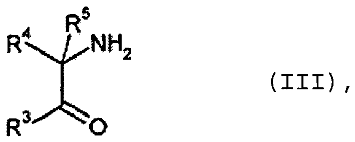 dans laquelle R³, R⁴ et R⁵ ont chacun les significations indiquées dans les revendications 1 à 3,
ou
[B] on fait réagir un composé de formule (IV) 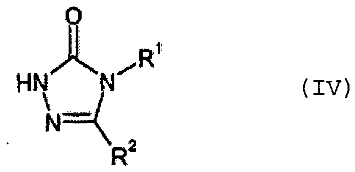 dans laquelle R¹ et R² ont chacun les significations indiquées dans les revendications 1 à 3,
dans un solvant inerte, en présence d'une base, avec un composé de formule (V) 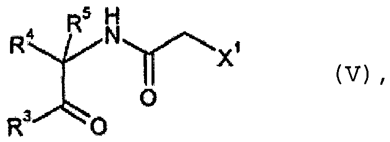 dans laquelle R³, R⁴ et R⁵ ont chacun les significations indiquées dans les revendications 1 à 3
et
X¹ représente un groupe partant, comme par exemple un atome d'halogène, le groupe mésylate ou tosylate,
ou
[C] on fait réagir un composé de formule (I-A) 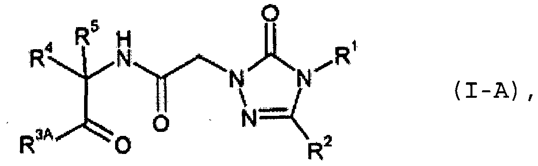 dans laquelle R¹, R², R⁴ et R⁵ ont chacun les significations indiquées dans les revendications 1 à 3
et
R^{3A} représente le groupe hydroxy,
dans un solvant inerte, avec activation de la fonction carboxy, avec une amine de formule (VI) 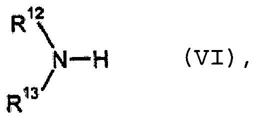 dans laquelle R¹² et R¹³ ont chacun les significations indiquées dans les revendications 1 à 3
et éventuellement on convertit les composés de formule (I) résultants, à l'aide (i) des solvants correspondants et/ou (ii) des bases correspondantes ou acides correspondants, en leurs produits de solvatation, sels et/ou produits de solvatation des sels.

5. Composé de formule (I), tel que défini dans l'une quelconque des revendications 1 à 3, destiné au traitement et/ou à la prophylaxie de maladies.

6. Composé de formule (I), tel que défini dans l'une quelconque des revendications 1 à 3, destiné à l'utilisation dans un procédé pour le traitement et/ou la prophylaxie de l'insuffisance cardiaque aiguë ou chronique, de l'hyponatrémie hypervolumique ou euvolumique, de la cirrhose du foie, d'ascites, d'oedèmes et du syndrome de la sécrétion inadéquate d'ADH (SIADH).

7. Utilisation d'un composé de formule (I), tel que défini dans l'une quelconque des revendications 1 à 3, pour la fabrication d'un médicament destiné au traitement et/ou à la prophylaxie de l'insuffisance cardiaque aiguë ou chronique, de l'hyponatrémie hypervolumique ou euvolumique, de la cirrhose du foie, d'ascites, d'oedèmes et du syndrome de la sécrétion inadéquate d'ADH (SIADH).

8. Médicament contenant un composé de formule (I), tel que défini dans l'une quelconque des revendications 1 à 3, en association avec un adjuvant inerte, non toxique, pharmaceutiquement approprié.

9. Médicament contenant un composé de formule (I), tel que défini dans l'une quelconque des revendications 1 à 3, en association avec une ou plusieurs autres substances actives choisies dans le groupe constitué par les diurétiques, antagonistes d'angiotensine AII, inhibiteurs d'ACE, inhibiteurs des récepteurs bêta, antagonistes des récepteurs de minéralocorticoïdes, nitrates organiques, donneurs de NO et substances à action inotrope positive.

10. Médicament selon la revendication 8 ou 9, destiné au traitement et/ou à la prophylaxie de l'insuffisance cardiaque aiguë ou chronique, de l'hyponatrémie hypervolumique ou euvolumique, de la cirrhose du foie, d'ascites, d'oedèmes et du syndrome de la sécrétion inadéquate d'ADH (SIADH).

11. Médicament selon la revendication 8 ou 9, destiné au traitement et/ou à la prophylaxie de défaillances rénales aiguës ou chroniques ainsi que de l'insuffisance rénale chronique.
